# EUROPEAN PATENT SPECIFICATION

(11) **EP 1 474 147 B1**
(45) Date of publication and mention of the grant of the patent: **05.05.2010**
(21) Application number: 02799913.5
(22) Date of filing: 09.12.2002
(51) Int. Cl.: A61K 31/506, C07D 239/94, A61P 35/00, C07D 401/12, C07D 403/12, C07D 405/14, C07D 409/14, C07D 417/12, C07D 413/12, C07D 409/12, C07D 401/04, C07D 409/04, C07D 495/04, C07D 471/04, C07D 475/00

(54) **PYRIMIDINE-BASED COMPOUNDS USEFUL AS GSK-3 INHIBITORS**
VERBINDUNGEN AUF PYRIMIDIN-BASIS ALS GSK-3-HEMMER
COMPOSES A BASE DE PYRIMIDINE UTILES EN TANT QU'INHIBITEURS DES GSK-3

(30) Priority: 07.12.2001 US 338857 P
(43) Date of publication of application: 10.11.2004
(60) Divisional application: 10158386.2
(73) Proprietor: VERTEX PHARMACEUTICALS INCORPORATED, Cambridge, MA 02139-4242 (US)
(72) Inventor: CHOQUETTE, Deborah, Medford, MA 02155 (US); DAVIES, Robert, J., Arlington, MA 02474 (US); WANNAMAKER, Marion, W., Stow, MA 01775 (US)
(74) Representative: Gambell, Derek
(86) International application number: PCT/US2002/039190
(87) International publication number: WO 2003/049739

(56) References cited:
- EP-A- 0 826 673
- WO-A-01/44206
- WO-A-01/44246
- WO-A-02/22605
- WO-A-98/09960
- WO-A-99/65897
- WO-A-02/053557
- US-A- 3 506 665
- DATABASE CHEMCATS [Online] CHEMICAL ABSTRACTS SERVICES COLUMBUS, OHIO, US; XP002232960 & "Maybridge HTS" 11 November 2002 (2002-11-11) , MAYBRIDGE PLC , TINAGEL, CORNWALL, PL34 0HW UNITED KINGDOM
- VAN MUIJLWIJK-KOEZEN ET AL: "Isoquinoline and Quinazoline Urea Analogues as Antagonists for the Human Adenosine A3 Receptor" JOURNAL OF MEDICINAL CHEMISTRY, AMERICAN CHEMICAL SOCIETY, US, vol. 43, no. 5, 1 June 2000 (2000-06-01), pages 2227-2338, XP002147879 ISSN: 0022-2623
- DATABASE CA [Online] CHEMICAL ABSTRACTS SERVICE, COLUMBUS, OHIO, US; KOMKOV, A. V. ET AL: "5-Acetyl-6-amino-4-(methylthio)-2-phenylp yrimidine and its use in the synthesis of functionalized pyrido[2,3-d]pyrimidines and pyrimido[4,5-d]pyrimidines" retrieved from STN Database accession no. 124:117226 XP002232961 & IZVESTIYA AKADEMII NAUK, SERIYA KHIMICHESKAYA (1995), (7), 1324-8 ,

## Description

### CROSS REFERENCE TO RELATED APPLICATIONS

The present application claims priority under 35 U.S.C. § 119(e) to U.S. Provisional Application number 60/338,857, filed December 7, 2001, entitled "Pyrimidine-Based Compounds Useful as GSK-3 Inhibitors", the entire contents of which are hereby incorporated by reference.

### FIELD OF THE INVENTION

The present invention is in the field of medicinal chemistry and relates to compounds that are protein kinase inhibitors, compositions containing such compounds and methods of use. More particularly, this invention relates to compounds that are inhibitors of Glycogen synthase kinase-3 (GSK-3). The invention also relates to methods of treating diseases associated with the protein kinase GSK-3, such as diabetes, cancer, stroke, and Alzheimer's disease.

### BACKGROUND OF THE INVENTION

The search for new therapeutic agents has been greatly aided in recent years by better understanding of the structure of enzymes and other biomolecules associated with target diseases. One important class of enzymes that has been the subject of extensive study is the protein kinases.

Protein kinases mediate intracellular signal transduction. They do this by effecting a phosphoryl transfer from a nucleoside triphosphate to a protein acceptor that is involved in a signaling pathway. There are a number of kinases and pathways through which extracellular and other stimuli cause a variety of cellular responses to occur inside the cell. Examples of such stimuli include environmental and chemical stress signals (e.g. osmotic shock, heat shock, ultraviolet radiation, bacterial endotoxin, H₂O₂), cytokines (e.g. interleukin-1 (IL-1) and tumor necrosis factor α (TNF-α)), and growth factors (e.g. granulocyte macrophage-colony-stimulating factor (GM-CSF), and fibroblast growth factor (FGF). An extracellular stimulus may effect one or more cellular responses related to cell growth, migration, differentiation, secretion of hormones, activation of transcription factors, muscle contraction, glucose metabolism, control of protein synthesis and regulation of cell cycle.

Many diseases are associated with abnormal cellular responses triggered by protein kinase-mediated events. These diseases include autoimmune diseases, inflammatory diseases, neurological and neurodegenerative diseases, cancer, cardiovascular diseases, allergies and asthma, Alzheimer's disease and hormone-related diseases. Accordingly, there has been a substantial effort in medicinal chemistry to find protein kinase inhibitors that are effective as therapeutic agents.

Glycogen synthase kinase-3 (GSK-3) is a serine/threonine protein kinase comprised of α and β isoforms that are each encoded by distinct genes [Coghlan et al., Chemistry & Biology, 7, 793-803 (2000); and Kim and Kimmel, Curr. Opinion Genetics Dev., 10, 508-514 (2000)]. GSK-3 has been implicated in various diseases including diabetes, Alzheimer's disease, CNS disorders such as manic depressive disorder and neurodegenerative diseases, and cardiomyocyte hypertrophy [WO 99/65897; WO 00/38675; and Haq et al., J. Cell Biol. (2000) 151, 117-130]. These diseases may be caused by, or result in, the abnormal operation of certain cell signaling pathways in which GSK-3 plays a role. GSK-3 has been found to phosphorylate and modulate the activity of a number of regulatory proteins. These proteins include glycogen synthase, which is the rate limiting enzyme necessary for glycogen synthesis, the microtubule associated protein Tau, the gene transcription factor β-catenin, the translation initiation factor e1F2B, as well as ATP citrate lyase, axin, heat shock factor-1, c-*Jun,* c-*myc,* c-*myb,* CREB, and CEPBα. These diverse protein targets implicate GSK-3 in many aspects of cellular metabolism, proliferation, differentiation and development.

In a GSK-3 mediated pathway that is relevant for the treatment of type II diabetes, insulin-induced signaling leads to cellular glucose uptake and glycogen synthesis. Along this pathway, GSK-3 is a negative regulator of the insulin-induced signal. Normally, the presence of insulin causes inhibition of GSK-3 mediated phosphorylation and deactivation of glycogen synthase. The inhibition of GSK-3 leads to increased glycogen synthesis and glucose uptake [Klein et al., PNAS, 93, 8455-8459 (1996); Cross et al., Biochem. J., 303, 21-26 (1994); Cohen, Biochem. Soc. Trans., 21, 555-567 (1993); and Massillon et al., Biochem J. 299, 123-128 (1994)]. However, in a diabetic patient, where the insulin response is impaired, glycogen synthesis and glucose uptake fail to increase despite the presence of relatively high blood levels of insulin. This leads to abnormally high blood levels of glucose with acute and long term effects that may ultimately result in cardiovascular disease, renal failure and blindness. In such patients, the normal insulin-induced inhibition of GSK-3 fails to occur. It has also been reported that in patients with type II diabetes, GSK-3 is overexpressed [WO 00/38675]. Therapeutic inhibitors of GSK-3 are therefore potentially useful for treating diabetic patients suffering from an impaired response to insulin.

GSK-3 activity has also been associated with Alzheimer's disease. This disease is characterized by the well-known β-amyloid peptide and the formation of intracellular neurofibrillary tangles. The neurofibrillary tangles contain hyperphosphorylated Tan protein, in which Tau is phosphorylated on abnormal sites. GSK-3 has been shown to phosphorylate these abnormal sites in cell and animal models. Furthermore, inhibition of GSK-3 has been shown to prevent hyperphosphorylation of Tau in cells [Lovestone et al., Current Biology 4, 1077-86 (1994); and Brownlees et al., Neuroreport 8, 3251-55 (1997)]. Therefore, it is believed that GSK-3 activity may promote generation of the neurofibrillary tangles and the progression of Alzheimer's disease.

Another substrate of GSK-3 is β-catenin which is degradated after phosphorylation by GSK-3. Reduced levels of β-catenin have been reported in schizophrenic patients and have also been associated with other diseases related to increase in neuronal cell death [Zhong et al., Nature, 395, 698-702 (1998); Takashima et al., PNAS, 90, 7789-93 (1993); and Pei et al., J. Neuropathol. Exp, 56, 70-78 (1997)].

GSK-3 activity has also been associated with stroke [Wang et al., Brain Res, 859, 381-5 (2000); Sasaki et al., Neurol Res, 23, 588-92 (2001); Hashimoto et al., J. Biol. Chem, 277, 32985-32991 (2002)].

Considering the lack of currently available treatment options for the majority of the conditions associated with GSK-3 and other protein kinases, there is still a great need for new therapeutic agents that inhibit these protein targets.

We are aware of Chemical Abstracts RDR03878, RDR03874 (Interchim), RDR03879. RDR03877, RDR03876, RDR03871, RDR03870, RH 0210S (Maybridge plc) 11 November 2002 which disclose certain substituted ethane diamines of unspecified properties.

International patent publication WO 02/053557 ACTELION PHARMACEUTICALS LTD at al. which relates to novel sulfamides and their use as active ingredients in the preparation of pharmaceutical compositions. The disclosure also concerns related aspects including processes for the preparation of the compounds, pharmaceutical compositions containing one or more of those compounds and especially their use as endothelin receptor antagonists

International patent publication WO 02/22605 VERTEX PHARMA et al. describes novel pyrazole compositions comprising a pharmaceutically acceptable carrier. The compounds are useful as protein kinase inhibitors, especially as inhibitors of aurora-2 and GSK-3, for treating diseases such as cancer, diabetes and Alzheimer's disease.

International patent publication WO 99/65897 CHIRON CORP et al, describes new pyrimidine or pyridine based compounds, compositions and methods of inhibiting the activity of glycogen synthase kinase (GSK3) in vitro and of treatment of GSK3 mediated disorders in vivo are provided. The methods, compounds and compositions of the invention may be employed alone, or in combination with other pharmacologically active agents in the treatment of disorders mediated by GSK3 activity, such as in the treatment of diabetes, Alzheimer's disease and other neurodegenerative disorders, obesity, atherosclerotic cardiovascular disease, essential hypertension, polycystic ovary syndrome, syndrome X, ischemia, traumatic brain injury, bipolar disorder immunodeficiency or cancer.

WO 01/44206 CHIRON CORP et al. describes new bicyclic based compounds, compositions and methods of inhibiting the activity of glycogen synthase kinase (GSK3) *in vitro* and of treatment of GSK3 mediated disorders *in vivo* are provided. The methods, compounds and compositions of the invention may be employed alone, or in combination with other pharmacologically active agents in the treatment of disorders mediated by GSK3 activity, such as in the treatment of diabetes, Alzheimer's disease and other neurodegenerative disorders, obesity, atherosclerotic cardiovascular disease, essential hypertension, polycystic ovary syndrome, syndrome X, ischemia, traumatic brain injury, bipolar disorder, immunodeficiency or cancer.

International patent publication WO 01/44246 CHIRON CORP et al. describes bicyclic based compounds, compositions and methods of inhibiting the activity of glycogen synthase kinase (GSK3) *in vitro* and a treatment of GSK3 mediated disorders *in vivo* are provided. The methods, compounds and compositions of the invention may be employed alone, or in combination with other pharmacologically active agents in the treatment of disorders mediated by GSK3 activity, such as in the treatment of diabetes, Alzheimer's disease and other neurodegenerative disorders, obesity, atherosclerotic cardiovascular disease, essential hypertension, polycystic ovary syndrome, syndrome X, ischemia, traumatic brain injury, bipolar disorder, immunodeficiency or cancer.

Van Muijlwik-Koetzen et al. Journal of Medicinal Chemistry, American Chemical Society, vol 43, No. 5, 1 June 2000, pages 2227-2338 describe isoquinoline and quinazoline urea analogues as antagonists for the human adenosine A3 receptor.

European patent publication EP 0 826 673 DAINIPPON PHARMACEUTICAL CO describes acetamide derivatives and physiologically acceptable acid-addition salts thereof. The compounds act electively on the peripheral BZ omega 3 receptor and exhibit excellent pharmocological effects, which makes them useful as a remedy and preventive for central diseases, for example, diseases associated with anxiety, depression and epilepsy, etc.

International patent publication WO 98/09960 DAINIPPON PHARMACEUTICAL CO et al. describes 2,4-Disubatituted pyrimidine derivatives or physiologically acceptable acid-addition salts thereof. These compounds are expected to be useful as remedies and preventives for central diseases, for example, diseases associated with anxiety, such as neurosis and psychosomatic disorder, depression and epilepsy; circulatory diseases such as angina pectoris and hypertension; immunologic nervous diseases such as multiple sclerosis; or immunologic inflammatory diseases such as rheumatism,

United States patent US 3506665 AMERICAN HOME PRODUCTS CORPORATION describes 4,4'-(ethylenediimino)bis[2-phenylpyrimidine-5-carboxylic acid] dialky esters, 4,4'-(o-phenylenediimino)bis[2-phenylpyrimidine-5-carboxylic acid] dialky esters, and 4,4'-(1,4 piperazinediyl)bis[2-phenyl-5-pyrimidinecarboxylic acid] dialky esters which are pharmacologically active as mydriatic agents.

Finally, we are aware that Chemical Abstracts Accession No. 124:117226 (KOMKOV et al.) describes 5-acetyl-6-amino-4-(methylthio)-2-phenylpyrimidine and its use in the synthesis of functionalized pyrido [2,3-d]pyrimidines and pyrimidol [4,5-d]pyrimidines.

### SUMMARY OF THE INVENTION

The present invention addresses this need by providing compounds and pharmaceutical compositions thereof that are effective as protein kinase inhibitors, particularly as inhibitors of GSK-3. Essential and optional features of the present invention are set out in the accompanying main- and sub-claims respectively. The compounds of the invention have the general formula I: 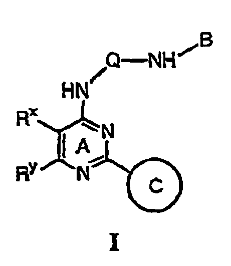 or a pharmaceutically acceptable derivative or prodrug thereof, wherein B, Q, Ring C, R^{X}, and R^{Y} are as defined below.

In another embodiment, the invention provides pharmaceutical compositions comprising a GSK-3 inhibitor of this invention. These compositions may be utilized in methods for treating or preventing a variety of GSK-3 mediated conditions, such as neurodegenerative disorders, diabetes, Alzheimer's disease, Huntington's Disease, Parkinson's Disease, AIDS-associated dementia, amyotrophic lateral sclerosis (ALS, Lou Gehrig's Disease), multiple sclerosis (MS), schizophrenia, cardiomyocyte hypertrophy, reperfusion/ ischemia, baldness, and stroke.

The compositions of this invention are also useful in methods for enhancing glycogen synthesis and/or lowering blood levels of glucose and therefore are especially useful for diabetic patients. These compositions are also useful in methods for inhibiting the production of hyperphosphorylated Tau protein, which is useful in halting or slowing the progression of Alzheimer's disease, and in a method for inhibiting the phosphorylation of β-catenin, which is useful for treating schizophrenia.

### DESCRIPTION OF THE INVENTION

It has now been found that compounds of this invention and pharmaceutical compositions thereof are effective as protein kinase inhibitors, particularly as inhibitors of GSK-3. These compounds have the general formula I: 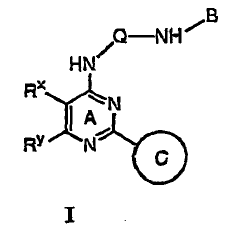 or a pharmaceutically acceptable derivative or prodrug thereof, wherein Ring A, B, Q, Ring C, R^{X} and R^{Y} are as defined in claim 1.

As used herein, the following definitions shall apply unless otherwise indicated.

The phrase "optionally substituted" is used interchangeably with the phrase "substituted or unsubstituted." Unless otherwise indicated, an optionally substituted group may have a substituent at each substitutable position of the group, and each substitution is independent of the other.

The term "aliphatic" or "aliphatic group", as used herein, means a straight-chain or branched, substituted or unsubstituted C₁-C₈ hydrocarbon chain that is completely saturated or that contains one or more units of unsaturation, or a monocyclic C₃-C₈ hydrocarbon or bicyclic C₈-C₁₂ hydrocarbon that is completely saturated or that contains one or more units of unsaturation, but which is not aromatic (also referred to herein as "carbocycle" "cycloaliphatic" or "cycloalkyl"), that has a single point of attachment to the rest of the molecule wherein any individual ring in said bicyclic ring system has 3-7 members. For example, suitable aliphatic groups include, but are not limited to, linear or branched, substituted or unsubstituted alkyl, alkenyl, alkynyl groups and hybrids thereof such as (cycloalkyl)alkyl, (cycloalkenyl)alkyl or (cycloalkyl)alkenyl.

The terms "alkyl", "alkoxy", "hydroxyalkyl", "alkoxyalkyl", and "alkoxycarbonyl", used alone or as part of a larger moiety include both straight and branched chains containing one to twelve carbon atoms. The terms "alkenyl" and "alkynyl" used alone or as part of a larger moiety shall include both straight and branched chains containing two to twelve carbon atoms.

The term "heteroatom" means nitrogen, oxygen, or sulfur and includes any oxidized form of nitrogen and sulfur, and the quatemized form of any basic nitrogen. Also the term "nitrogen" includes a substitutable nitrogen of a heterocyclic ring. As an example, in a saturated or partially unsaturated ring having 0-3 heteroatoms selected from oxygen, sulfur or nitrogen, the nitrogen may be N (as in 3,4-dihydro-2*H*-pyrrolyl), NH (as in pyrrolidinyl) or NR⁺ (as in N-substituted pyrrolidinyl).

The term "unsaturated", as used herein, means that a moiety has one or more units of unsaturation, and includes aryl rings.

The term "aryl" used alone or as part of a larger moiety as in "aralkyl", "aralkoxy", or "aryloxyalkyl", refers to monocyclic, bicyclic and tricyclic ring systems having a total of five to fourteen ring members, wherein at least one ring in the system is aromatic and wherein each ring in the system contains 3 to 7 ring members. The term "aryl" may be used interchangeably with the term "aryl ring". The term "aryl" also refers to heteroaryl ring systems as defined hereinbelow.

The term "heterocycle", "heterocyclyl", "heterocycloaliphatic", or "heterocyclic" as used herein means non-aromatic, monocyclic, bicyclic or tricyclic ring systems having five to fourteen ring members in which one or more ring members is a heteroatom, wherein each ring in the system contains 3 to 7 ring members.

The term "heteroaryl", used alone or as part of a larger moiety as in "heteroaralkyl" or "heteroarylalkoxy", refers to monocyclic, bicyclic and tricyclic ring systems having a total of five to fourteen ring members, wherein at least one ring in the system is aromatic, at least one ring in the system contains one or more heteroatoms, and wherein each ring in the system contains 3 to 7 ring members. The term "heteroaryl" may be used interchangeably with the term "heteroaryl ring" or the term "heteroaromatic".

An aryl (including aralkyl, aralkoxy, aryloxyalkyl and the like) or heteroaryl (including heteroaralkyl and heteroarylalkoxy and the like) group may contain one or more substituents. Suitable substituents on the unsaturated carbon atom of an aryl, heteroaryl, aralkyl, or heteroaralkyl group are selected from halogen, -R°, -OR°, -SR°, 1,2-methylene-dioxy, 1,2-ethylenedioxy, phenyl (Ph) optionally substituted with R°, -O(Ph) optionally substituted with R°, -CH₂(Ph) optionally substituted with R°, -CH₂CH₂(Ph),optionally substituted with R°, -NO₂, -CN, -N(R°)₂, -NR°C(O)R°, -NR°C(O)N(R°)₂, -NR°CO₂R°, -NR°NR°C(O)R°, -NR°NR°C(O)N(R°)₂, -NR°NR°CO₂R°, -C(O)C(O)R°, -C(O)CH₂C(O)R°, -CO₂R°, -C(O)R°, -C(O)N(R°)₂, -OC(O)N(R°)₂, -S(O)₂R°, -SO₂N(R°)₂, -S(O)R°, -NR°SO₂N(R°)₂, -NR°SO₂R°, -C(=S)N(R°)₂, -C(=NH)-N(R°)₂, or -(CH₂)_{q}NHC(O)R° wherein q is 0-2, and wherein each R° is independently selected from hydrogen, optionally substituted C₁₋₆ aliphatic, an unsubstituted 5-6 membered heteroaryl or heterocyclic ring, phenyl, -O(Ph), or -CH₂(Ph), or wherein two occurrences of R°, on the same substituent or different substituents, taken together, form a 5-8-membered heterocyclyl or heteroaryl ring having 1-3 heteroatoms independently selected from nitrogen, oxygen, or sulfur. Optional substituents on the aliphatic group of R° are selected from NH₂, NH(C₁₋₄ aliphatic), N(C₁₋₄ aliphatic)₂, halogen, C₁₋₄ aliphatic, OH, O(C₁₋₄ aliphatic), NO₂, CN, CO₂H, CO₂(C₁₋₄ aliphatic), O(halo C₁₋₄ aliphatic), or halo C₁₋₄ aliphatic.

An aliphatic group or a non-aromatic heterocyclic ring may contain one or more substituents. Suitable substituents on the saturated carbon of an aliphatic group or of a non-aromatic heterocyclic ring are selected from those listed above for the unsaturated carbon of an aryl or heteroaryl group and the following: =O, =S, =NNHR*, =NN(R*)₂, =NNHC(O)R*, =NNHCO₂(alkyl), =NNHSO₂(alkyl), or =NR*, where each R* is independently selected from hydrogen or an optionally substituted C₁₋₆ aliphatic. Optional substituents on the aliphatic group of R^{*} are selected from NH₂, NH(C₁₋₄ aliphatic), N(C₁₋₄ aliphatic)₂, halogen, C₁₋₄ aliphatic, OH, O(C₁₋₄ aliphatic), NO₂, CN, CO₂H, CO₂(C₁₋₄ aliphatic), O(halo C₁₋₄ aliphatic), or halo(C₁₋₄ aliphatic).

Optional substituents on the nitrogen of a non-aromatic heterocyclic ring are selected from -R⁺, -N(R⁺)₂, -C(O)R⁺, -CO₂R⁺, -C(O)C(O)R⁺, -C(O)CH₂C(O)R⁺, -SO₂R⁺, -SO₂N(R⁺)₂, -C(=S)N(R⁺)₂, -C(=NH)-N(R⁺)₂, or -NR⁺SO₂R⁺; wherein R⁺ is hydrogen, an optionally substituted C₁₋₆ aliphatic, optionally substituted phenyl, optionally substituted -O(Ph), optionally substituted -CH₂(Ph), optionally substituted -CH₂CH₂(Ph), or an unsubstituted 5-6 membered heteroaryl or heterocyclic ring, or wherein two occurrences of R⁺, on the same substituent or different substituents, taken together, form a 5-8-membered heterocyclyl or heteroaryl ring having 1-3 heteroatoms independently selected from nitrogen, oxygen, or sulfur. Optional substituents on the aliphatic group or the phenyl ring of R⁺ are selected from NH, NH(C₁₋₄ aliphatic), N(C₁₋₄ aliphatic)₂, halogen, C₁₋₄ aliphatic, OH, O(C₁₋₄ aliphatic), NO₂, CN, CO₂H, CO₂(C₁₋₄ aliphatic), O(halo C₁₋₄ aliphatic), or halo(C₁₋₄ aliphatic).

The term "alkylidene chain" refers to a straight or branched carbon chain that may be fully saturated or have one or more units of unsaturation and has two points of attachment to the rest of the molecule.

A combination of substituents or variables is permissible only if such a combination results in a stable or chemically feasible compound. A stable compound or chemically feasible compound is one that is not substantially altered when kept at a temperature of 40°C or less, in the absence of moisture or other chemically reactive conditions, for at least a week.

It will be apparent to one skilled in the art that certain compounds of this invention may exist in tautomeric forms, all such tautomeric forms of the compounds being within the scope of the invention.

Unless otherwise stated, structures depicted herein are also meant to include all stereochemical forms of the structure; i.e., the R and S configurations for each asymmetric center. Therefore, single stereochemical isomers as well as enantiomeric and diastereomeric mixtures of the present compounds are within the scope of the invention. Unless otherwise stated, structures depicted herein are also meant to include compounds that differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon are within the scope of this invention. Such compounds are useful, for example, as analytical tools or probes in biological assays.

*II. Description of Certain Exemplary Compounds:*

As described generally above, compounds of formula **I** include both monocyclic and bicyclic pyrimidine systems. For example, in certain embodiments, R^{X} and R^{Y} may be taken together to form a fused ring, providing a bicyclic ring system. Preferred R^{X}/R^{Y} rings include a 5-, 6-, 7-, or 8-membered partially saturated or aromatic ring having 0-2 heteroatoms, wherein said R^{X}/R^{Y} ring is optionally substituted with oxo or one or more occurrences of TR³ or R⁴. Examples of certain preferred pyrimidine ring A systems of formula I are the mono- and bicyclic ring A systems shown below, wherein B, Q, TR³, R⁴ and ring C are as defined generally above and herein, and q is 0-4.

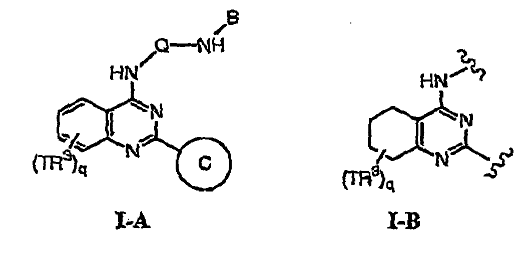

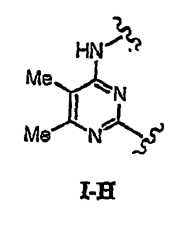

Exemplary R⁴ substituents include -R⁷, -COR⁷, -CO₂(C₁₋₆ aliphatic), -CON(R⁷)₂, or -SO₂R⁷, or two R⁴ on the same nitrogen are taken together to form a 5-8 membered heterocyclyl or heteroaryl ring, and R' and R⁷ are as defined herein. Preferred R^{X}/R^{Y} ring substituents include -halo, -R', -OR', -COR', -CO₂R', -CON(R⁴)₂, -CN, or -N(R⁴)₂ wherein R' is H or an optionally substituted C₁₋₆ aliphatic group.

As described generally above, Q is a C₁₋₄ alkylidene chain, wherein each methylene unit of said Q is substituted by R² and R^{2'}. Each of R² and R^{2'} is hydrogen. In certain preferred embodiments, Q is -CH₂CH₂-, -CH₂CH₂CH₂-, or -CH₂CH₂CH₂CH₂-.

In certain embodiments, B is an optionally substituted carbocyclic aryl having 5-10 carbon ring atoms. In certain other embodients, B is an optionally substituted heteroaryl having 5-10 carbon ring atoms and one or more ring heteroatoms. Preferred B groups include pyridyl, pyrimidinyl, thiazolyl, indolyl, imidazolyl, oxadiazolyl, tetrazolyl, pyrazinyl, triazolyl, thionyl, furanyl, quinolinyl, purinyl, naphthyl, benzothiazolyl, benzopyridyl, benzimidazolyl, indazolyl, isothiazolyl, pyrazolyl, pyridazinyl, isoxazolyl, phenyl, benzothiophenyl, and pyridothiophenyl, all of which may be substituted with at least one and no more than three substituents. Exemplary substituents are independently selected from, without limitation, nitro, amino, cyano, halo, thioamido, amidino, oxamidino, alkoxyamidino, imidino, guanidino, sulfonamido, carboxyl, formyl, alkyl, haloalkyl, alkoxy, haloalkoxy, alkoxyalkyl, alkylaminoalkoxy, alkylcarbonyl aralkylcarbonyl, heteroaralkylcarbonyl, alkylthio, aminoalkyl, cyanoalkyl, hydroxy, alkoxycarbonyl, aryl, aralkyl, heteroaryl, and heteroaralkyl, wherein all alkyl moieties have 1-10 carbon atoms and are unsubstituted unless otherwise indicated.

In other preferred embodiments, B is optionally substituted 2-pyridyl and compounds have the general formula **II**: 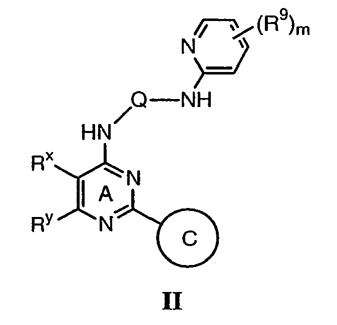 wherein m is 0-4, and each occurrence of R⁹ is independently selected from H, nitro, amino, cyano, halo, thioamido, amidino, oxamidino, alkoxyamidino, imidino, guanidino, sulfonamido, carboxyl, formyl, alkyl, haloalkyl, alkoxy, haloalkoxy, alkoxyalkyl, alkylaminoalkoxy, alkylcarbonyl, aralkylcarbonyl, heteroaralkylcarbonyl, alkylthio, aryl, aralkyl, heteroaryl, and heteroaralkyl, wherein all alkyl moieties have 1-10 carbon atoms and are unsubstituted unless otherwise indicated. In preferred embodiments, m is 0, 1, or 2. In more preferred embodiments, m is 1 or 2. Most preferred R⁹ substituents include hydrogen, C₁₋₄alkyl, nitro, amino, cyano, cyanomethyl, trifluoromethyl, hydroxy, and methoxy.

In other preferred embodiments, B is indazolyl, pyrazolyl, or thiazolyl optionally substituted with one or more independent occurrences of R⁹. Preferred R⁹ substituents on indazolyl, pyrazolyl, or thiazolyl include hydrogen, C₁₋₄alkyl, nitro, amino, cyano, cyanomethyl, trifluoromethyl, hydroxy, and methoxy.

Preferred formula **I** Ring C groups are phenyl and pyridinyl. When two adjacent substituents on Ring C are taken together to form a fused ring, Ring C is contained in a bicyclic ring system. Preferred fused rings include a benzo or pyrido ring. Such rings preferably are fused at ortho and meta positions of Ring C. Examples of preferred bicyclic Ring C systems include naphthyl, quinolinyl, isoquinolinyl and benzodioxolyl.

Preferably, Ring C groups have one to two ortho substituents independently selected from R¹. An ortho position on Ring C is defined relative to the position where the pyrimidine ring is attached. Preferred R¹ groups include -halo, an optionally substituted C₁₋₆ aliphatic groups, phenyl, -COR⁶, -OR⁶, -CN, -SO₂R⁶, -SO₂NH₂, -N(R⁶)_{2,} -CO₂R⁶, -CONH₂, -NHCOR⁶, -OC(O)NH₂, or -NHSO₂R⁶. When R¹ is an optionally substituted C₁₋₆ aliphatic group, the most preferred optional substituents are halogen. Examples of preferred R¹ groups include -CF₃, -Cl, -F, -CN, -COCH₃, -OCH₃, -OH, -CH₂CH₃, -OCH₂CH₃, -CH₃, - CF₂CH₃, cyclohexyl, t-butyl, isopropyl, cyclopropyl, -C≡CH, -C≡C-CH₃, -SO₂CH₃, -SO₂NH₂, -N(CH₃)₂, -CO₂CH₃, -CONH₂, -NHCOCH₃, -OC(O)NH₂, -NHSO₂CH₃, and -OCF₃.

On Ring C of formula **I,** preferred R⁵ substituents, when present, include -halo, -CN, -NO₂, -N(R⁴)₂, optionally substituted C₁₋₆ aliphatic group, -OR', -C(O)R', -CO₂R', -CONH(R⁴), -N(R⁴)COR', -SO₂N(R⁴)₂, and -N(R⁴)SO₂R'. More preferred R⁵ substituents include -Cl, -F, -CN, -CF₃, -NH₂, NH(C₁₋₄ aliphatic), -N(C₁₋₄ aliphatic)₂, -O(C₁₋₄ aliphatic), C₁₋₄ aliphatic, and -CO₂(C₁₋₄ aliphatic). Examples of such preferred R⁵ substituents include -Cl, F, -CN, -CF₃, -NH₂, -NHMe, -NMe₂, -OEt, methyl, ethyl, cyclopropyl, isopropyl, t-butyl, and -CO₂Et.

More preferred ring systems of formula **I** are the following, which may be substituted as described above, wherein B is pyridyl and R^{X} and R^{Y} are each methyl, or R^{X} and R^{Y} are taken together with the pyrimidine ring to form a quinazoline, tetrahydroquinazoline, or tetrahydropyridopyrimidine ring:

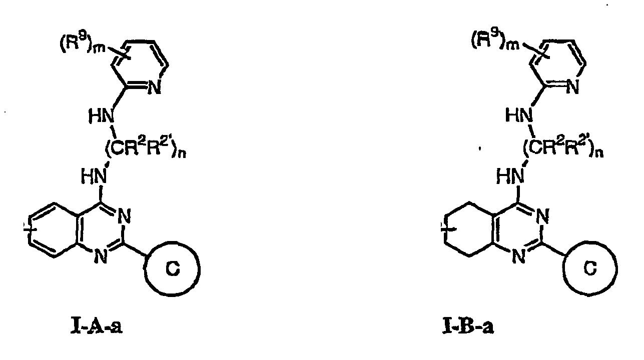

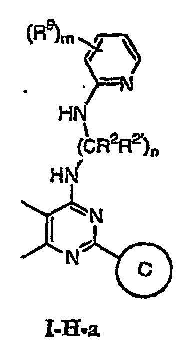

Particularly preferred are those compounds of formula I-A-a, I-B-a, or I-H-a, wherein ring C is a phenyl ring and R¹ is halo, methyl, cyano, OMe, OH, or trifluoromethyl.

Preferred formula **I** compounds include those compounds where one or more of, or each of, B, ring C, or R¹ is defined such that:
(a) B is an optionally substituted 5-6 membered monocyclic or 8-10 membered bicyclic aromatic ring, wherein said monocyclic or bicyclic ring has 1-4 heteroatoms selected from oxygen, sulfur or nitrogen;
(b) Ring C is a phenyl or pyridinyl ring having one or two ortho substituents independently selected from -R¹, wherein Ring C is further substituted by -R⁵ and wherein when Ring C and two adjacent substituents thereon form a bicyclic ring system, the bicyclic ring system is selected from a naphthyl, quinolinyl or isoquinolinyl ring; and
(c) R¹ is -halo, an optionally substituted C₁₋₆ aliphatic group, phenyl -COR⁶, -OR⁶, -CN, -SO₂R⁶, -SO₂NH₂, -N(R⁶)₂, -CO₂R⁶, -CONH₂, -NHCOR⁶, -OC(O)NH₂, or -NHSO₂R⁶.

Other preferred formula **I** compounds include those compounds where one or more of, or each of, B, R², ring C, R¹, or R⁵ is defined such that:
(a) B is an optionally substituted group selected from pyridyl, pyrimidinyl, thiazolyl, indolyl, imidazolyl, oxadiazolyl, tetrazolyl, pyrazinyl, triazolyl, thienyl, furanyl, quinolinyl, purinyl, naphthyl, benzothiazolyl, benzopyridyl, benzimidazolyl, indazolyl, isothiazolyl, pyrazolyl, pyridazinyl, isoxazolyl, phenyl, benzothiophenyl, or pyridothiophenyl;
(b) R² is H, alkyl, haloalkyl, heterocycloaminoalkyl, alkylaminoalkyl, and alkyl, wherein all alkyl moieties have 1-10 carbon atoms are unsubstituted;
(c) Ring C is a phenyl or pyridinyl ring having one or two ortho substituents independently selected from -R¹, wherein Ring C is further substituted by -R⁵ and wherein when Ring C and two adjacent substituents thereon form a bicyclic ring system, the bicyclic ring system is a naphthyl ring;
(d) R¹ is -halo, a C₁₋₆ haloaliphatic group, a C₁₋₆ aliphatic group, phenyl, OMe, OH, or -CN; and
(e) each R⁵ is independently selected from-halo, -CN, -NO₂, -N(R⁴)₂, optionally substituted C₁₋₆ aliphatic group, -OR', -C(O)R', -CO₂R', -CONH(R⁴), -N(R⁴)COR', -SO₂N(R⁴)₂, or -N(R⁴)SO₂R'.

Still other preferred formula **I** compounds include those compounds where one or more of, or each of, B, R², ring C, R¹, or R⁵ is defined such that:
(a) B is an optionally substituted pyridyl group optionally substituted by 0, 1, or 2 occurrences of R⁹, wherein each occurrence of R⁹ is independently hydrogen, C₁₋₄alkyl, nitro, amino, cyano, cyanomethyl, trifluoromethyl, hydroxy, and methoxy;
(b) R² is H, alkyl, haloalkyl, heterocycloaminoalkyl, alkylaminoalkyl, and alkyl, wherein all alkyl moieties have 1-10 carbon atoms are unsubstituted;
(c) Ring C is a phenyl or pyridinyl ring having one or two ortho substituents independently selected from -R¹, wherein Ring C is further substituted by -R⁵ and wherein when Ring C and two adjacent substituents thereon form a bicyclic ring system, the bicyclic ring system is a naphthyl ring;
(d) R¹ is -halo, a C₁₋₆ haloaliphatic group, a C₁₋₆ aliphatic group, phenol OMe, OH, or -CN; and
(e) each R⁵ is independently selected from halo, -CN, -NO₂, -N(R⁴)₂, optionally substituted C₁₋₆ aliphatic group, -OR', -C(O)R', -CO₂R', -CONH(R⁴), -N(R⁴)COR', -SO₂N(R⁴)₂, or -N(R⁴)SO₂R'.

Even more preferred formula **I** compounds include those compounds where one or more of, or each of, B, R², ring C, R¹, or R⁵ is defined such that:
(a) B is an optionally substituted pyridyl group optionally substituted with 2 independent occurrences of R⁹, wherein each occurrence of R⁹ is independently hydrogen, C₁₋₄alkyl, nitro, amino, cyano, cyanomethyl, trifluoromethyl, hydroxy, and methoxy;
(b) each R² and R^{2'} is H;
(c) Ring C is a phenyl ring having one or two ortho substituents independently selected from -R¹, wherein Ring C is further substituted by -R⁵;
(d) R¹ is -halo, a C₁₋₄ aliphatic group optionally substituted with halogen, OMe, OH, or -CN; and
(e) each R⁵ is independently selected from -Cl, -F, -CN, -CF₃, -NH₂, -NH(C₁₋₄ aliphatic), -N(C₁₋₄ aliphatic)₂, -O(C₁₋₄ aliphatic), optionally substituted C₁₋₄ aliphatic, and -CO₂(C₁₋₄ aliphatic).

Representative compounds of formula **I** are shown below in Table 1.

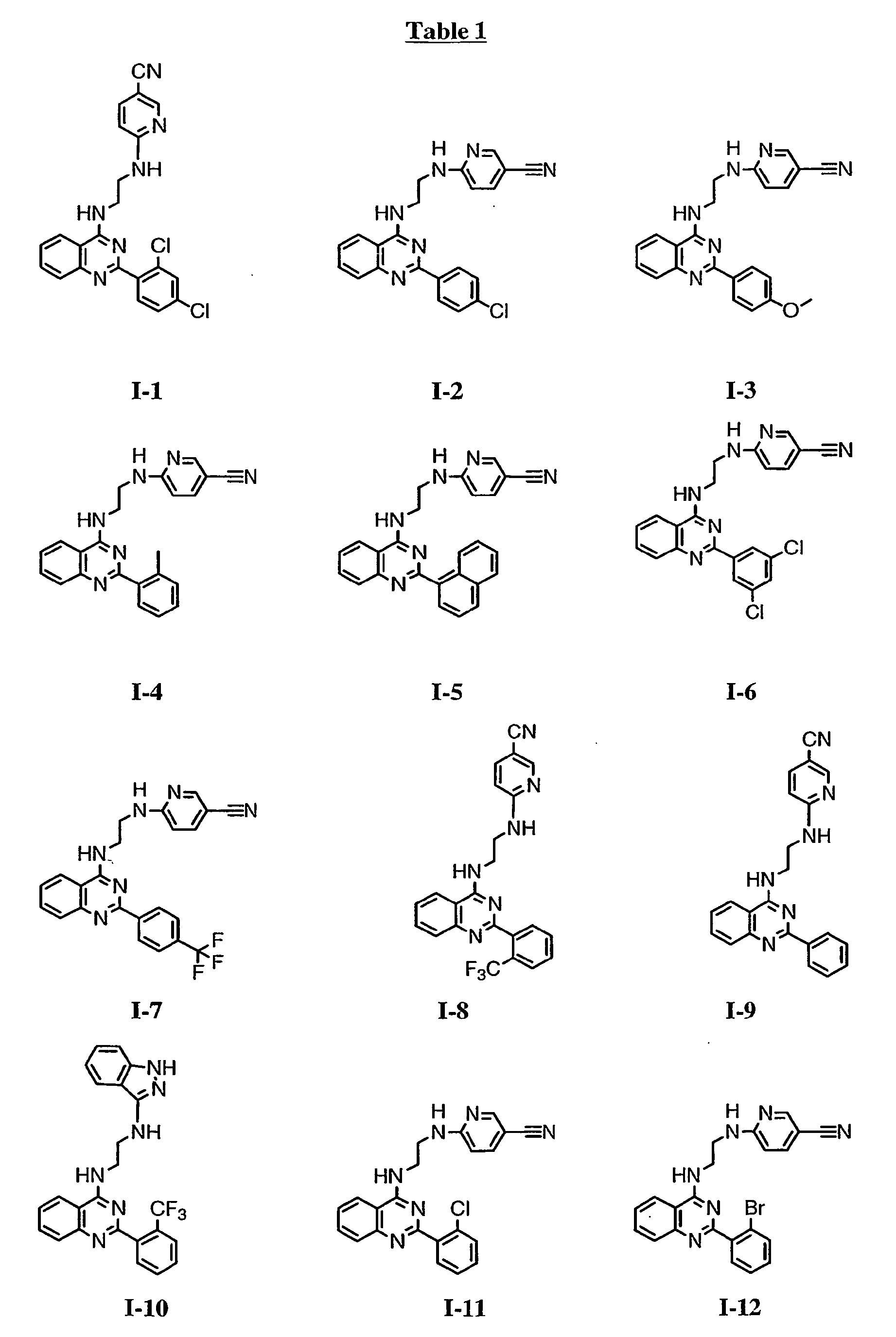

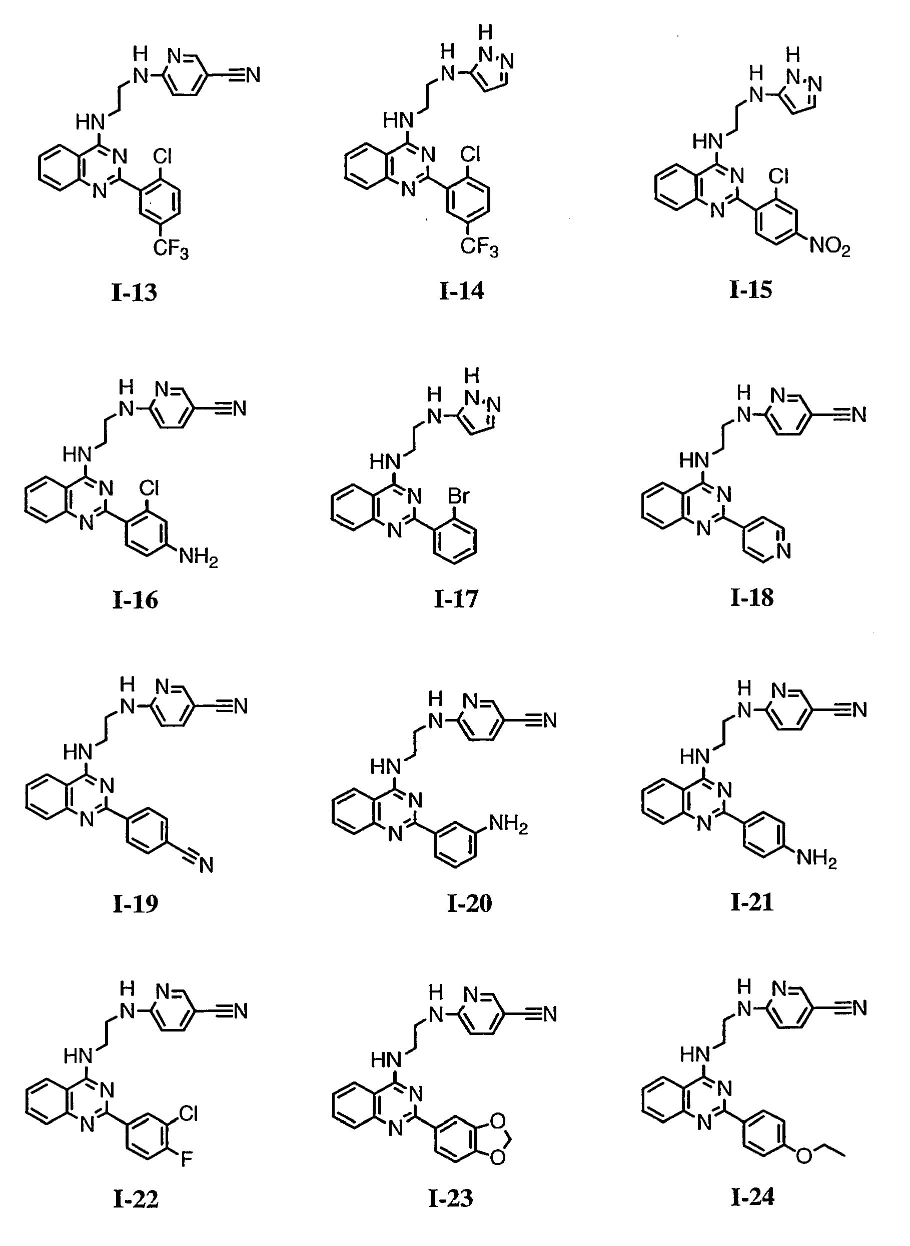

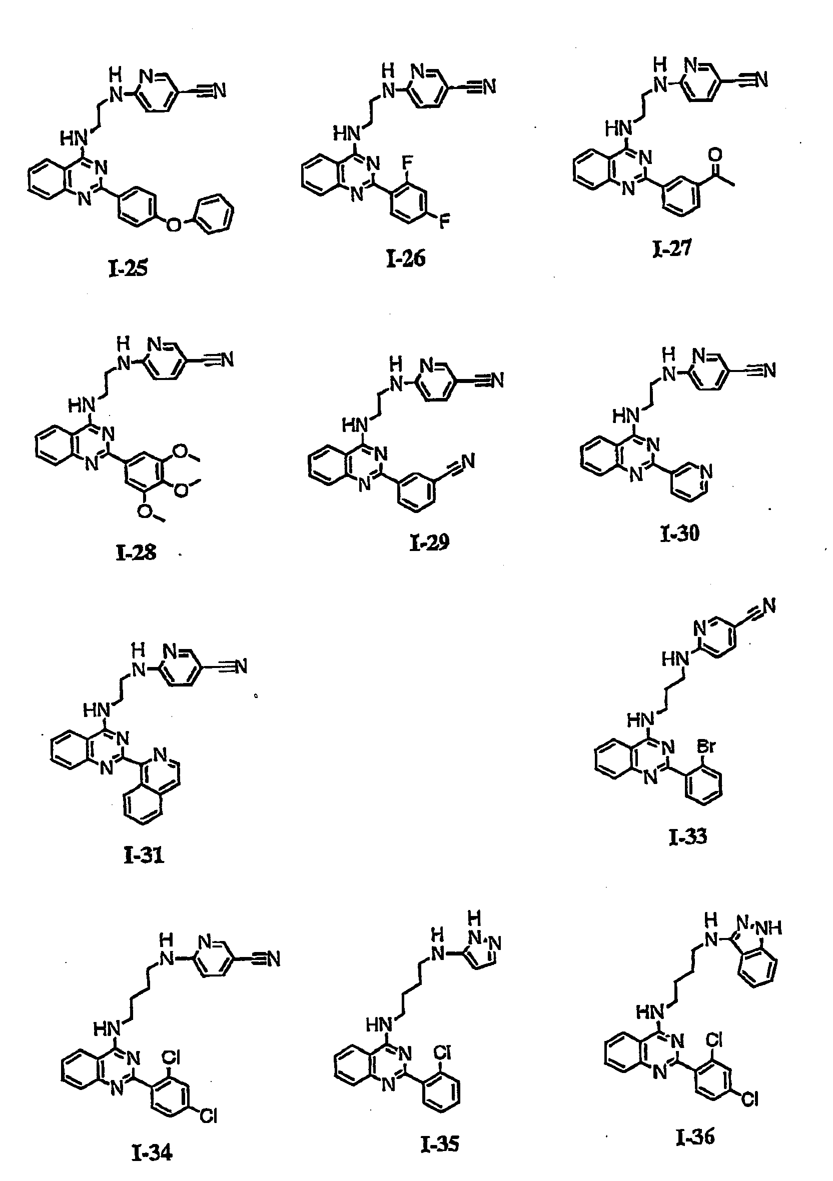

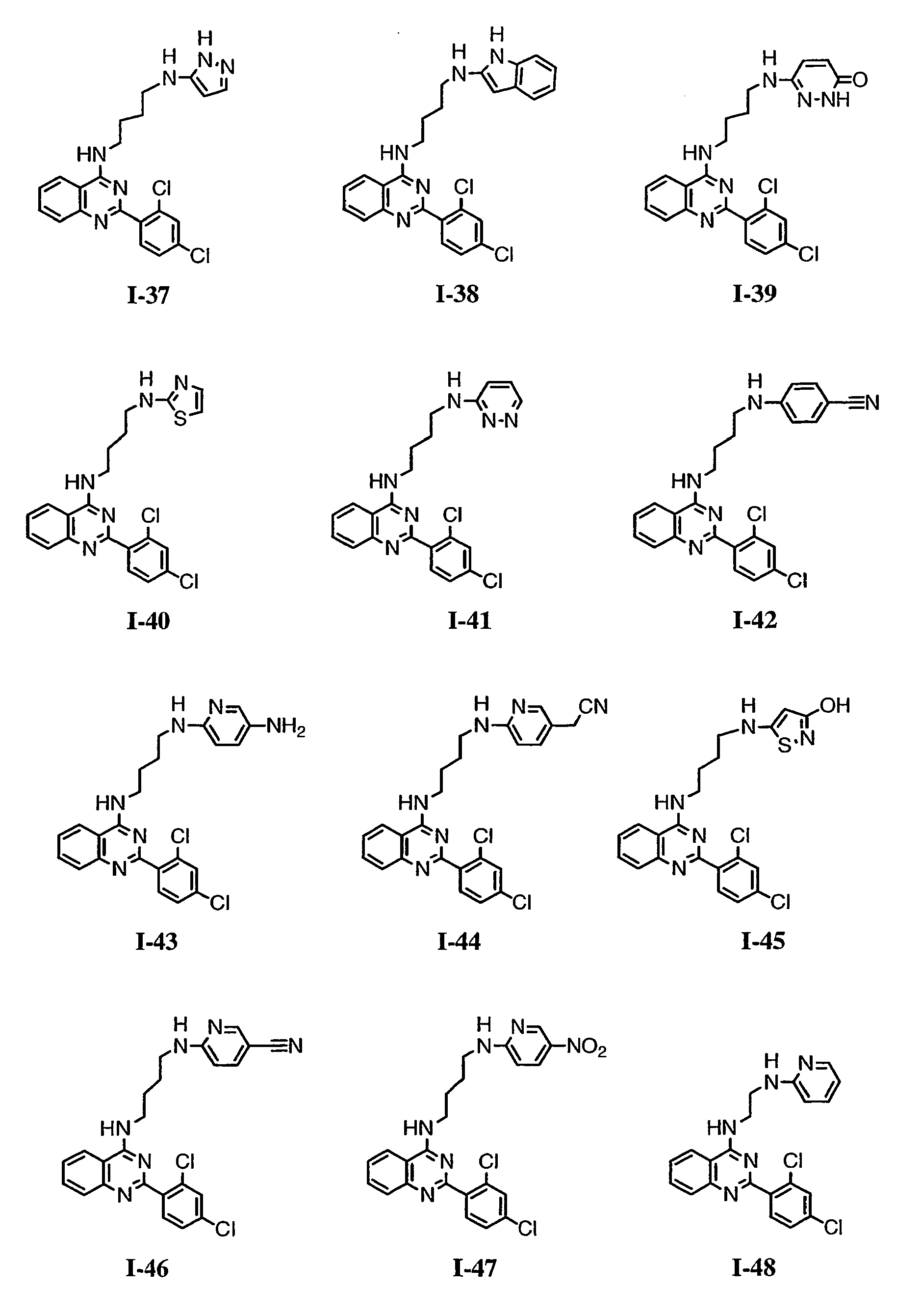

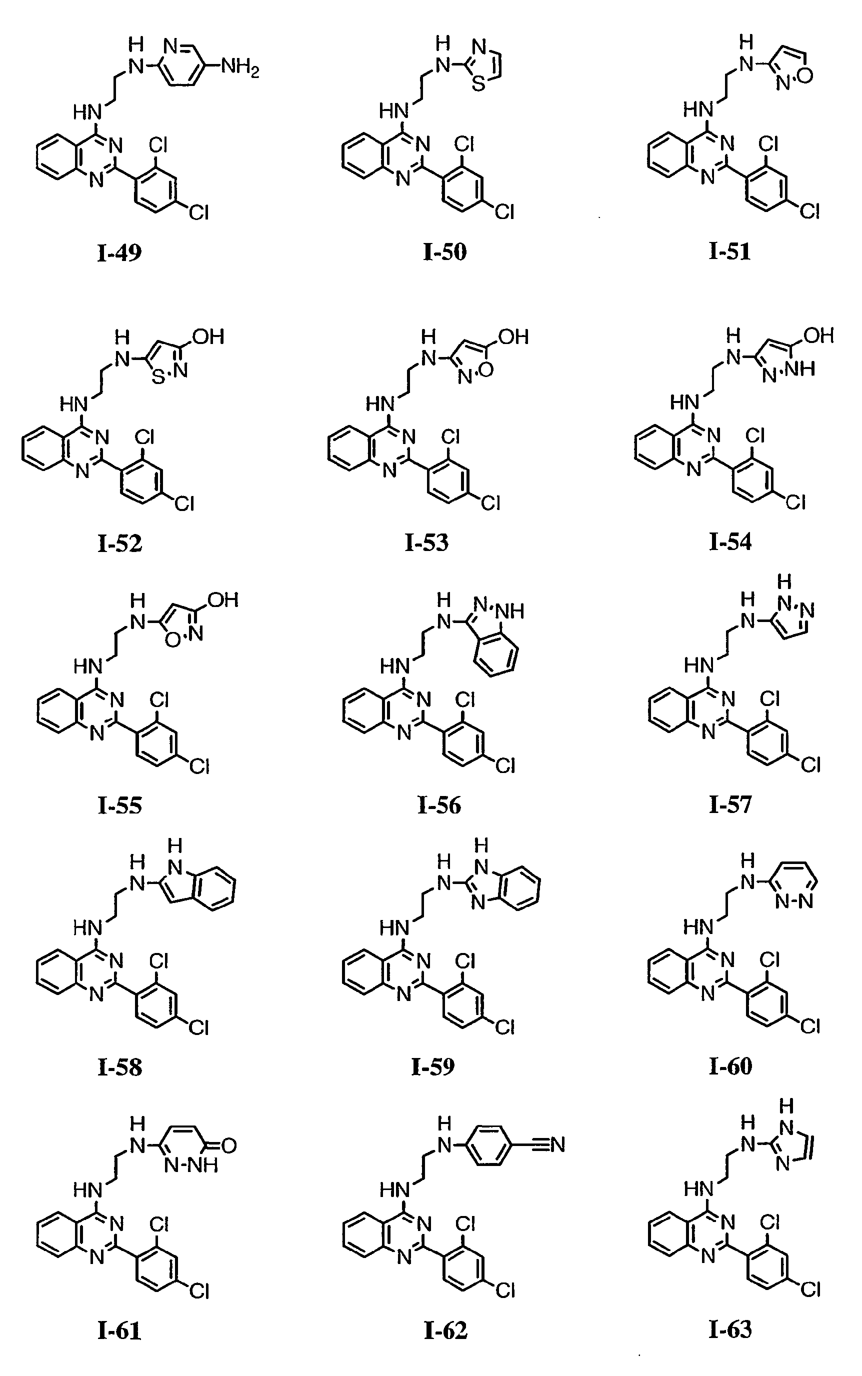

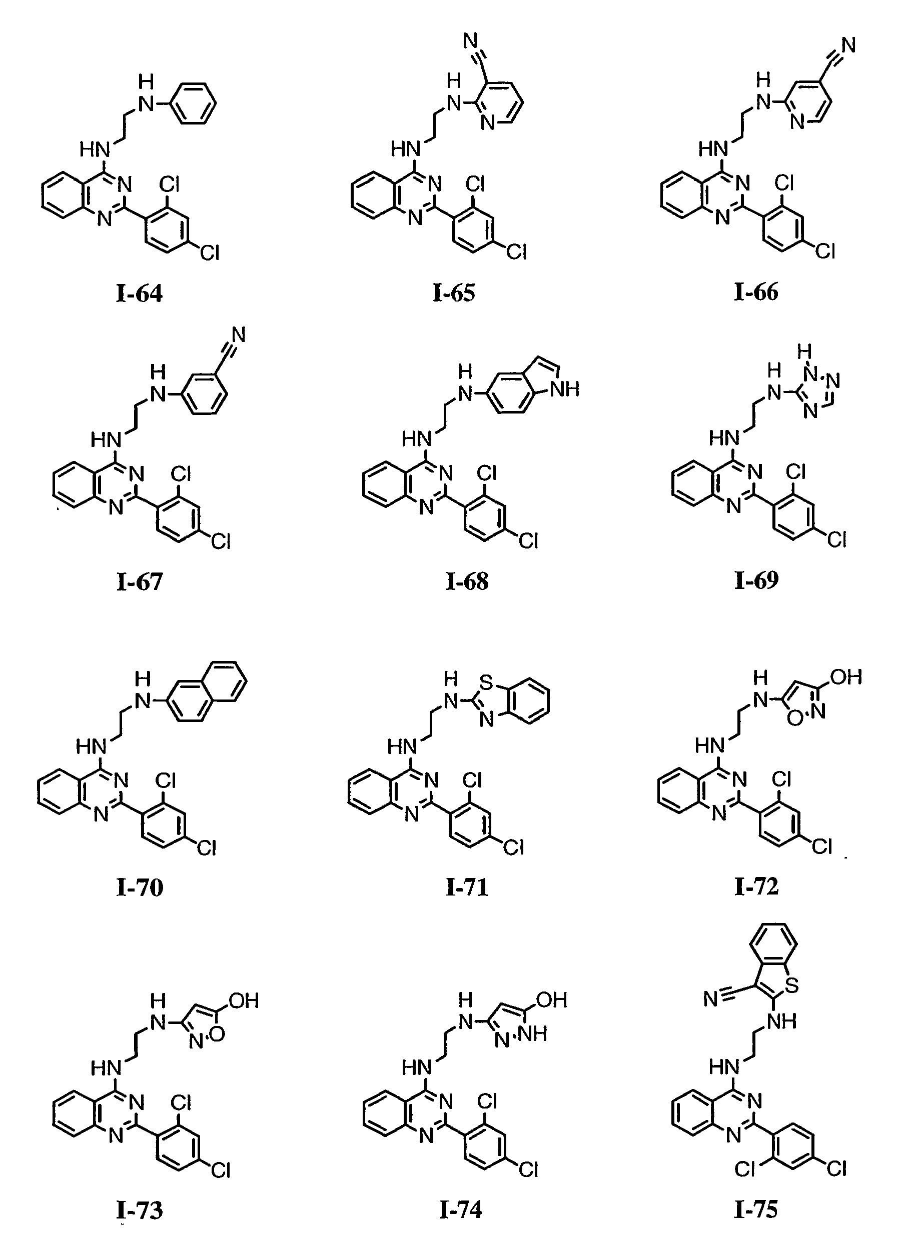

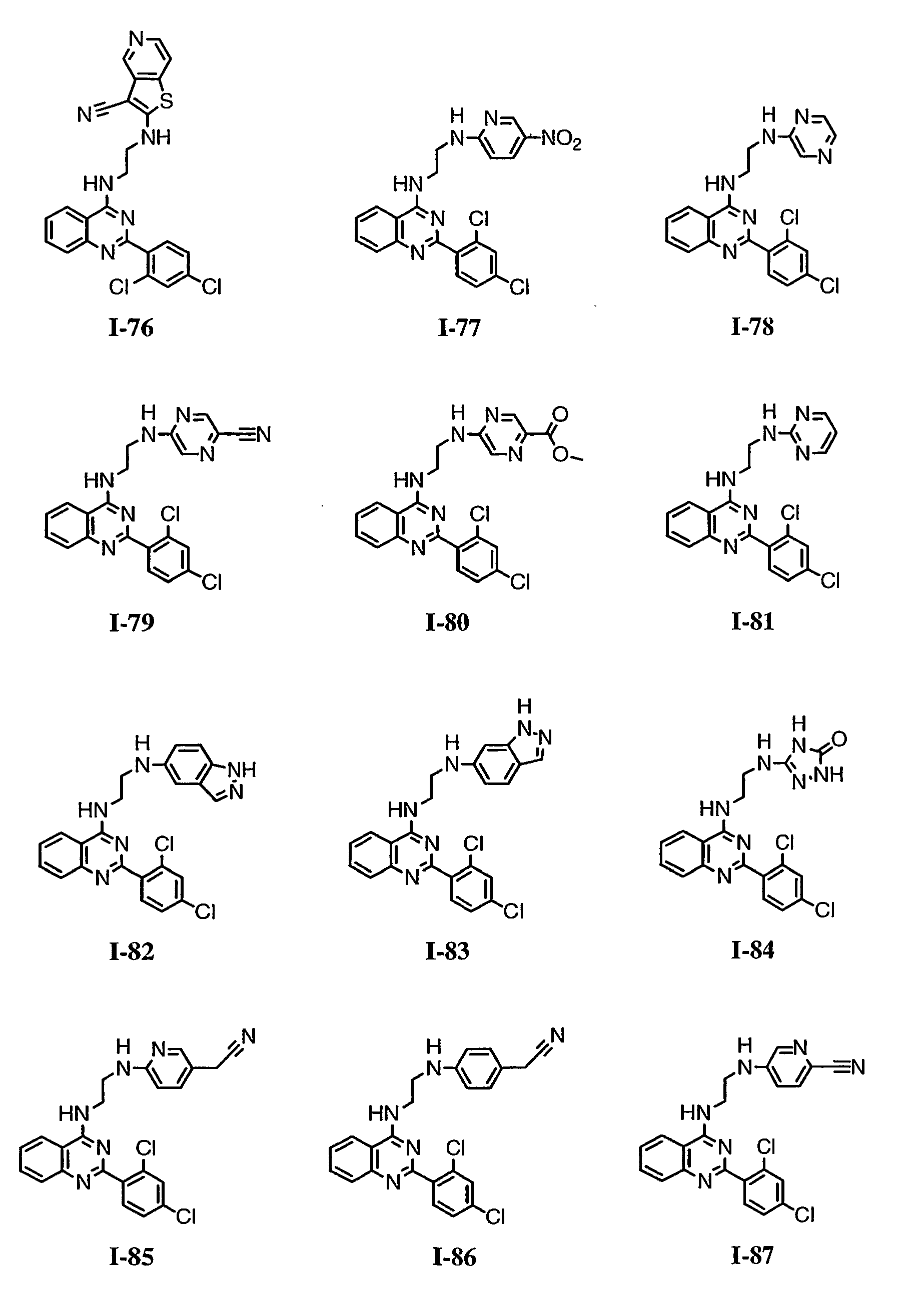

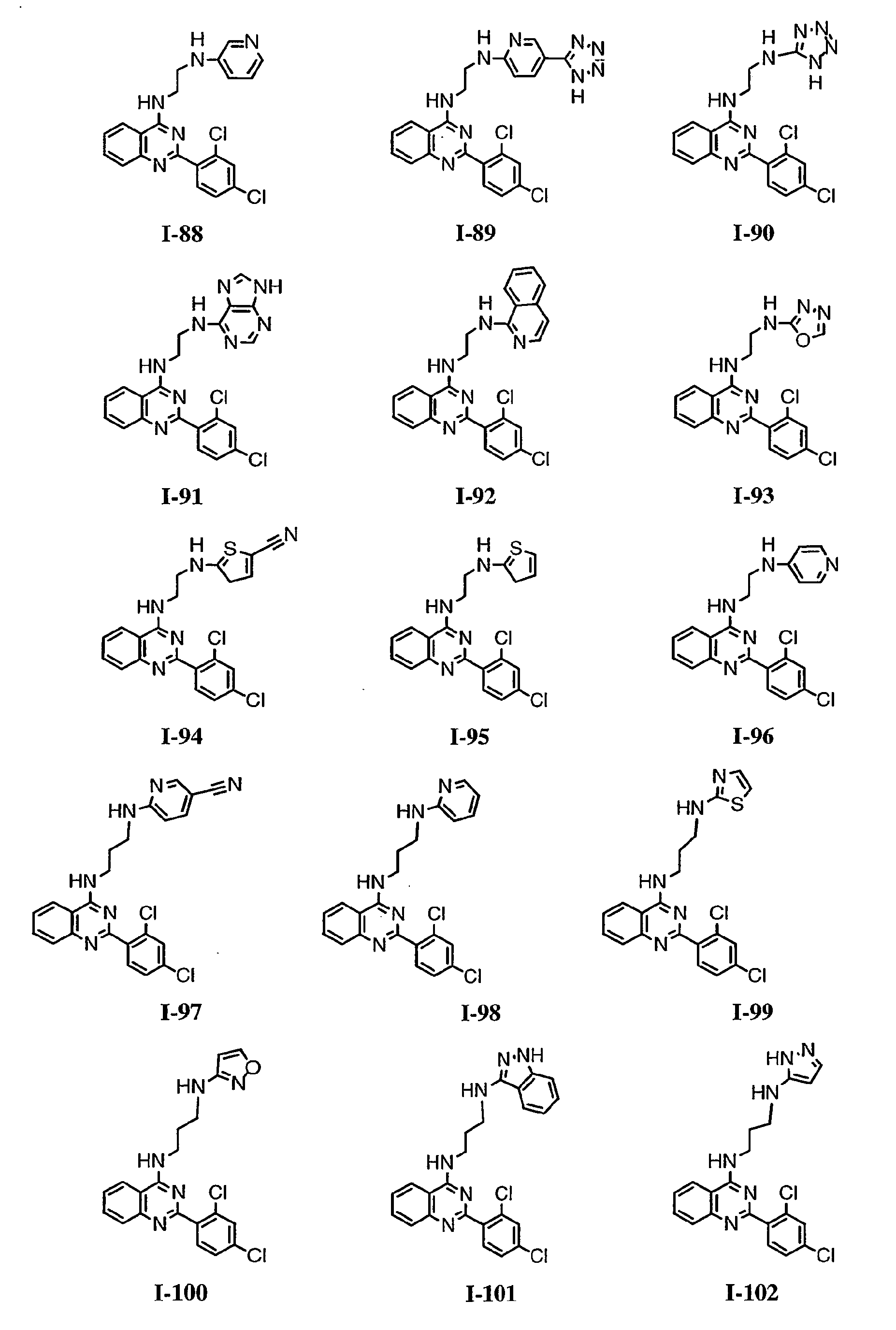

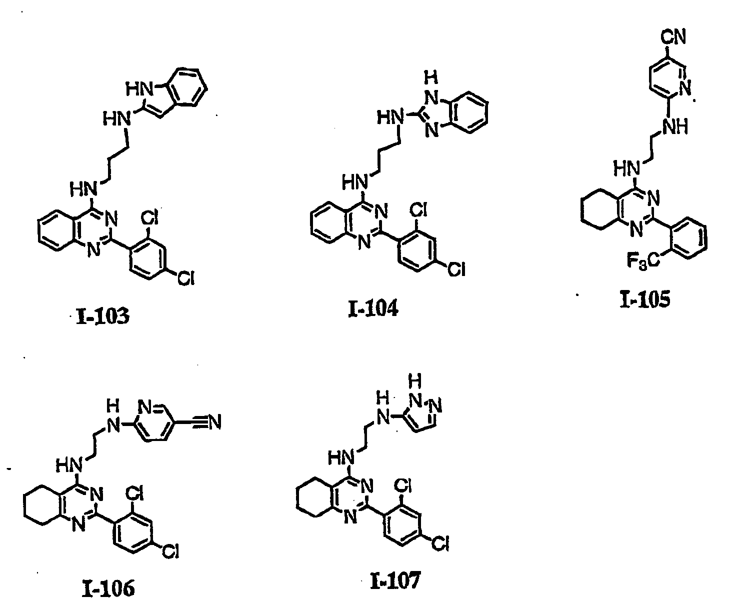

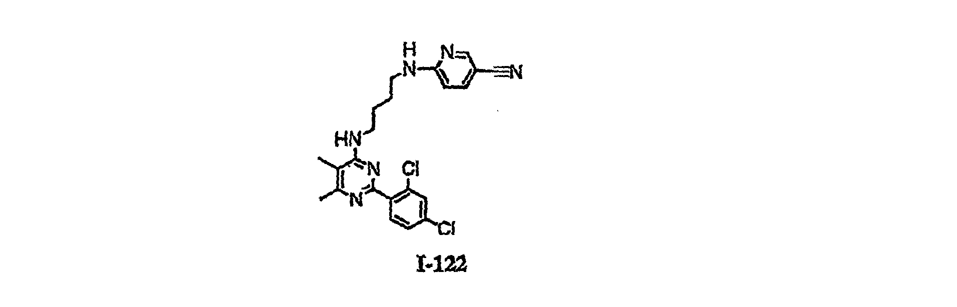

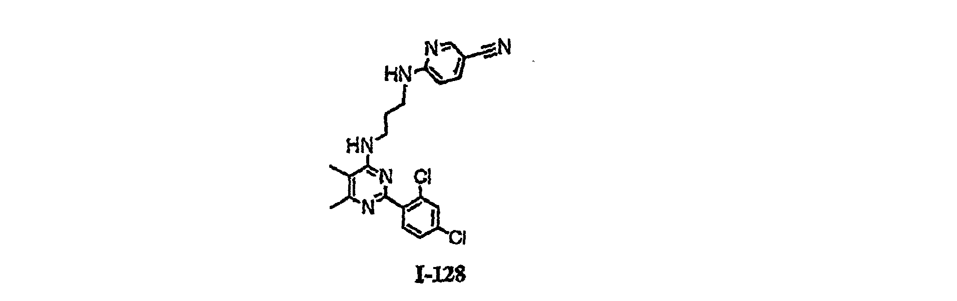

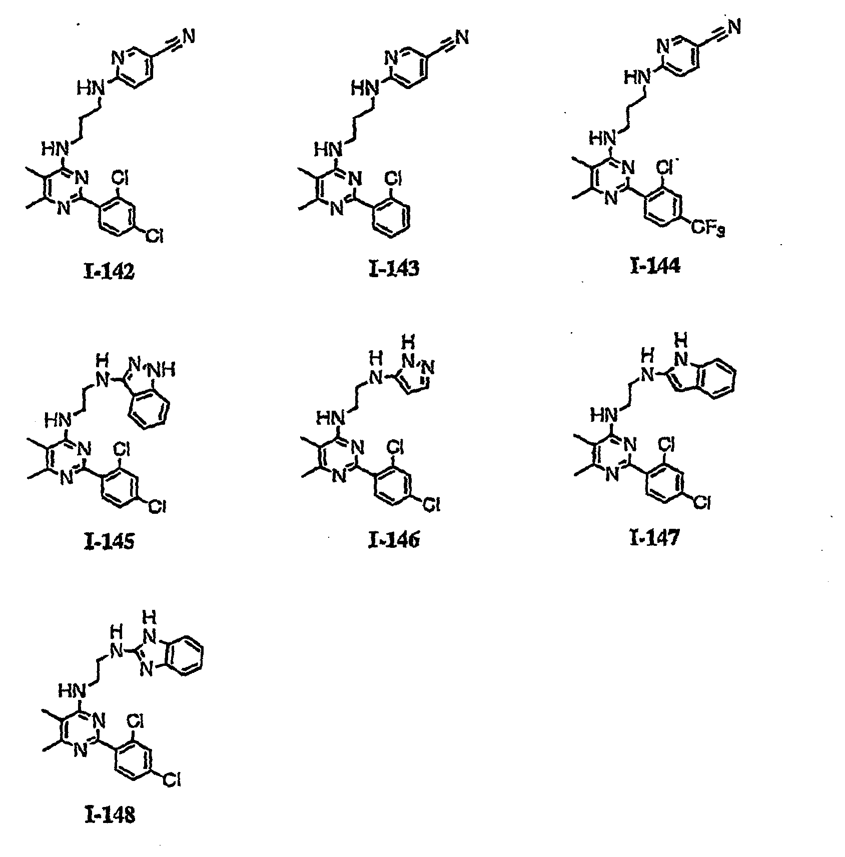

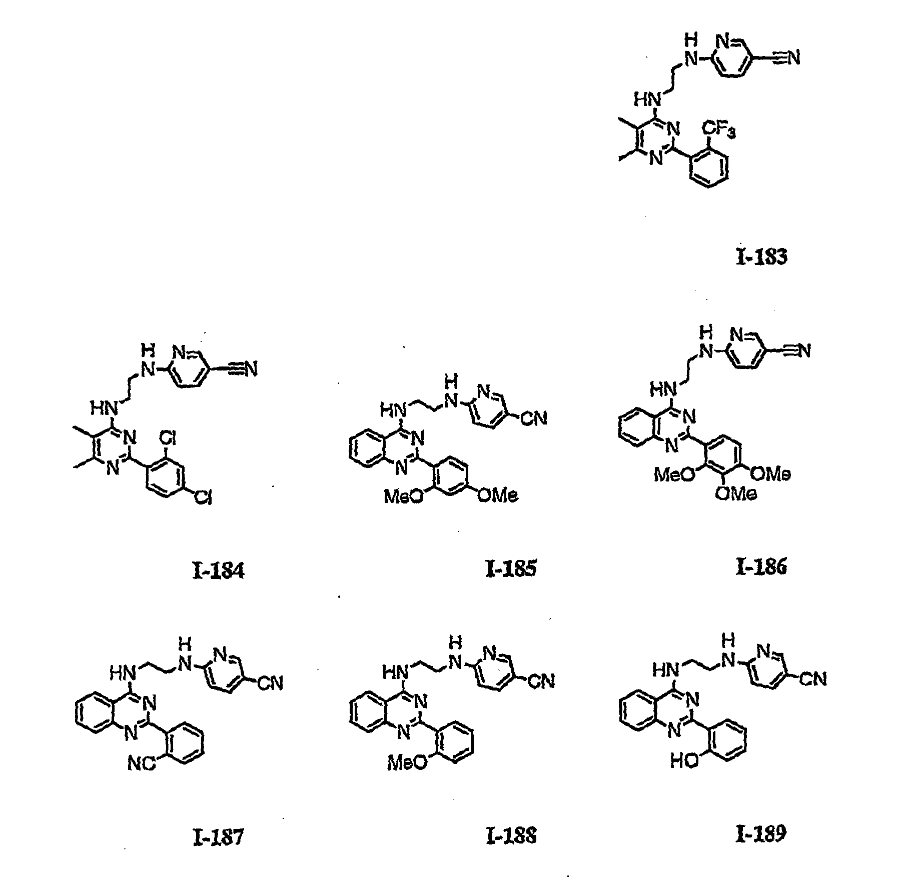

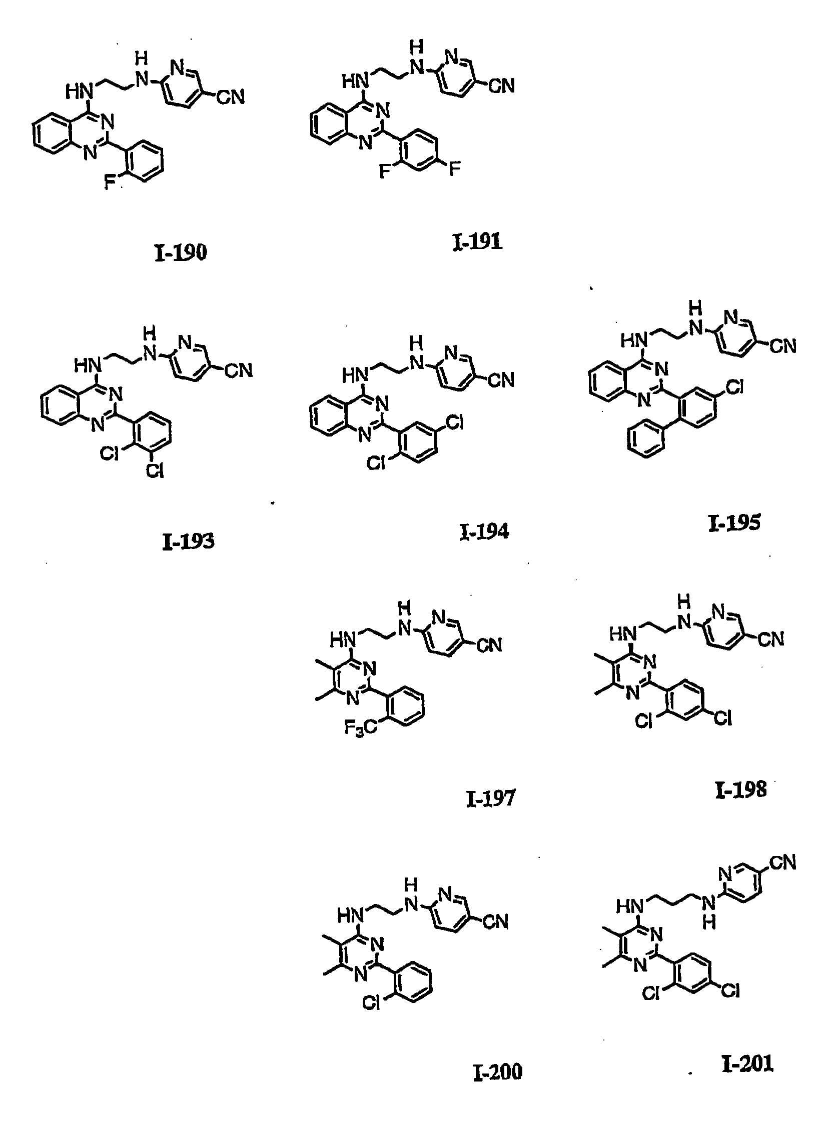

*III*. *General Synthetic Methodology:*

The compounds of this invention may be prepared in general by methods known to those skilled in the art for analogous compounds, as illustrated by the general scheme below, and the preparative examples that follow.

Although certain exemplary embodiments are depicted and described above and herein, it will be appreciated that a compounds of the invention can be prepared according to the methods described generally above using appropriate starting materials.

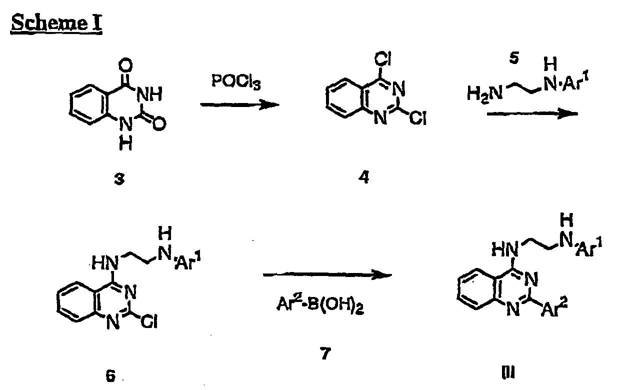

Scheme I shows a general approach for making compounds of formula **III.** In Scheme I, Ar¹ is a synthetic equivalent of B in formula I and Ar² is a synthetic equivalent of Ring C in formula **I.** Preparation of the dichloropyrimidine **4** may be achieved in a manner similar to that described in Chem. Pharm. Bull., 30, 3121-3124 (1982). The chlorine in position **4** of intermediate **4** may be replaced by a diamine **5** to provide intermediate **6** in a manner similar to that described in J. Med. Chem., 38, 3547-3557 (1995). The diamine **5** may be either commercially available, readily synthesized via methods known in the art such as those described in WO 99/65897 (Chiron), or provided by methods disclosed in the synthetic examples. Although an example where Q is -CH₂CH₂- is depicted above, it will be appreciated that additional compounds where Q is described generally for formula **I** can also be prepared according to the methods cited above. Ring C is then introduced using a boronic ester **7** under palladium catalysis [see Tetrahedron, 48, 8117-8126 (1992)]. This method is illustrated by the following procedure.

A suspension of 1*H*-quinazoline-2,4-dione **3** (10.0 g, 61.7 mmol) in POCl₃ (60 mL, 644 mmol) and N,N-dimethylaniline (8mL, 63.1 mmol) is heated under reflux for 2 h. Excess POCl₃ is evaporated under vacuum, the residue is poured into ice, and the precipitate is collected by filtration. The crude solid 2,4-dichloroquinazoline product **4** may be used without further purification.

To a solution of 2,4-dichloro-quinazoline **4** (3.3 g, 16.6 mmol) in anhydrous ethanol (150 mL) is added a diamine **5** (32.9 mmol). The mixture is stirred at room temperature for 4 hours, and the resulting precipitate is collected by filtration, washed with ethanol, and dried under vacuum to afford intermediate **6.**

To a solution of intermediate **6** (0.19 mmol) in DMF (1.0 mL) is added the desired boronic acid **7** (0.38 mmol), 2M Na₂CO₃ (0.96 mmol), and tri-t-butylphosphine (0.19 mmol). Under nitrogen, PdCl₂(dppf) (0.011 mmol) is added in one portion. The reaction mixture is then heated at 80°C for 5 to 10 hours, cooled to room temperature, and poured into water (2 mL). The resulting precipitate is collected by filtration, washed with water, and purified by HPLC.

Compounds where one methylene unit of Q is replaced by SO₂ can be prepared according to the general methods depicted in Schemes 2, 3, 4 and 5 below. Scheme 2 illustrates a general method for the preparation of compounds of formula **13.** Although compounds where Ring A is quinazolinyl are depicted below, it will be appreciated that compounds of general formula **I** (for example compounds having other ring systems as described generally herein) can also be prepared according to these methods.

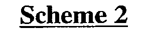

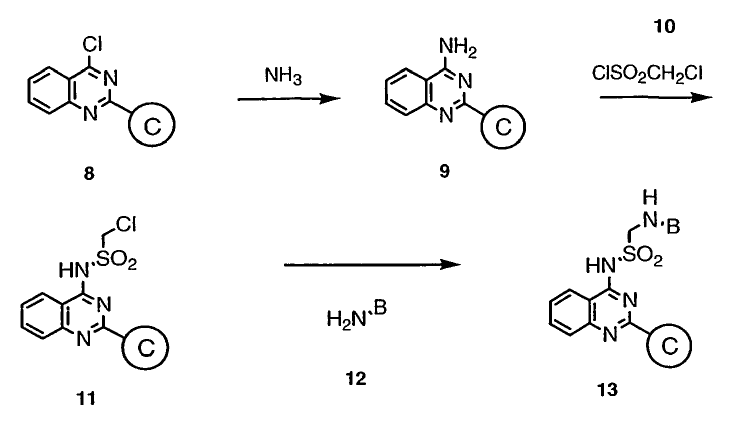

Scheme 3 depicts the preparation of certain exemplary compounds where ring C is a substituted phenyl moiety, and B is a substituted pyridyl moiety.

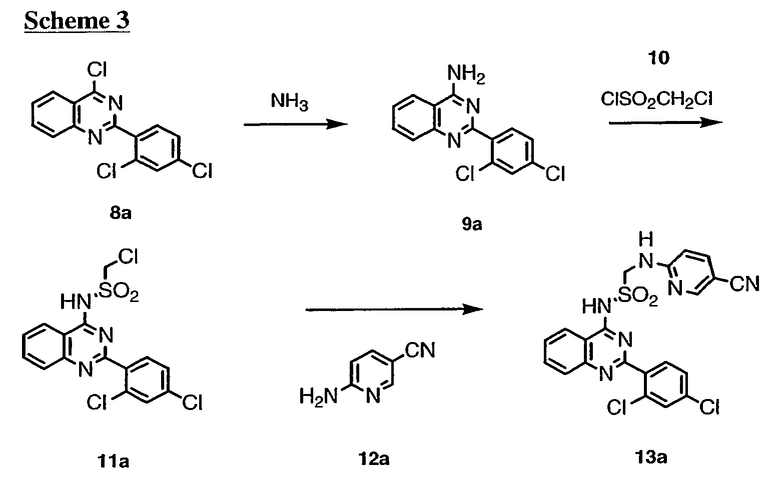

Scheme 4 illustrates a general method for the preparation of compounds of formula **16.** Although compounds where Ring A is quinazolinyl are depicted below, it will be appreciated that compounds of general formula **I** (for example compounds having other ring systems as described generally herein) can also be prepared according to these methods.

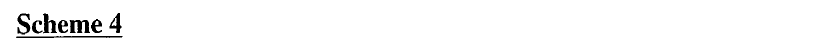

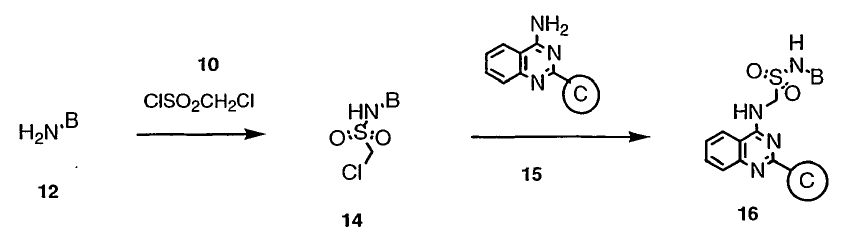

Scheme 5 depicts the preparation of certain exemplary compounds where ring C is a substituted phenyl moiety, and B is a substituted pyridyl moiety.

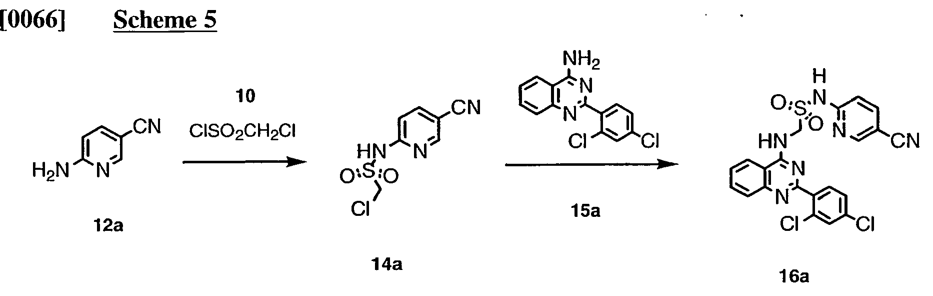

One having ordinary skill in the art may synthesize other compounds of this invention following the teachings of the specification using reagents that are readily synthesized or commercially available.

*IV*. *Uses of Compounds of the Invention:*

The activity of a compound utilized in this invention as an inhibitor of GSK-3 kinase may be assayed *in vitro, in vivo* or in a cell line according to methods known in the art. *In vitro* assays include assays that determine inhibition of either the phosphorylation activity or ATPase activity of activated GSK-3. Alternate *in vitro* assays quantitate the ability of the inhibitor to bind to GSK-3. Inhibitor binding may be measured by radiolabelling the inhibitor prior to binding, isolating the inhibitor/GSK-3 complex and determining the amount of radiolabel bound. Alternatively, inhibitor binding may be determined by running a competition experiment where new inhibitors are incubated with GSK-3 bound to known radioligands. Detailed conditions for assaying a compound utilized in this invention as an inhibitor of GSK-3 kinase are set forth in the Examples below.

According to another embodiment, the invention provides a composition comprising a compound of this invention or a pharmaceutically acceptable derivative thereof and a pharmaceutically acceptable carrier, adjuvant, or vehicle. Preferably, the amount of compound in the compositions of this invention is such that is therapeutically effective, and most preferably effective to detectably inhibit a protein kinase, particularly GSK-3 kinase, in a biological sample or in a patient. Preferably the composition of this invention is formulated for administration to a patient in need of such composition. Most preferably, the composition of this invention is formulated for oral administration to a patient.

The term "patient", as used herein, means an animal, preferably a mammal, and most preferably a human.

The term "pharmaceutically acceptable carrier, adjuvant, or vehicle" refers to a non-toxic carrier, adjuvant, or vehicle that does not destroy the pharmacological activity of the compound with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The term "detectably inhibit", as used herein means a measurable change in GSK-3 activity between a sample comprising said composition and a GSK-3 kinase and an equivalent sample comprising GSK-3 kinase in the absence of said composition.

Pharmaceutically acceptable salts of the compounds of this invention include those derived from pharmaceutically acceptable inorganic and organic acids and bases. Examples of suitable acid salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, formate, fumarate, glucoheptanoate, glycerophosphate, glycolate, hemisulfate, heptanoate, hexanoate, hydrochloride, hydrobromide, hydroiodide, 2-hydroxyethanesulfonate, lactate, maleate, malonate, methanesulfonate, 2-naphthalenesulfonate, nicotinate, nitrate, oxalate, palmoate, pectinate, persulfate, 3-phenylpropionate, phosphate, picrate, pivalate, propionate, salicylate, succinate, sulfate, tartrate, thiocyanate, tosylate and undecanoate. Other acids, such as oxalic, while not in themselves pharmaceutically acceptable, may be employed in the preparation of salts useful as intermediates in obtaining the compounds of the invention and their pharmaceutically acceptable acid addition salts.

Salts derived from appropriate bases include alkali metal (e.g., sodium and potassium), alkaline earth metal (e.g., magnesium), ammonium and N⁺(C₁₋₄ alkyl)₄ salts. This invention also envisions the quaternization of any basic nitrogen-containing groups of the compounds disclosed herein. Water or oil-soluble or dispersible products may be obtained by such quaternization.

The compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The pharmaceutically acceptable compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, the pharmaceutically acceptable compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

The pharmaceutically acceptable compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, the pharmaceutically acceptable compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutically acceptable compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutically acceptable compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutically acceptable compositions may be formulated in an ointment such as petrolatum.

The pharmaceutically acceptable compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Most preferably, the pharmaceutically acceptable compositions of this invention are formulated for oral administration.

The amount of the compounds of the present invention that may be combined with the carrier materials to produce a composition in a single dosage form will vary depending upon the host treated, the particular mode of administration. Preferably, the compositions should be formulated so that a dosage of between 0.01-100 mg/kg body weight/day of the inhibitor can be administered to a patient receiving these compositions.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of a compound of the present invention in the composition will also depend upon the particular compound in the composition.

Depending upon the particular condition, or disease, to be treated or prevented, additional therapeutic agents, which are normally administered to treat or prevent that condition, may also be present in the compositions of this invention. As used herein, additional therapeutic agents that are normally administered to treat or prevent a particular disease, or condition, are known as "appropriate for the disease, or condition, being treated".

For example, chemotherapeutic agents or other anti-proliferative agents may be combined with the compounds of this invention to treat proliferative diseases and cancer. Examples of known chemotherapeutic agents include, but are not limited to, Gleevec^{™}, adriamycin, dexamethasone, vincristine, cyclophosphamide, fluorouracil, topotecan, taxol, interferons, and platinum derivatives.

Other examples of agents the inhibitors of this invention may also be combined with include, without limitation: treatments for Alzheimer's Disease such as Aricept^{®} and Excelon^{®}; treatments for Parkinson's Disease such as L-DOPA/carbidopa, entacapone, ropinrole, pramipexole, bromocriptine, pergolide, trihexephendyl, and amantadine; agents for treating Multiple Sclerosis (MS) such as beta interferon (e.g., Avonex^{®} and Rebif^{®}), Copaxone^{®}, and mitoxantrone; treatments for asthma such as albuterol and Singulair^{®}; agents for treating schizophrenia such as zyprexa, risperdal, seroquel, and haloperidol; anti-inflammatory agents such as corticosteroids, TNF blockers, IL-1 RA, azathioprine, cyclophosphamide, and sulfasalazine; immunomodulatory and immunosuppressive agents such as cyclosporin, tacrolimus, rapamycin, mycophenolate mofetil, interferons, corticosteroids, cyclophophamide, azathioprine, and sulfasalazine; neurotrophic factors such as acetylcholinesterase inhibitors, MAO inhibitors, interferons, anti-convulsants, ion channel blockers, riluzole, and anti-Parkinsonian agents; agents for treating cardiovascular disease such as beta-blockers, ACE inhibitors, diuretics, nitrates, calcium channel blockers, and statins; agents for treating liver disease such as corticosteroids, cholestyramine, interferons, and anti-viral agents; agents for treating blood disorders such as corticosteroids, anti-leukemic agents, and growth factors; agents for treating immunodeficiency disorders such as gamma globulin; and agents for treating diabetes such as insulin or insulin analogues, in injectable or inhalation form, glitazones, alpha glucosidase inhibitors, biguanides, insulin sensitizers, and sulfonyl ureas.

The amount of additional therapeutic agent present in the compositions of this invention will be no more than the amount that would normally be administered in a composition comprising that therapeutic agent as the only active agent. Preferably the amount of additional therapeutic agent in the presently disclosed compositions will range from about 50% to 100% of the amount normally present in a composition comprising that agent as the only therapeutically active agent.

In an alternate embodiment, the methods of this invention that utilize compositions that do not contain an additional therapeutic agent, comprise the additional step of separately administering to said patient an additional therapeutic agent. When these additional therapeutic agents are administered separately they may be administered to the patient prior to, sequentially with or following administration of the compositions of this invention.

According to another embodiment, the invention relates to a method of inhibiting GSK-3 kinase activity in a biological sample comprising the step of contacting said biological sample with a compound of this invention, or a composition comprising said compound.

The term "biological sample", as used herein, includes, without limitation, cell cultures or extracts thereof; biopsied material obtained from a mammal or extracts thereof; and blood, saliva, urine, feces, semen, tears, or other body fluids or extracts thereof.

Inhibition of GSK-3 kinase activity in a biological sample is useful for a variety of purposes that are known to one of skill in the art. Examples of such purposes include, but are not limited to, blood transfusion, organ-transplantation, biological specimen storage, and biological assays.

According to another embodiment, the invention provides a method for treating or lessening the severity of a GSK-3-mediated disease or condition in a patient comprising the step of administering to said patient a composition according to the present invention.

The term "GSK-3-mediated disease" as used herein, means any disease or other deleterious condition or disease in which GSK-3 is known to play a role. Such diseases or conditions include, without limitation, autoimmune diseases, inflammatory diseases, metabolic, neurological and neurodegenerative diseases, cardiovascular diseases, allergy, asthma, diabetes, Alzheimer's disease, Huntington's disease, Parkinson's disease, AIDS-associated dementia, amyotrophic lateral sclerosis (AML, Lou Gehrig's disease), multiple sclerosis (MS), schizophrenia, cardiomyocyte hypertrophy, reperfusion/ischemia, stroke, and baldness.

According to a preferred embodiment, the method of the present invention relates to treating or lessening the severity of stroke.

According to another preferred embodiment, the method of the present invention relates to treating or lessening the severity of a neurodegenerative or neurological disorder.

In other embodiments, the invention relates to a method of enhancing glycogen synthesis and/or lowering blood levels of glucose in a patient in need thereof, comprising administering to said patient a therapeutically effective amount of a composition comprising a compound of formula I. This method is especially useful for diabetic patients.

In yet another embodiment, the invention relates to a method of inhibiting the production of hyperphosphorylated Tau protein in a patient in need thereof, comprising administering to said patient a therapeutically effective amount of a composition comprising a compound of formula **I**. This method is especially useful in halting or slowing the progression of Alzheimer's disease.

In still another embodiments, the invention relates to a method of inhibiting the phosphorylation of β-catenin in a patient in need thereof, comprising administering to said patient a therapeutically effective amount of a composition comprising a compound of formula I. This method is especially useful for treating schizophrenia.

The compounds of this invention or pharmaceutically acceptable compositions thereof may also be incorporated into compositions for coating an implantable medical device, such as prostheses, artificial valves, vascular grafts, stents and catheters. Vascular stents, for example, have been used to overcome restenosis (re-narrowing of the vessel wall after injury). However, patients using stents or other implantable devices risk clot formation or platelet activation. These unwanted effects may be prevented or mitigated by pre-coating the device with a pharmaceutically acceptable composition comprising a kinase inhibitor. Suitable coatings and the general preparation of coated implantable devices are described in US Patents 6,099,562; 5,886,026; and 5,304,121. The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccarides, polyethylene glycol, phospholipids or combinations thereof to impart controlled release characteristics in the composition. Implantable devices coated with a compound of this invention are another embodiment of the present invention.

In order that the invention described herein may be more fully understood, the following examples are set forth. It should be understood that these examples are for illustrative purposes only and are not to be construed as limiting this invention in any manner.

### SYNTHETIC EXAMPLES

The following HPLC methods were used in the analysis of the compounds as specified in the Synthetic Examples set forth below.

HPLC conditions

A)LC-MS: Column: Waters (YMC) ODS-AQ 2.0x50mm, S5, 120A. Gradient: 90% water (0.2% Formic acid), 10% acetonitrile (containing 0.1% Formic acid) to 10% water (0.1% formic acid), 90% acetonitrile (containing 0.1% formic acid) over 5.0 min, hold for 0. min and return to initial conditions. Total run time 7.0 min. Flow rate: 1.0 mL/min

HPLC: Column: C18, 3 um, 2.1 X 50 mm, "Lighting" by Jones Chromatography. Gradient: 100% water (containing 1% acetonitrile, 0.1% TFA) to 100% acetonitrile (containing 0.1% TFA) over 4.0 min, hold at 100% acetonitrile for 1.4 min and return to initial conditions. Total run time 7.0

Example 1 **6-{2-[2-(2-Trifluoromethyl-phenyl)-5,6,7,8-tetrahydro-quinazolin-4-ylamino]-ethylamino}-nicotinonitrile** (Compound I-105)

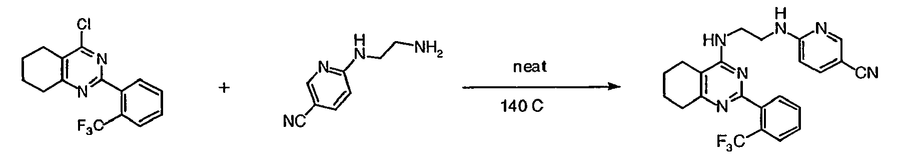

A mixture of 4-chloro-2-(2-trifluoromethyl-phenyl)-5,6,7,8-tetrahydroquinazoline and 6-(2-amino-ethylamino)-nicotinonitrile (prepared according to the method described by Nuss et al, WO 99/65997) was dissolved in ethanol (2 mL). The solvent was removed under a stream of nitrogen gas and the residue was heated at 140°C for 18 hours. Purification was achieved by silica gel chromatography (1:1 hexane/ethyl acetate, 1:2 hexane/ethyl acetate) to yield the title compound as a white solid (35 mg, 50%).

¹H NMR (500 MHz, d₆-DMSO, ppm) δ8.35 (s, 1H), 7.8 (d, 1H), 7.73-7.65 (m, 2H), 7.65-7.6 (m, 2H), 5.7 (br s, 1H), 6.9 (br s, 1H), 6.57-6.47 (m, 1H), 3.58-3.52 (m, 2H), 3.52-3.42 (m, 2H), 2.64-2.58 (m, 2H), 2.4-2.34 (m, 2H), and 1.83-1.75 (m, 4H); LC-MS (ES+) m/e= 439.18 (M+H); Rₜ= 2.06 min

Example 2 **6-{2-[2-(Trifluoromethyl-phenyl)-quinazolin-4-ylamino]-ethylamino}-nicotinonitrile** (Compound I-8)

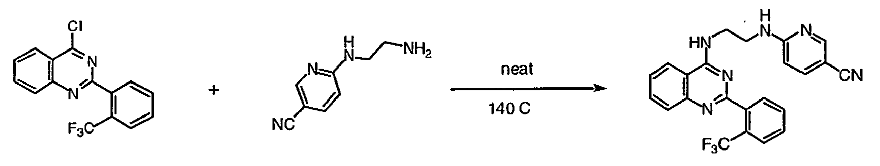

This compound was prepared in an analogous manner as described in Example 1 using 4-chloro-2-(2-trifluoromethyl-phenyl)-quinazoline. Purification was achieved by silica gel chromatography (1:1 hexane/ethyl acetate, 1:2 hexane/ethyl acetate) to yield the title compound (34 mg, 49%).

¹H NMR (500 MHz, d₆-DMSO, ppm) δ8.58 (br s, 1H), 8.37 (s, 1H), 8.28 (d, 1H), 7.85-7.74 (m, 6H), 7.7-7.66 (m, 1H), 7.6-7.55 (m, 2H), 6.53 (br s, 1H), 3.77-3.72 (m, 2H), 3.65-3.57 (m, 2H); LC-MS (ES+) m/e= 435.09
(M+H); Rₜ= 2.09 min.

Example 3 **6-[2-(2-Phenyl-quinazolin-4-ylamino)-ethylamino]-nicotinonitrile** (Compound I-9)

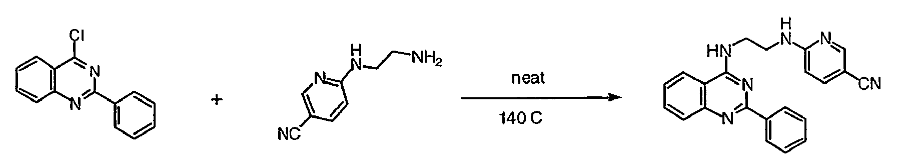

This compound was prepared in an analogous manner as described in Example 1 using 4-chloro-2-phenyl-quinazoline. Purification was achieved by silica gel chromatography (2:1 hexane/ethyl acetate, 1:1 hexane/ethyl acetate) to yield the title compound as a white solid (37 mg, 49%).

¹H NMR (500 MHz, d₆-DMSO, ppm) δ8.52-8.4 (m, 4H), 8.23 (d, 1H), 7.88-7.82 (m, 1H), 7.78-7.71 (m, 2H), 7.66-7.6 (m, 1H), 7.52-7.43 (m, 4H), 6.58-6.49 (m, 1H), 3.9-3.84 (m, 2H), 3.83-3.65 (m, 2H); LC-MS (ES+) m/e= 367.08 (M+H); Rₜ= 1.91 min.

Example 4 **6-{2-[2-(2,4-Dichloro-phenyl)-quinazolin-4-ylamino]-ethylamino}-nicotinonitrile** (Compound I-1)

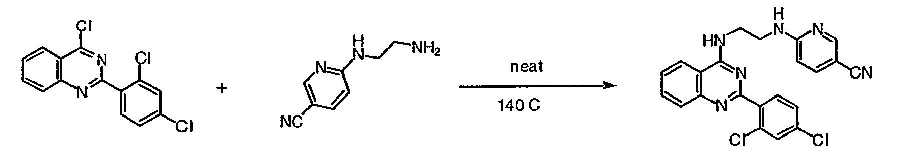

This compound was prepared in an analogous manner as described in Example 1 using 4-chloro-2-(2,4-dichloro-phenyl)-quinazoline and 6-(2-amino-ethylamino)-nicotinonitrile. Purification was achieved by silica gel chromatography (2:1 hexane/ethyl acetate, 1:1 hexane/ethyl acetate) to yield the title compound as a white solid (36 mg, 51%).

¹H NMR (500 MHz, d₆-DMSO, ppm) δ8.64-8.56 (m, 1H), 8.37 (s, 1H), 8.31-8.24 (m, 1H), 7.84-7.69 (m, 5H), 7.62-7.50 (m, 3H), 6.59-6.48 (m, 1H), 3.80-3.70 (m, 2H), 3.72-3.59 (m, 2H); LC-MS (ES+) m/e= 434.96 (M+H); Rₜ= 2.18 min.

Example 5 **6-{2-[7-Benzyl-2-(2-trifluoromethyl-phenyl)-5,6,7,8-tetrahydro-pyrido[3,4-*d*]pyrimidin-4-ylamino]-ethylamino}-nicotinonitrile** (Compound I-113)

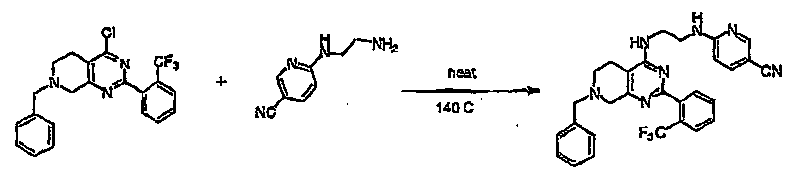

This compound was prepared in an analogous manner as described in Example 1 using 7-benzyl-4-chloro-2-(2-trifluoromethyl-phenyl)-5,6,7,8-tetrahydro-pyrido[3,4-*d*]pyrimidine and 6-(2-amino-ehylamino)-nicotinonitrile. Purification was achieved by silica gel chromatography (1:3 hexane/ethyl acetate, 1:4 hexane/ethyl acetate) to yield the title compound as a yellow foam (76 mg, 58%).

¹H NMR (500 MHz, CDCl₃, ppm) δ8.30 (s, 1H), 7.75 (d, 1H), 7.69-7.65 (m, 1H), 7.62 (t, 1H), 7.60-7.52 (m, 1H), 7.43-7.27 (m, 6H), 6.11-5.98 (m, 2H), 5.33 (br s, 1H), 3.84-3.73 (m, 4H), 3.66-3.57 (m, 4H), 2.92-2.82 (m, 2H), 2.53-2.42 (m, 2H); LC-MS (ES+) m/e= 530.05 (M+H); Rₜ= 2.29 min.

Example 7 **6-{2-[2-(2-Chloro-phenyl)-quinazolin-4-ylamino]-ethylamino}-nicotinonitrile** (Compound I-11)

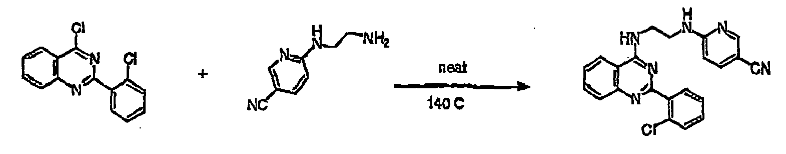

This compound was prepared in an analogous manner as described in Example 1 using 4-chloro-2-(2-chloro-phenyl)-quinazoline. Purification was achieved by silica gel chromatography (1:1 hexane/ethyl acetate, 1:2 hexane/ethyl acetate)to yield the title compound as a white solid (47 mg, 64%).

¹H NMR (500 MHz, d₆-DMSO, ppm) δ8.60-8.55 (m, 1H), 8.39 (s, 1H), 8.26 (d, 1H), 7.84-7.78 (m, 2H), 7.76 (d, 1H), 7.71-7.69 (m, 1H), 7.65-7.52 (m, 3H), 7.50-7.42 (m, 2H), 6.61-6.46 (m, 1H), 3.77-3.59 (m, 4H); LC-MS (ES+) m/e= 401.03 (M+H); Rₜ= 1.89 min.

Example 8 **6-{2-[2-(2,4-Dimethoxy-phenyl)-quinazolin-4-ylamino]-ethylamino}-nicotinonitrile** (Compound I-185)

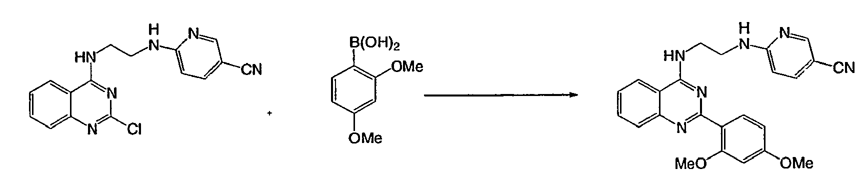

A mixture of 6-[2-(2-Chloro-quinazolin-4-ylamino)-ethylamino]-nicotinonitrile (65mg, 0.2 mmol), 2,4-dimethoxyphenylboronic acid (55mg, 0.3 mmol), tetrakis(triphenylphosphine)palladium (2mg) and aqueous saturated NaHCO₃ (0.5 ml) in 1,2-dimethoxyethane (3 ml) was stirred at 110°C under nitrogen for 14 h. The reaction mixture was concentrated. The residue was dissolved in a mixture of DMSO and MeOH and purified by reverse-phase HPLC. The title compound was obtained as a white solid (76 mg, 69%). ¹H NMR (500 MHz, DMSO(d₆)): δ13.20 (s, 1H), 10.20 (m, 1H), 8.38 (d, 2H), 8.00 (m, 2H), 7.82 (m, 1H), 7.78 (d, 2H), 7.72 (t, 1H), 6.80 (s, 1H), 6.70 (d, 1H), 3.90 (m, 7H), 3.83 (s, 3H). FIA-MS: (ES-) m/e= 425.3 (M-H), (ES+) m/e= 427.2 (M+H).

Example 9 **6-{2-[2-(4-Methoxy-phenyl)-quinazolin-4-ylamino]-ethylamino}-nicotinonitrile** (compound I-3)

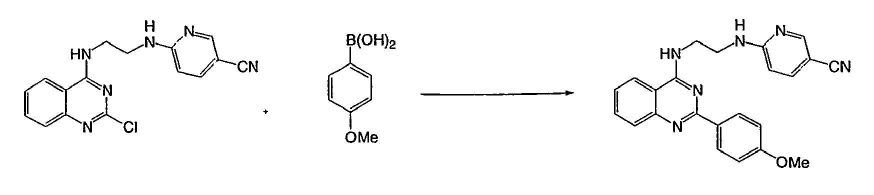

This compound was prepared in an analogous manner as described in Example 8 using 4-methoxyphenylboronic acid. Purification was achieved on silica gel, eluting with 40-50% EtOAc in hexanes to give the tit le compound as a white solid (18.5mg, 30%).

¹H NMR (500 MHz, d₆-DMSO, ppm) δ8.57-8.33 (m, 5H), 8.18 (d, 1H), 7.92-7.78 (m, 1H), 7.77-7.58 (m, 3H), 7.51-7.37 (m, 1H), 7.01 (d, 2H), 6.59-6.40 (m, 1H), 3.90-3.80 (m, 5H), 3.75-3.62 (m, 2H). LC-MS (ES+) m/e= 397.06 (M+H), (ES-) 395.11 (M-H); Rₜ= 2.10 min.

Example 10 **6-{2-[2-(2,3,4-Trimethoxy-phenyl)-quinazolin-4-ylamino]-ethylamino}-nicotinonitrile** (Compound I-186)

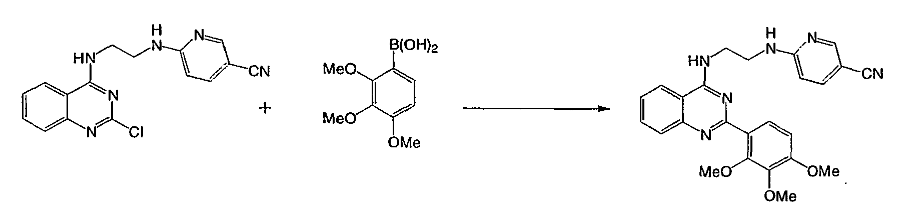

This compound was prepared in an analogous manner to described in Example 8 using 2,3,4-trimethoxyphenyl boronic acid. Purification was achieved on silica gel, eluting with 50-60% EtOAc in hexanes to give the title compound as a white solid (13mg, 18%).

¹H NMR (500 MHz, d₆-DMSO, ppm) δ 8.39 (br s, 2H), 8.29-8.16 (m, 1H), 7.87-7.65 (m, 3H), 7.64-7.55 (m, 1H), 7.54-7.45 (m, 1H), 7.39 (d, 1H), 6.87 (d, 1H), 6.61-6.39 (br, 1H), 3.85 (d, 6H), 3.78 (s, 5H), 3.69-3.56 (m, 2H). LC-MS (ES+) m/e= 457.3 (M+H); Rₜ= 2.36 min.

Example 11 **6-{2-[2-(2-Cyano-phenyl)-quinazolin-4-ylamino]-ethylamino}-nicotinonitrile** (Compound I-187)

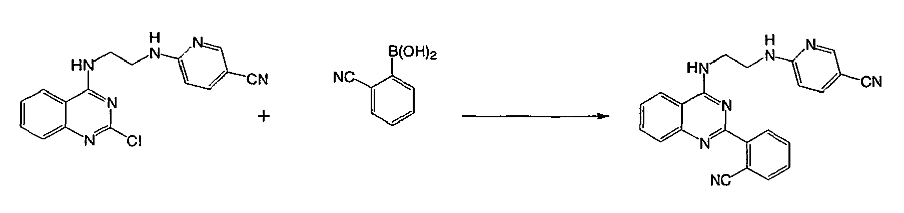

This compound was prepared in an analogous manner to Example 8 using 2-cyano-phenylboronic acid. Purification was achieved purified by reverse-phase HPLC

¹H NMR (500 MHz, DMSO(d₆)): δ9.7 (m, 1H), 8.38 (d, 2H), 8.00 (m, 2H), 7.82 (m, 1H), 7.78 (d, 2H), 7.72 (t, 1H), 6.80 (s, 1H), 6.70 (d, 1H), 4.04 (m, 2H), 3.65 (m, 2H). FIA-MS: (ES+) m/e= 392.3 (M+H).

Example 12 **6-{2-[2-(3-Cyano-phenyl)-quinazolin-4-ylamino]-ethylamino}-nicotinonitrile** (compound I-29)

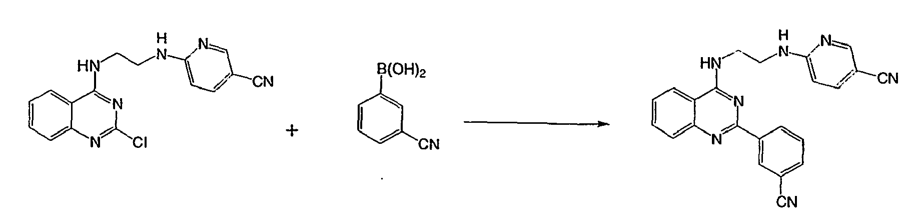

This compound was prepared in an analogous manner to Example 8 using 3-cyano-phenylboronic acid. Purification was achieved purified by reverse-phase HPLC

¹H NMR (500 MHz, DMSO(d₆)): δ10.02 (m, 1H), 8.65 (s, 1H), 8.50 (d, 1H), 8.42 (m, 2H), 8.19 (d, 1H), 8.05 (t, 1H), 7.95 (d, 1H), 7.80 (m, 2H), 7.77 (t, 1H), 7.50 (br.s, 1H), 6.40 (br.s, 1H), 4.05 (m, 2H), 3.75 (m, 2H). FIA-MS: (ES+) m/e= 392.3 (M+H), (ES-) m/e= 504.1 (M + TFA).

Example 13 **6-{2-[2-(4-Cyano-phenyl)-quinazolin-4-ylamino]-ethylamino}-nicotinonitrile** (compound I-19)

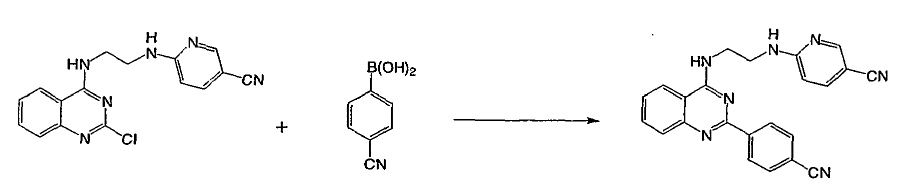

This compound was prepared in an analogous manner to Example 8 using 4-cyano-phenylboronic acid. Purification was achieved purified by reverse-phase HPLC

¹H NMR (500 MHz, DMSO(d₆)): δ9.7 (m, 1H), 8.38 (m, 4H), 8.05 (d, 2H), 7.95 (t, 1H), 7.87 (d, 1H), 7.80 (m, 1H), 7.70 (m, 1H), 7.50 (m, 1H), 6.40 (m, 1H), 4.00 (m, 2H), 3.75 (m, 2H). FIA-MS: (ES+) m/e= 392.2 (M+H), (ES-) m/e= 504.1 (M + TFA).

Example 14 **6-{2-[2-(2-Methoxy-phenyl)-quinazolin-4-ylamino]-ethylamino}-nicotinonitrile** (Compound I-188)

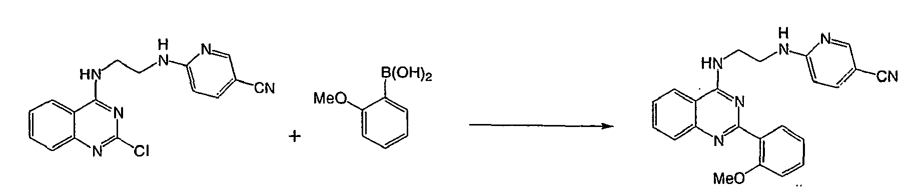

This compound was prepared in an analogous manner to Example 8 using 2-methoxy-phenylboronic acid. Purification was achieved purified by reverse-phase HPLC
¹H NMR (500 MHz, DMSO(d₆)): δ 13.80 (s, 1H), 10.30 (t, 1H), 8.48 (d, 1H), 8.32 (s, 1H), 8.06 (t, 1H), 7.93 (d, 1H), 7.76 (m, 2H), 7.68 (m, 2H), 7.50 (d, 1H), 7.29 (d, 1H), 7.10 (t, 1H), 6.40 (br.s, 1H), 4.05 (m, 2H), 4.00 (s, 3H), 3.75 (m, 2H). FIA-MS: (ES+) m/e= 397.3 (M+H), (ES-) m/e= 395.2 (M-H).

Example 15 **6-{2-[2-(2-Hydroxy-phenyl)-quinazolin-4-ylamino]-ethylamino}-nicotinonitrile** (Compound I-189)

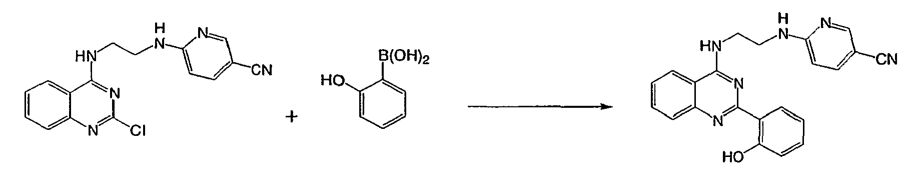

This compound was prepared in an analogous manner to Example 8 using 2-hydroxy-phenylboronic acid. Purification was achieved purified by reverse-phase HPLC
¹H NMR (500 MHz, DMSO(d₆)): δ14.80 (s, 1H), 8.85 (t, 1H), 8.48 (s, 1H), 8.35 (d, 1H), 8.27 (d, 1H), 7.85 (m, 2H), 7.80 (d, 1H), 7.67 (m, 1H), 7.60 (t, 1H), 7.36 (t, 1H), 6.92 (m, 2H), 6.58 (m, 1H), 3.90 (m, 2H), 3.73 (m, 2H). FIA-MS: (ES+) m/e= 383.2 (M+H), (ES-) m/e= 381.2 (M-H).

Example 16 **6-{2-[2-(2-Fluoro-phenyl)-quinazolin-4-ylamino]-ethylamino}-nicotinonitrile** (Compound I-190)

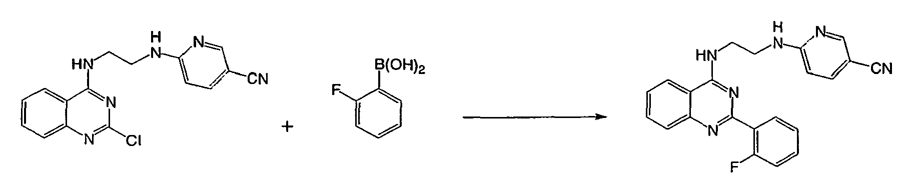

This compound was prepared in an analogous manner to Example 8 using 2-fluoro-phenylboronic acid. Purification was achieved purified by reverse-phase HPL

¹H NMR (500 MHz, DMSO(d₆)): δ10.40 (s, 1H, 8.47 (d, 1H), 8.33 (s, 1H), 8.10 (t, 1H), 7.94 (d, 1H), 7.80 (m, 4H), 7.50 (m, 2H), 7.42 (t, 1H), 6.50 (m, 1H), 4.00 (m, 2H), 3.73 (m, 2H). FIA-MS: (ES+) m/e= 385.2 (M+H), (ES-) m/e= 383.2 (M-H).

Example 17 **6-{2-[2-(2,4-Difluoro-phenyl)-quinazolin-4-ylamimo]-ethylamino}-nicotinonitrile** (Compound I-191)

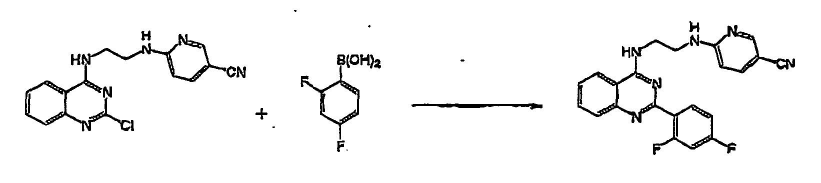

This compound was prepared in an analogous manner to Example 8 using 2,4-difluoro-phenylboronic acid. Purification was achieved purified by reverse-phase HPLC

¹H NMR (500 MHz, DMSO(d₆)): δ10.30 (s, 1H), 8.43 (d, 1H), 8.30 (s, 1H), 8.10 (t, 1H), 7.90 (m, 2H), 7.75 (m, 2H), 7.55 (td, 1H), 7.48 (m, 1H), 7.33 (td, 1H), 6.45 (m, 1H), 4.00 (m, 2H), 3.71 (m, 2H). FIA-MS: (ES+) m/e= 403.2 (M+H), (ES-) m/e= 515.1 (M+TFA).

Examples 19 **6-[2-(2-Thiophen-3-yl-quinazolin-4-ylamino)-ethylamino]-nicotinonitrile** (compound I-32)

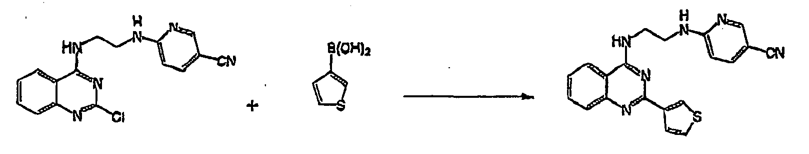

¹H NMR (500 MHz, DMSO(d₆)): δ10.17 (s, 1H), 8.73 (s, 1H), 8.40 (m, 2H), 8.05 (t, 1H), 7.93 (d, 1H), 7.85 (m, 2H), 7.80 (d, 1H), 7.70 (t, 1H), 7.58 (br.s, 1H), 6.50 (br.s, 1H), 4.07 (m, 2H), 3.75 (m, 2H). FIA-MS: (ES+) m/e= 373.2 (M+H), (ES-) m/e= 485.1 (M+TFA).

Example 20 **6-{2-[2-(2,3-Dichloro-phenyl)-quinazolin-4-ylamino]-ethylamino}-nicotinonitrile** (Compound I-193)

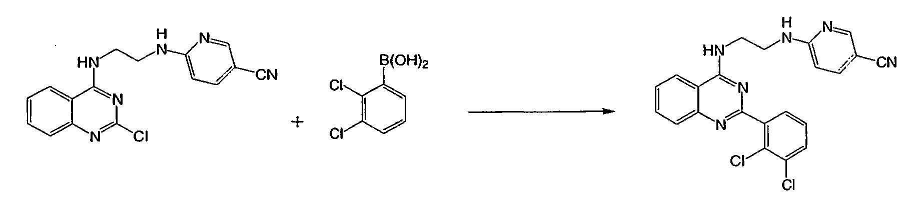

This compound was prepared in an analogous manner to Example 8 using 2,3-dichloro-phenylboronic acid. Purification was achieved purified by reverse-phase HPLC.

¹H NMR (500 MHz, DMSO(d₆)): δ10.37 (s, 1H), 8.48 (s, 1H), 8.30 (d, 1H), 8.08 (t, 1H), 7.97 (d, 1H), 7.80 (m, 2H), 7.78 (m, 1H), 7.70 (d, 1H), 7.58 (t, 1H), 7.50 (dd, 1H), 6.43 (br.s, 1H), 3.97 (m, 2H), 3.68 (m, 2H). FIA-MS: (ES+) m/e= 435.0 (M+H), (ES-) m/e= 547.0 (M+TFA).

Example 21 **6-{2-[2-(2,5-Dichloro-phenyl)-quinazolin-4-ylamino]-ethylamino}-nicotinonitrile** (Compound I-194)

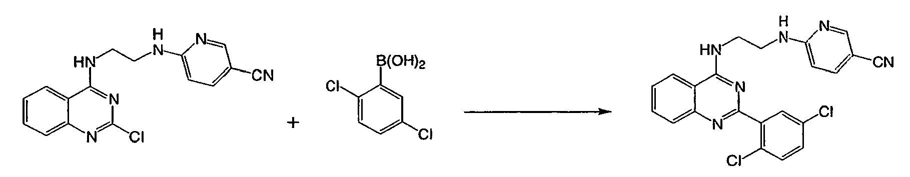

This compound was prepared in an analogous manner to Example 8 using 2,5-dichloro-phenylboronic acid. Purification was achieved purified by reverse-phase HPLC.

¹H NMR (500 MHz, DMSO(d₆)): δ10.35 (s, 1H), 8.52 (d, 1H), 8.30 (d, 1H), 8.10 (t, 1H), 7.97 (d, 1H), 7.82 (m, 3H), 7.78 (m, 1H), 7.70 (s, 1H), 7.48 (d, 1H), 6.43 (br.s, 1H), 3.92 (m, 2H), 3.60 (m, 2H). FIA-MS: (ES+) m/e= 435.0 (M+H), (ES-) m/e= 547.0 (M+TFA).

Example 22 **6-{2-[2-(4-Chloro-biphenyl-1-yl)-quinazolin4-ylamino]-ethylamino}-nicotinonitrile** (Compound I-195)

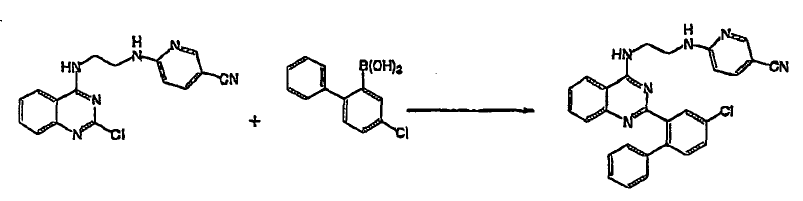

This compound was prepared in an analogous manner to Example 8 using 4-chlorobiphenylboronic acid. Purification was achieved purified by reverse-phase HPL

¹H NMR (500 MHz, DMSO(d₆)): δ10.20 (s, 1H), 8.39 (d, 1H), 8.30 (s, 1H). 8.05 (t, 1H), 7.85 (d, 1H), 7.78 (m, 3H), 7.70 (t, 1H), 7.65 (d, 1H), 7.55 (dd, 1H), 7.30 (m, 5H). 6.45 (d, 1H), 3.45 (m, 2H), 3.33 (m, 2H). FIA-MS: (ES+) m/e= 443.2 (M+H), (ES-) m/e= 441.2 (M-H), 477.2 (M+TFA).

Example 24 **6-{2-[5,6-Dimethyl-2-(2-trifluoromethyl-phenyl)-pyrimidin-4-ylamino]-ethylamino}-nicotinonitrile** (Compound I-197)

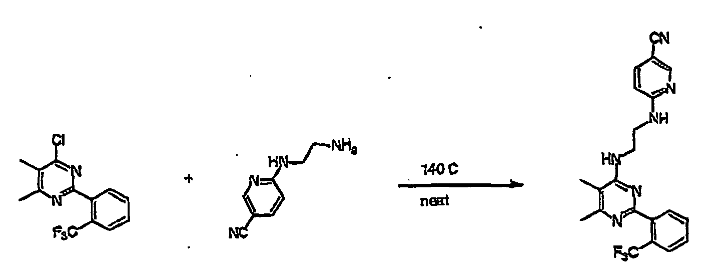

This compound was prepared in an analogous manner as described in Example 1 using 4-chloro-5,6-dimethyl-2-(2-trifluoromethyl-phenyl)-pyrimidine. Purification was achieved by silica gel chromatography (1:1 ethyl acetate/ hexane, 2:1 ethyl acetate/hexane) to yield the title compound (43 mg, 60%).

¹H NMR (500 MHz, d₆-DMSO, ppm) δ2.25s, 3H), 2.30 (s, 3H), 3.45-3.59 (m, 4H), 6.50 (br s, 1H), 6.94 (br s, 1H), 7.52-7.60 (m, 1H), 7.60-7.66 (m, 2H), 7.69-7.75 (m, 2H), 7.78-7.84 (m, 1H), 8.36 (s, 1H); LC-MS (ES+) m/e= 413.12 (M+H); Rₜ=1.90 min.

Example 25 **6-{2-[2-(2,4-Dichloro-phenyl)-5,6-dimethyl-pyrimidin-4-ylamino]-ethylamino}-nicotinonitrile** (Compound I-198)

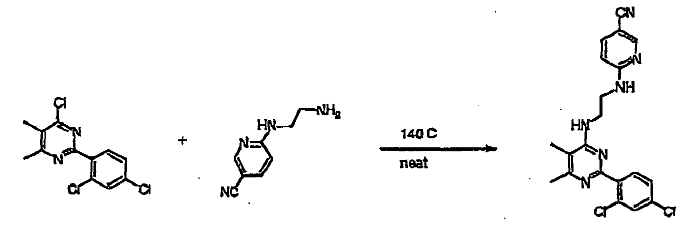

This compound was prepared in an analogous manner as described in Example 1 using 4-chloro-2-(2-4-dichloro-phenyl)-5,6-dimethyl-pyrimidine. Purification was achieved by silica gel chromatography (1:1 ethyl acetate/ hexane. 2:1 ethyl acetate/hexane) to yield the title compound (31 mg, 43%).

¹H NMR (500 MHz, d₆-DMSO, ppm) δ 2.02 (s, 3H), 2.30 (s, 3H), 3.48-3.56 (m, 4H), 6.50 (br s, 1H). 7.00 (br s, 1H), 7.47 (d, 1H), 7.55-7.60 (m, 1H), 7.61-7.69 (m, 2H), 7.70-7.75 (m, 1H), 8.35 (s, 1H); LC-MS (ES+) m/e= 413.00 (M+H); Rₜ=1.95 min.

Example 27 **6-{3-[2-(2,4-Dichloro-phenyl)-quinazolin-4-ylamino]-propylamino}-nicotinonitrile** (Compound I-97)

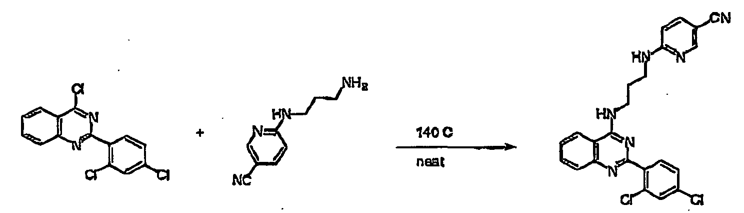

This compound was prepared in an analogous manner as described in Example 1 using 4-chloro-2-(2,4-dichloro-phenyl)-quinazoline and 6-(3-amino-propylamino)-nicotinonitrile, (which was prepared in a similar fashion to 6-(2-amino-ethylamino)-nicotinonitrile). Purification was achieved by silica gel chromatography (1:1 ethyl acetate/ hexane, 2:1 ethyl acetate/hexane) to yield the title compound (50 mg, 57%).

¹H NMR (500 MHz, d₆-DMSO, ppm) δ 1.91-2.0 (m, 2H), 3.37-3.45 (m, 2H), 3.60-3.70 (m, 2H), 6.47 (d, 1H), 7.50-7.54 (m, 1H), 7.54-7.60 (m, 1H), 7.60-7.65 (m, 2H), 7.68-7.70 (m, 1H), 7.72-7.85 (m, 3H), 8.28 (d, 1H), 8.35 (s, 1H), 8.42-8.52 (m, 1H); LC-MS (ES+) m/e= 448.94 (M+H); Rₜ= 2.31 min.

Example 28 **6-{2-[2-(2-Chloro-phenyl)-5,6-dimethyl-primidin-4-ylamino]-ethylamino}-nicotinonitrile** (Compound I-143)

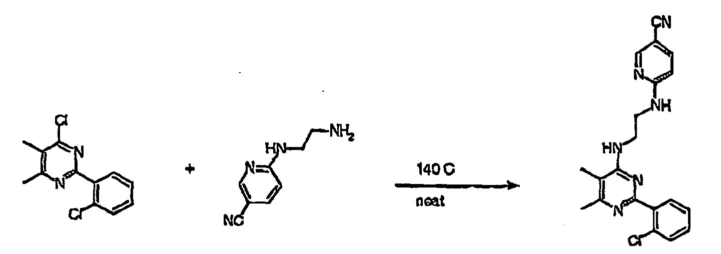

This compound was prepared in an analogous manner as described in Example 1 using 4-chloro-2-(2-chloro-phenyl)-5,6-dimethyl-pyrimidine. Purification was achieved by silica gel chromatography (2:1 ethyl acetate/ hexane, 4:1 ethyl acetate/hexane) to yield the title compound (46 mg, 61%).

¹H NMR (500 MHz, d₆-DMSO, ppm) δ2.02 (s, 3H), 2.31 (s, 3H), 3.46-3.65 (m, 4H), 6.47-6.60 (m, 1H), 6.92-7.04 (m, 1H), 7.36-7.45 (m, 2H), 7.48-7.52 (m, 1H), 7.54-7.68 (m, 2H), 7.70-7.80 (m, 1H), 8.34-8.42 (m, 1H); LC-MS (ES+) m/e= 379.02 (M+H); Rₜ= 1.72 min.

Example 29 **6-{3-[2-(2,4-Dichloro-phenyl)-5,6-dimethyl-pyrimidin-4-ylamino]-propylamino}-nicotinonitrile** (Compound I-142)

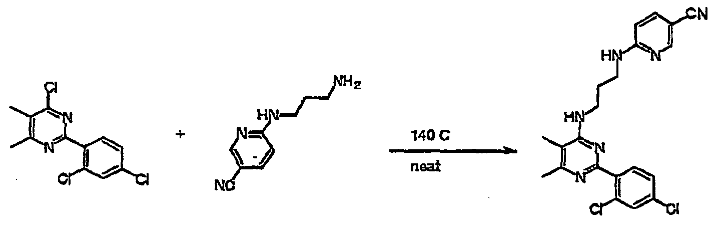

This compound was prepared in an analogous manner as described in Example 1 using 4-chloro-2-(2,4-dichloro-phenyl)-5,6-dimethyl-pyrimidine and 6-(3-amino-propylamino)-nicotinonitrile, (which was prepared in a similar fashion to 6-(2-aminoethylamino)-nicotinomtrile). Purification was achieved by silica gel chromatography (1:1 ethyl acetate/ hexane, 2:1 ethyl acetate/hexane) to yield the title compound (32 mg, 43%).

¹H NMR (500 MHz, d₆-DMSO, ppm) δ1.79-1.86 (m, 2H), 2.01 (s, 3H), 2.30 (s, 3H), 3.30-3.40 (m, 2H), 3.41-3.50 (m, 2H), 6.46 (d, 1H), 6.83-6.92 (m, 1H), 7.42-7.50 (m, 1H), 7.56-7.70 (m, 4H), 8.31 (s, 1H); LC-MS (ES+) m/e=426.98 (M+H); Rₜ= 2.07 min.

Example 33 **6-{2-[2-(2,4-Difluoro-phenyl)-quinazolin-4-ylamino]-ethylamino}-nicotinonitrile** (Compound I-2)

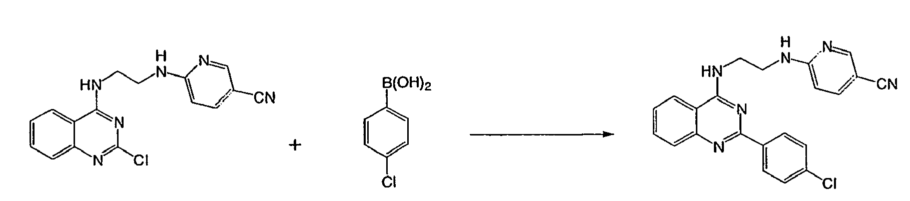

This compound was prepared in an analogous manner to Example 8 using 4-chloro-phenylboronic acid. Purification was achieved purified by reverse-phase HPL

¹H NMR (500 MHz, DMSO(d₆)): δ8.52-8.37 (m, 2H), 8.32-8.20 (m, 2H), 8.30 (s, 1H),8.11-7.39 (m, 8H), 6.53-6.34 (m, 1H), 4.09-3.95 (m, 2H), 3.80-3.65 (m, 2H). LC-MS (ES+) m/e= 401.10 (M+H); Rₜ= 2.20 min

Example 34 **2-{2-[2-(2,4-Dichloro-phenyl)-quinazolin-4-ylamino]-ethylamino}-nicotinonitrile** (Compound I-65)

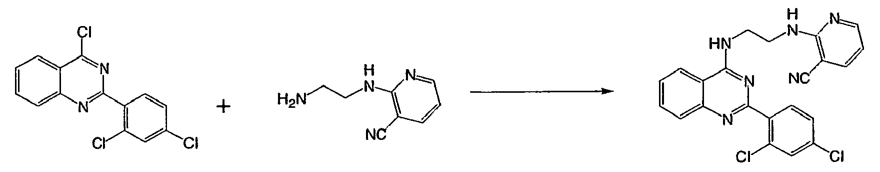

This compound was prepared in an analogous manner to Example 1 using 2-(2-amino-ethylamino)nicotinonitrile (prepared from 6-chloro pyridine-2-carbonitrile according to the method described by Nuss et al, WO 99/65997). Isolated as a white solid (3.0mg, 2%).

¹H NMR (500 MHz, DMSO(d₆)): δ10.30-10.10 (1H, bs), 8.45 (1H, m), 8.17 (1H, m), 8.10-8.05 (1H, m), 7.90-7.70 (6H, m), 7.35 (1H, m), 6.55 (1H, m), 4.0-7.90 (2H, m), 3.80-3.70 (2H, m). HPLC Rt= 3.10 min,; FIA (M+H) 434.9, (M-H) 432.6,;

Example 35 **4-{2-[2-(2,4-Dichloro-phenyl)-quinazolin-4-ylamino]-ethylamino}-benzonitrile** (Compound I-62)

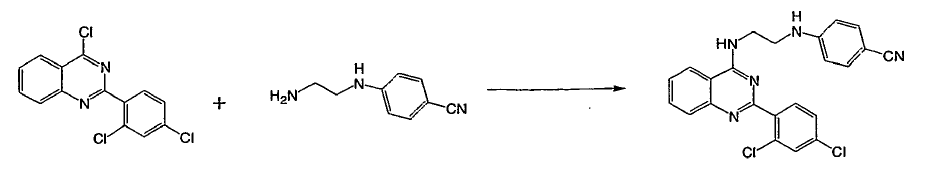

This compound was prepared in an analogous manner to Example 1 using 4-(2-amino-ethylamino)-benzonitrile (prepared from 4-fluorobenzonitrile according to the method described by Nuss et al, WO 99/65997). Isolated as a white solid (23.6mg, 28%.

¹H NMR (500 MHz, DMSO-d₆: δ10.20-10.00 (1H, bs), 8.40 (1H, m), 8.00 (1H, m), 8.0-7.60 (6H, m), 7.20 (2H, m), 6.60 (2H, m), 3.80-3.70 (2H, m), 3.45-3.35 (2H, m. HPLC Rt = 3.23 min,; FIA (M+H) m/e= 434.0, (M-H) m/e= 431.7

Example 36 ***N*-[2-(2,4-Dichloro-phenyl)-quinazolin-4-yl]-*N*'-phenyl-ethane-1,2-diamine** (Compound I-64)

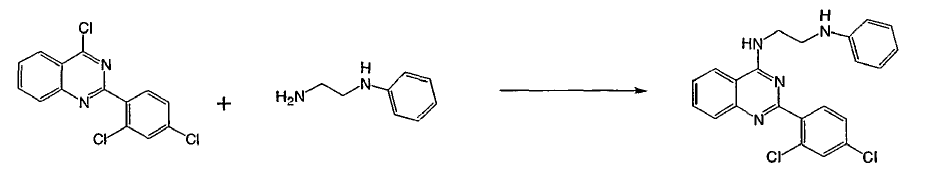

This compound was prepared in an analogous manner to Example 1 using N-phenylethylenediamine. Isolated as a white solid (49.9mg, 28%)

¹H NMR (500 MHz, DMSO-d₆: δ10.40-10.25 (1H, bs), 8.45 (1H, m), 8.05 (1H, m), 7.92 (1H, s), 8.85-7.60 (4H, m), 6.90 (2H, m), 6.60 (2H, m), 6.45 (1H, m), 3.85-3.75 (2H, m), 3.40-3.30 (2H, m). HPLC Rt = 3.22 min; FIA (M+H) m/e= 409.0, (M-H) m/e= 407.0

Example 37 **N-(1H-Indazol-3-yl)-N'-[2-(2-trifluoromethyl-phenyl)-quinazolin-4-yl]-ethane-1,2-diamine,** trifluoro-acetic acid salt (Compound I-10)

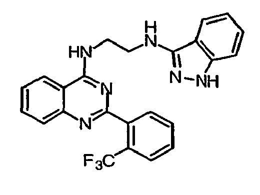

**N1-(1H-Indazol-3-yl)-ethane-1,2-diamine**

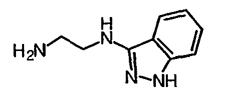

A mixture of 3-aminoindazole (0.40 g, 3 mmol) and 2-bromoethylamine hydrobromide (0.62 g, 3 mmol) in ethanol (15 mL) was sealed in a pressure tube and heated at 160-170°C for 2 days. The crude mixture was partitioned between aqueous sodium bicarbonate and 1:9 methanol:ethyl acetate. The organic layer was dried over sodium sulfate and the solvent was then evaporated. Purification by flash chromatography (SiO₂) eluted with 0.2:2:8 ammonium hydroxide:methanol:dichloromethane provided N1-(1H-indazol-3-yl)-ethane-1,2-diamine and 1-(2-aminoethyl)-1H-indazol-3-ylamine, both containing impurities.

N1-(1H-Indazol-3-yl)-ethane-1,2-diamine was isolated (52.5 mg, 10% yield, 74.7% purity by HPLC) as a colorless film. R_{f}=0.05 (SiO₂, 1:9 methanol: dichloromethane; ¹H-NMR (500 MHz, CD₃OD, ppm) δ 7.65 (d, J=7.3 Hz, 1H), 7.27 (m, 2H), 6.96 (dt, J=1.0, 7.2 Hz, 1H), 3.48 (t, J=6.5 Hz, 2H), 3.01 (t, J=6.0 Hz, 2H) (+ impurity peaks); MS (LC/MS) 177.01 (M+H); HPLC R₁=1.100 min.

1-(2-Aminoethyl)-1H-indazol-3-ylamine was isolated (57.1 mg, 11% yield) as a colorless film. R_{f}=0.16 (SiO₂, 1:9 methanol:dichloromethane); ¹H-NMR (500 MHz, CD₃OD, ppm) δ 7.56 (d, J=7.4 Hz, 1H), 7.23 (d, J=8.8 Hz, 1H), 7.14 (t, J=7.6 Hz, 1H), 6.75 (t, J=7.5 Hz, 1H), 4.19 (t, J=6.3 Hz, 2H), 3.08 (t, J=6.3 Hz, 2H) (+ impurity peaks); MS (LC/MS) 177.01 (M+H); HPLC Rₜ= 0.500 min.

A mixture of 4-chloro-2-(2-trifluoromethyl-phenyl)-quinazoline (0.05 g, 0.16 mmol) and N1-(1H-indazol-3-yl)-ethane-1,2-diamine (0.03 g, 0.16 mmol) in dimethylformamide (2 mL) was heated at 120-125°C for 2 h. After cooling, the reaction mixture was poured into water (30 mL) and was treated with saturated bicarbonate (10 mL). The resulting precipitate was filtered off and was then washed with water. Purification by preparative HPLC (Gilson) provided the title compound (3% yield) as a yellow film.

¹H-NMR (500 MHz, d₆-DMSO, ppm) δ 11.6 (s, 1H), 8.48 (d, J=7.8 Hz, 1H), 7.97 (m, 2H), 7.81 (m, 6H), 7.63 (d, J=8.0 Hz, 1H), 7.25 (d, J=2.5 Hz, 1H), 6.89 (m, 1H), 3.93 (t, J=5.8 Hz, 2H), 3.61 (t, J=6.1 Hz, 2H); MS (LC/MS) 449.14 (M+H); HPLC Rₜ= 2.677 min.

EXAMPLE 12 - BIOLOGICAL TESTING

The activity of the compounds as protein kinase inhibitors is assayed *in vitro, in vivo* or in a cell line. *In vitro* assays include assays that determine inhibition of either the phosphorylation activity or ATPase activity of the activated protein kinase. Alternate *in vitro* assays quantitate the ability of the inhibitor to bind to the protein kinase. Inhibitor binding may be measured by radiolabelling the inhibitor prior to binding, isolating the inhibitor/protein kinase complex and determining the amount of radiolabel bound. Alternatively, inhibitor binding may be determined by running a competition experiment where new inhibitors are incubated with the protein kinase bound to known radioligands.

Kᵢ DETERMINATION FOR THE INHIBITION OF GSK-3

Compounds were screened for their ability to inhibit GSK-3β (AA 1-420) activity using a standard coupled enzyme system [Fox et al. Protein Sci. 7, 2249 (1998)]. Reactions were carried out in a solution containing 100 mM HEPES (pH 7.5), 10 mM MgCl₂, 25 mM NaCl, 300 µM NADH, 1 mM DTT and 1.5% DMSO. Final substrata concentration in the assay were 20 µM ATP (Sigma Chemicals, St Louis, MO) and 300 µM peptide (American Peptide, Sunnyvale, CA). Reactions were carried out at 30 °C and 20 nM GSK-3β. Final concentrations of the components of the coupled enzyme system were 2.5 mM phosphoenolpyruvate, 300 µM NADH, 30 µg/ml pyruvate kinase and 10 µg/ml lactate dehydrogenase.

An assay stock buffer solution was prepared containing all of the reagents listed above with the exception of ATP and the test compound of interest. The assay stock buffer solution (175 µl) was incubated in a 96 well plate with 5 µl of the test compound of interest at final concentrations spanning 0.002 µM to 30 µM at 30 °C for 10 min. Typically, a 12 point titration was conducted by preparing serial dilutions (from 10 mM compound stocks) with DMSO of the test compounds in daughter plates. The reaction was inflated by the addition of 20 µl of ATP (final concentration 20 µM). Rates of redaction were obtained using a Molecular Devices Spectramax plate reader (Sunnyvale, CA) over 10 min at 30 °C. The Kᵢ values were determined from the rate data as a function of inhibitor concentration.

The following compounds were shown to have Kᵢ values less than 0.1 µM for GSK-3: compounds **I-1, I-2, I-8, I-9, I-11, I-19, I-62, I-183, I-188, I-189, I-190, I-191, I-195, I-197, I-198,** and **I-200.**

The following compounds were shown to have Kᵢ values between 0.1 and 1.0 µM for GSK-3: compounds **I-3, I-10, I-29, I-105, I-187, I-193,** and **I-194.**

While we have hereinbefore presented a number of embodiments of this invention, it is apparent that our basic construction can be altered to provide other embodiments that utilize the compounds and methods of this invention. Therefore, it will be appreciated that the scope of this invention is to be defined by the appended claims rather than by the specific embodiments that have been represented by way of example.

## Claims

1. A compound of formula **I**: 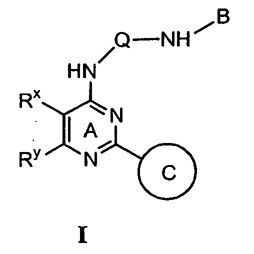 or a pharmaceutically acceptable salt thereof, wherein:
Ring A, taken together with R^{x} and R^{y}, is selected from 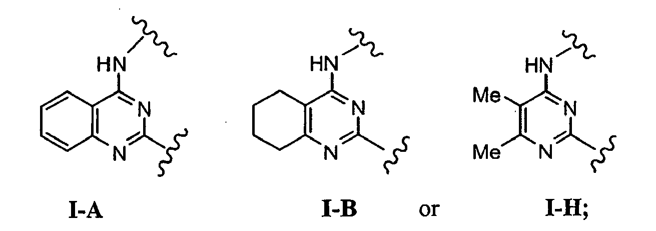
B is an optionally substituted pyridyl, pyrimidinyl, thiazolyl, indolyl, imidazolyl, oxadiazolyl, tetrazolyl, pyrazinyl, triazolyl, thienyl, furanyl, quinolinyl, purinyl, naphthyl, benzothiazolyl, benzopyridyl, benzimidazolyl, indazolyl, isothiazolyl, pyrazolyl, pyridazinyl, isoxazolyl, phenyl, benzothiophenyl, or pyridothiophenyl; wherein B is optionally substituted with nitro, amino, cyano, halo, thioamido, amidino, oxamidino, alkoxyamidino, imidino, guanidino, sulfonamido, carboxyl, formyl, alkyl, haloalkyl, alkoxy, haloalkoxy, alkoxyalkyl, alkylaminoalkoxy, alkylcarbonyl, aralkylcarbonyl, heteroaralkylcarbonyl, alkylthio, aminoalkyl, cyanoalkyl, hydroxy, alkoxycarbonyl, aryl, aralkyl, heteroaryl, or heteroaralkyl;
Q is a C₁₋₄alkylidene chain wherein each methylene unit of said Q is substituted by R² and R^{2'};
each R² and R^{2'} is H;
Ring C is selected from an optionally substituted phenyl or pyridinyl, wherein said Ring C optionally has one or two ortho substituents independently selected from -R¹, or any substitutable carbon position on Ring C is optionally independently substituted by -R⁵, or two adjacent substituents on Ring C are optionally taken together with their intervening atoms to form a naphthyl, quinolinyl, isoquinolinyl or benzodioxolyl ring, said ring being optionally substituted by halo, oxo, or -R⁸;
R¹ is selected from -halo, -CN, -NO₂, T-V-R⁶, phenyl, 5-6 membered heteroaryl ring, 5-6 membered heterocyclyl ring, or C₁₋₆ aliphatic group, said phenyl, heteroaryl, and heterocyclyl rings each optionally substituted by up to three groups independently selected from halo, oxo, or -R⁸, said C₁₋₆ aliphatic group optionally substituted with halo, cyano, nitro, or oxygen, or R¹ and an adjacent substituent taken together with their intervening atoms form said ring fused to Ring C;
R is selected from an optionally substituted group selected from C₁₋₁₀ aliphatic, aryl, aralkyl, heteroaryl, or heteroaralkyl; T is a valence bond or a C₁₋₄ alkylidene chain
each R⁴ is independently selected from -R⁷, -COR⁷, -CO₂(C₁₋₆ aliphatic), -CON(R⁷)₂, or -SO₂R⁷, or two R⁴ on the same nitrogen are taken together to form a 5-8 membered heterocyclyl or heteroaryl ring;
each R⁵ is independently selected from -R', halo, -OR', -C(=O)R', -CO₂R', -COCOR', -NO₂, -CN, -S(O)R', -SO₂R', -SR', -N(R⁴)₂, -CON(R⁴)₂, -SO₂N(R⁴)₂, -OC(=O)R', -N(R⁴)COR', -N(R⁴)CO₂(optionally substituted C₁₋₆ aliphatic), -N(R⁴)N(R⁴)₂, -C=NN(R⁴)₂, -C=N-OR', -N(R⁴)CON(R⁴)₂, -N(R⁴)SO₂N(R⁴)₂, -N(R⁴)SO₂R', or -OC(=O)N(R⁴)₂, or R⁵ and an adjacent substituent taken together with their intervening atoms form said ring fused to Ring C;
each R' is independently selected from hydrogen or an optionally substituted group selected from C₁₋₆ aliphatic, C₆₋₁₀ aryl, a heteroaryl ring having 5-10 ring atoms, or a heterocyclyl ring having 5-10 ring atoms;
V is -O-, -S-, -SO-, -SO₂-, -N(R⁶)SO₂-, -SO₂N(R⁶)-, -N(R⁶)-, -CO-, -CO₂-, -N(R⁶)CO-, -N(R⁶)C(O)O-, -N(R⁶)CON(R⁶)-, -N(R⁶)SO₂N(R⁶)-, -N(R⁶)N(R⁶)-, -C(O)N(R⁶)-, -OC(O)N(R⁶)-, -C(R⁶)₂O-, -C(R⁶)₂S-, -C(R⁶)₂SO-, -C(R⁶)₂SO₂-, -C(R⁶)₂SO₂N(R⁶)-, -C(R⁶)₂N(R⁶)-, -C(R⁶)₂N(R⁶)C(O)-, -C(R⁶)₂N(R⁶)C(O)O-, -C(R⁶)=NN(R⁶)-, -C(R⁶)=N-O-, -C(R⁶)₂N(R⁶)N(R⁶)-, -C(R⁶)₂N(R⁶)SO₂N(R⁶)-, or -C(R⁶)₂N(R⁶)CON(R⁶)-;
each R⁶ is independently selected from hydrogen or an optionally substituted C₁₋₄ aliphatic group, or two R⁶ groups on the same nitrogen atom are taken together with the nitrogen atom to form a 5-6 membered heterocyclyl or heteroaryl ring;
each R⁷ is independently selected from hydrogen or an optionally substituted C₁₋₆ aliphatic group, or two R⁷ on the same nitrogen are taken together with the nitrogen to form a 5-8 membered heterocyclyl or heteroaryl ring; and
each R⁸ is independently selected from an optionally substituted C₁₋₄ aliphatic group, -OR⁶, -SR⁶, -COR⁶, -SO₂R⁶, -N(R⁶)₂, -N(R⁶)N(R⁶)₂, -CN, -NO₂, -CON(R⁶)₂, or -CO₂R⁶.

2. The compound of claim 1, wherein B is an optionally substituted pyridyl, pyrimidinyl, thiazolyl, indolyl, imidazolyl, oxadiazolyl, tetrazolyl, pyrazinyl, triazolyl, thienyl, furanyl, quinolinyl, purinyl, naphthyl, benzothiazolyl, benzopyridyl, benzimidazolyl, indazolyl, isothiazolyl, pyrazolyl, pyridazinyl, isoxazolyl, benzothiophenyl, or pyridothiophenyl and Q is -CH₂CH₂-, -CH₂CH₂CH₂-, or -CH₂CH₂CH₂CH₂-.

3. The compound of claim 2, wherein B is optionally substituted 2-pyridyl and compounds have the general formula **II:** 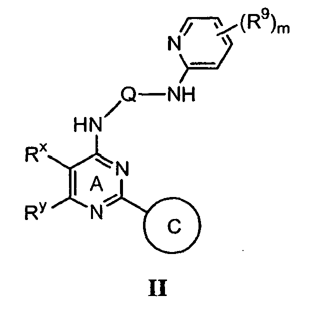 wherein m is 0-4, and each occurrence of R⁹ is independently selected from H, nitro, amino, cyano, halo, thioamido, amidino, oxamidino, alkoxyamidino, imidino, guanidino, sulfonamido, carboxyl, formyl, alkyl, haloalkyl, alkoxy, haloalkoxy, alkoxyalkyl, alkylaminoalkoxy, alkylcarbonyl, aralkylcarbonyl, heteroaralkylcarbonyl, alkylthio, aryl, aralkyl, heteroaryl, or heteroaralkyl.

4. The compound of claim 3 wherein R⁹ substituents are selected from hydrogen, C₁₋₄ alkyl, nitro, amino, cyano, cyanomethyl, trifluoromethyl, hydroxy, or methoxy.

5. The compound of claim 2, wherein B is indazolyl, pyrazolyl, or thiazolyl optionally substituted with one or more independent occurrences of R⁹, wherein R⁹ substituents are selected from hydrogen, C₁₋₄alkyl, nitro, amino, cyano, cyanomethyl, trifluoromethyl, hydroxy, or methoxy.

6. The compound of claim 2, wherein optional substituents on Ring C are selected from R¹ or R⁵, wherein each occurrence of R¹ is independently selected from -halo, an optionally substituted C₁₋₆ aliphatic group, phenyl, -COR⁶, -OR⁶, -CN, -SO₂R⁶, -SO₂NH₂, -N(R⁶)₂, -CO₂R⁶, -CONH₂, -NHCOR⁶, -OC(O)NH₂, or -NHSO₂R⁶; and each occurrence of R⁵ is selected from -halo, -CN, -NO₂, -N(R⁴)₂, optionally substituted C₁₋₆ aliphatic group, -OR', -C(O)R', -CO₂R', -CONH(R⁴), -N(R⁴)COR', -SO₂N(R⁴)₂, or -N(R⁴)SO₂R'.

7. The compound of claim 6, wherein R¹ groups are selected from -CF₃, -Cl, -F, -CN, -COCH₃, -OCH₃, -OH, -CH₂CH₃, -OCH₂CH₃, -CH₃, -CF₂CH₃, cyclohexyl, t-butyl, isopropyl, cyclopropyl, -C≡CH, -C≡C-CH₃, -SO₂CH₃, -SO₂NH₂, -N(CH₃)₂, -CO₂CH₃, -CONH₂, -NHCOCH₃, -OC(O)NH₂, -NHSO₂CH₃, or -OCF₃; and R⁵ groups are selected from -Cl, -F, -CN, -CF₃, -NH₂, -NH(C₁₋₄ aliphatic), -N(C₁₋₄ aliphatic)₂, -O(C₁₋₄ aliphatic), C₁₋₄ aliphatic, or -CO₂(C₁₋₄ aliphatic).

8. The compound of claim 2, having one of the formulas: 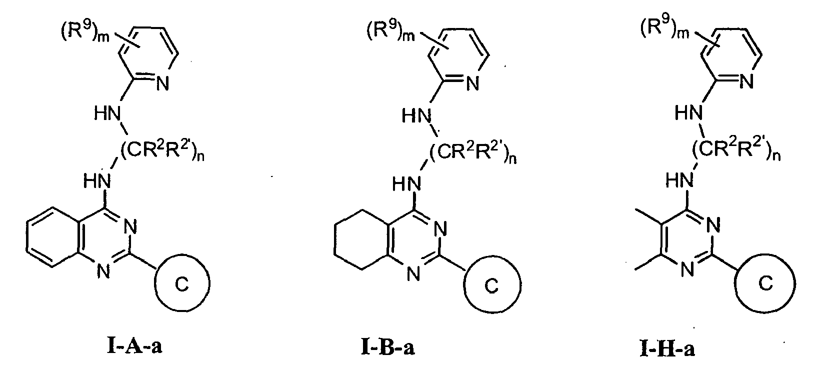 wherein m is 0-4, and each occurrence of R⁹ is independently selected from H, nitro, amino, cyano, halo, thioamido, amidino, oxamidino, alkoxyamidino, imidino, guanidino, sulfonamido, carboxyl, formyl, alkyl, haloalkyl, alkoxy, haloalkoxy, alkoxyalkyl, alkylaminoalkoxy, alkylcarbonyl, aralkylcarbonyl, heteroaralkylcarbonyl, alkylthio, aryl, aralkyl, heteroaryl, or heteroaralkyl.

9. The compound of claim 8, wherein ring C is a phenyl ring and R¹ is halo, methyl, cyano, OMe, OH, or trifluoromethyl.

10. The compound of claim 2; wherein Ring C is a phenyl or pyridinyl ring having one or two ortho substituents independently selected from -R¹, wherein Ring C is further substituted by -R⁵ and wherein when Ring C and two adjacent substituents thereon form a bicyclic ring system, the bicyclic ring system is selected from a naphthyl, quinolinyl or isoquinolinyl ring.

11. The compound of claim 2 or claim 10, wherein R¹ is -halo, an optionally substituted C₁₋₆ aliphatic group, phenyl, -COR⁶, -OR⁶, -CN, -SO₂R⁶, -SO₂NH₂, -N(R⁶)_{2,} -CO₂R⁶, -CONH₂, -NHCOR⁶, -OC(O)NH₂, or -NHSO₂R⁶.

12. The compound of claim 2, wherein Ring C is a phenyl or pyridinyl ring having one or two ortho substituents independently selected from -R¹, wherein Ring C is further substituted by -R⁵ and wherein when Ring C and two adjacent substituents thereon form a bicyclic ring system, the bicyclic ring system is a naphthyl ring.

13. The compound of claim 2 or claim 12, wherein R¹ is -halo, a C₁₋₆haloaliphatic group, a C₁₋₆ aliphatic group, phenyl, OMe, OH, or -CN.

14. The compound of any one of claims 2, 12, or 13, wherein each R⁵ is independently selected from -halo, -CN, -NO₂, -N(R⁴)₂, optionally substituted C₁₋₆ aliphatic group, -OR', -C(O)R', -CO₂R', -CONH(R⁴), -N(R⁴)COR', -SO₂N(R⁴)₂, or -N(R⁴)SO₂R'.

15. The compound of claim 2, wherein B is an optionally substituted pyridyl group optionally substituted by 0, 1, or 2 occurrences of R⁹, wherein each occurrence of R⁹ is independently hydrogen, C₁₋₄alkyl, nitro, amino, cyano, cyanomethyl, trifluoromethyl, hydroxy, or methoxy.

16. The compound of claim 15, wherein Ring C is a phenyl or pyridinyl ring having one or two ortho substituents independently selected from -R¹, wherein Ring C is further substituted by -R⁵ and wherein when Ring C and two adjacent substituents thereon form a bicyclic ring system, the bicyclic ring system is a naphthyl ring.

17. The compound of any one of claims 2, 15, or 16, wherein R¹ is -halo, a C₁₋₆ haloaliphatic group, a C₁₋₆ aliphatic group, phenyl, OMe, OH, or -CN.

18. The compound of any one of claims 2, 15, 16, or 17, wherein each R⁵ is independently selected from -halo, -CN, -NO₂, -N(R⁴)₂, optionally substituted C₁₋₆ aliphatic group, -OR', -C(O)R', -CO₂R', -CONH(R⁴), -N(R⁴)COR', -SO₂N(R⁴)₂, or -N(R⁴)SO₂R'.

19. The compound of claim 2, wherein B is an optionally substituted pyridyl group optionally substituted with 2 independent occurrences of R⁹, wherein each occurrence of R⁹ is independently hydrogen, C₁₋₄alkyl, nitro, amino, cyano, cyanomethyl, trifluoromethyl, hydroxy, or methoxy.

20. The compound of claim 2 or claim 19, wherein Ring C is a phenyl ring having one or two ortho substituents independently selected from -R¹, wherein Ring C is further substituted by -R⁵.

21. The compound of any one of claims 2, 19, or 20, wherein R¹ is -halo, a C₁₋₄ aliphatic group optionally substituted with halogen, OMe, OH, or -CN.

22. The compound of any one of claims 2, 19, 20, or 21, wherein each R⁵ is independently selected from -Cl, -F, -CN, -CF₃, -NH₂, -NH(C₁₋₄ aliphatic), -N(C₁₋₄ aliphatic)₂, -O(C₁₋₄ aliphatic), optionally substituted C₁₋₄ aliphatic, and -CO₂(C₁₋₄ aliphatic).

23. The compound of claim 1, selected from one of the compounds:

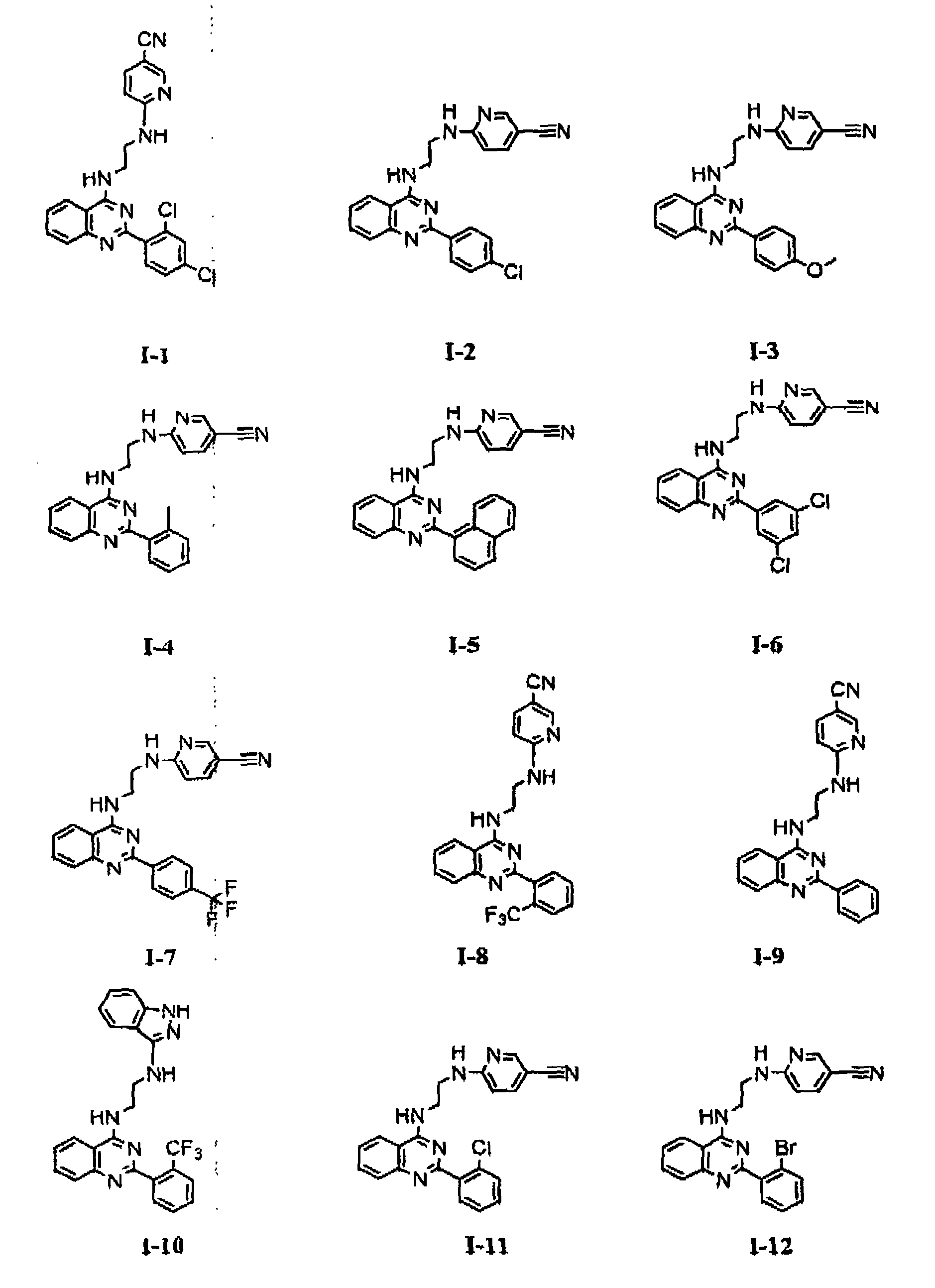

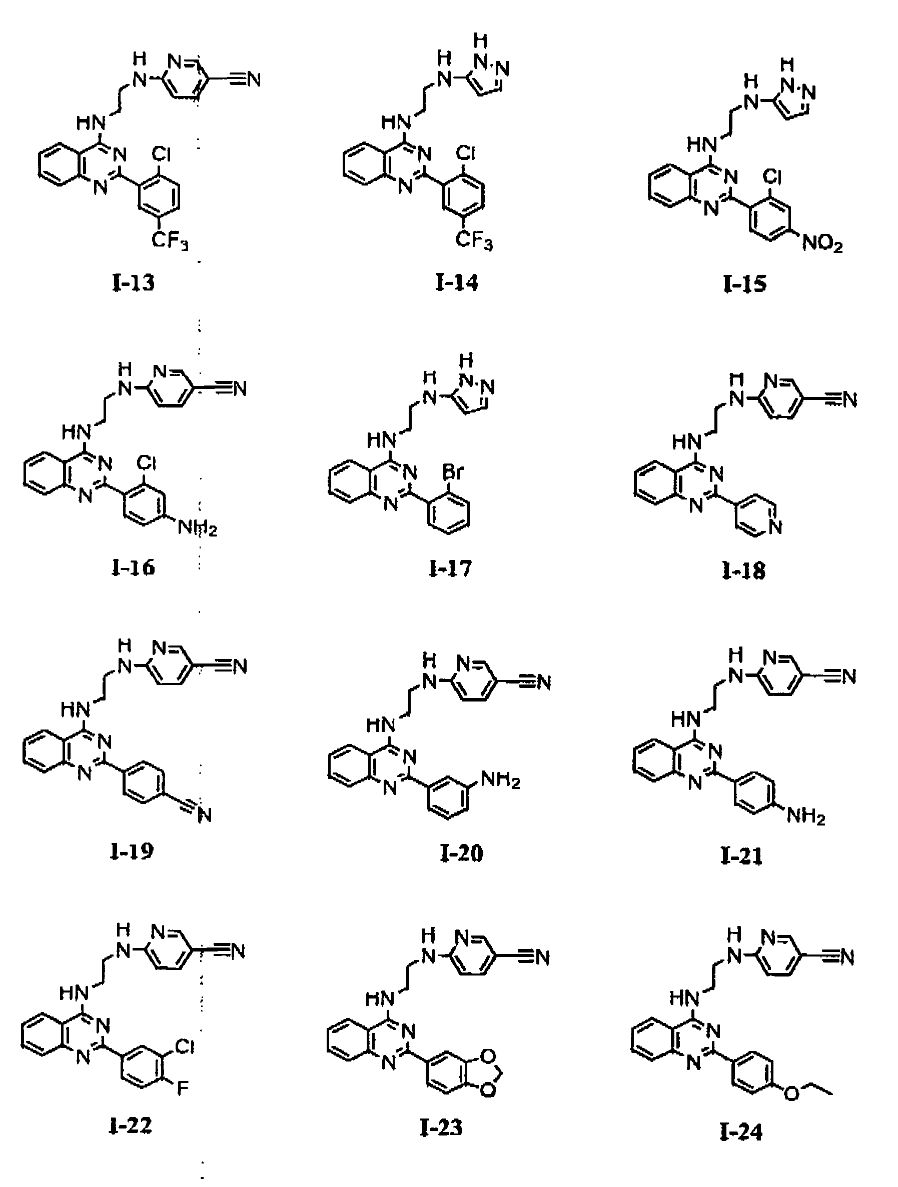

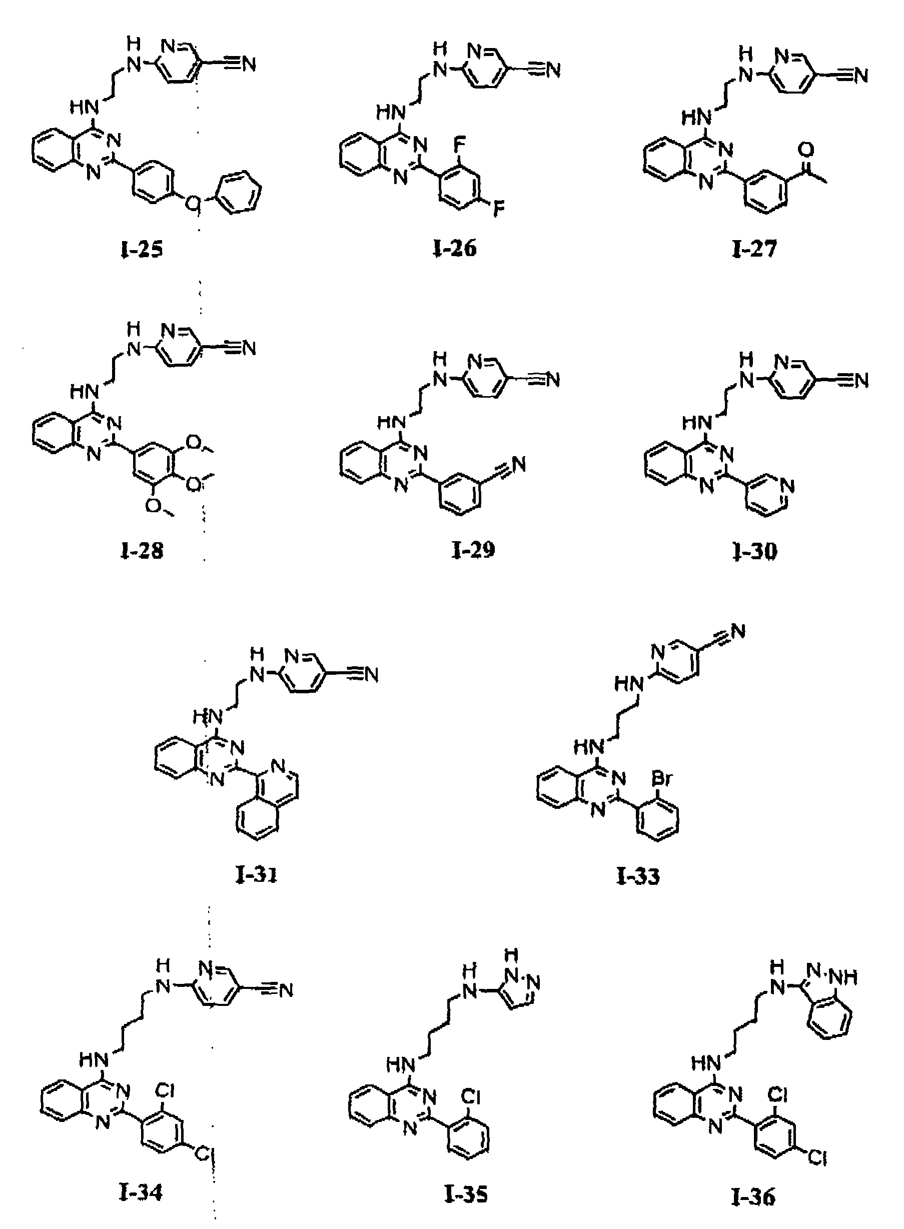

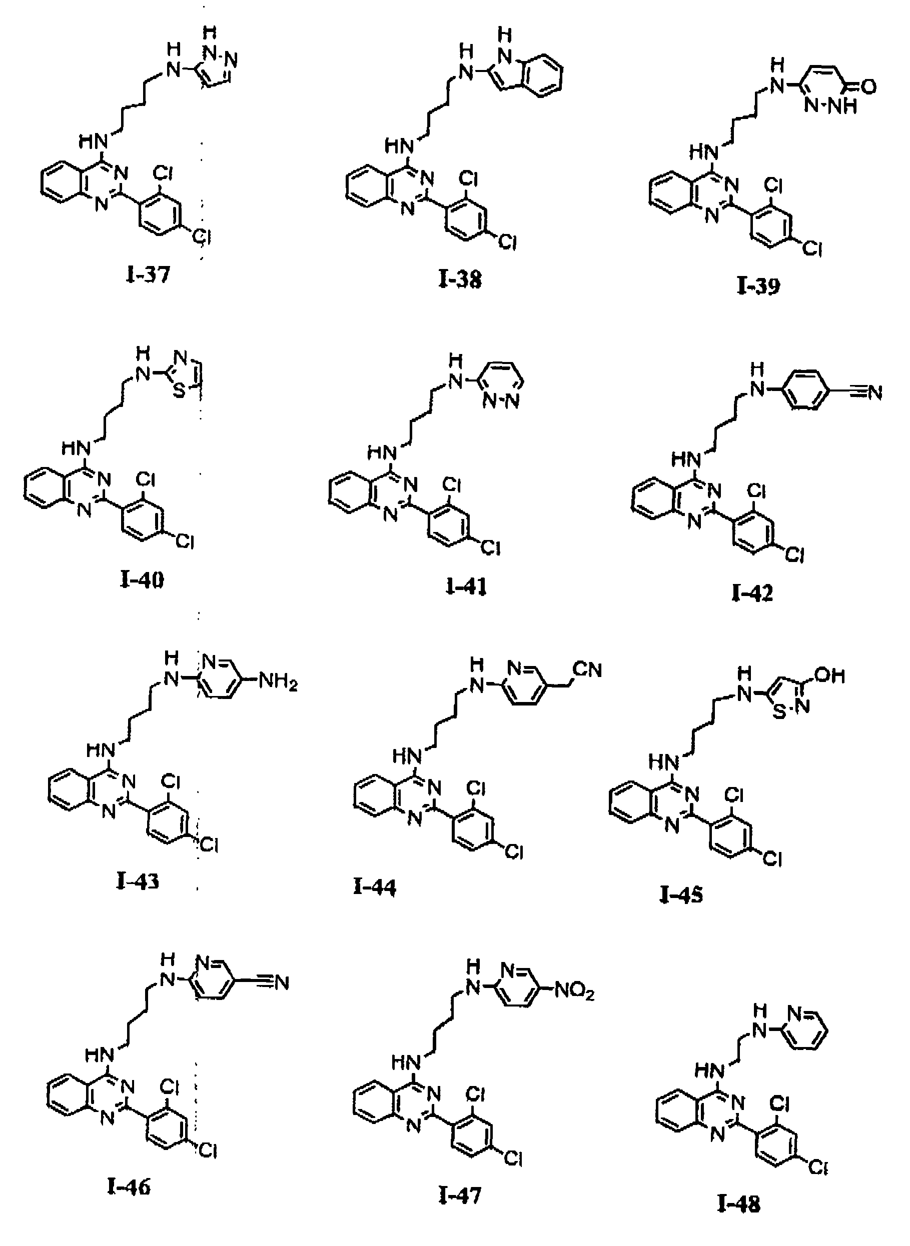

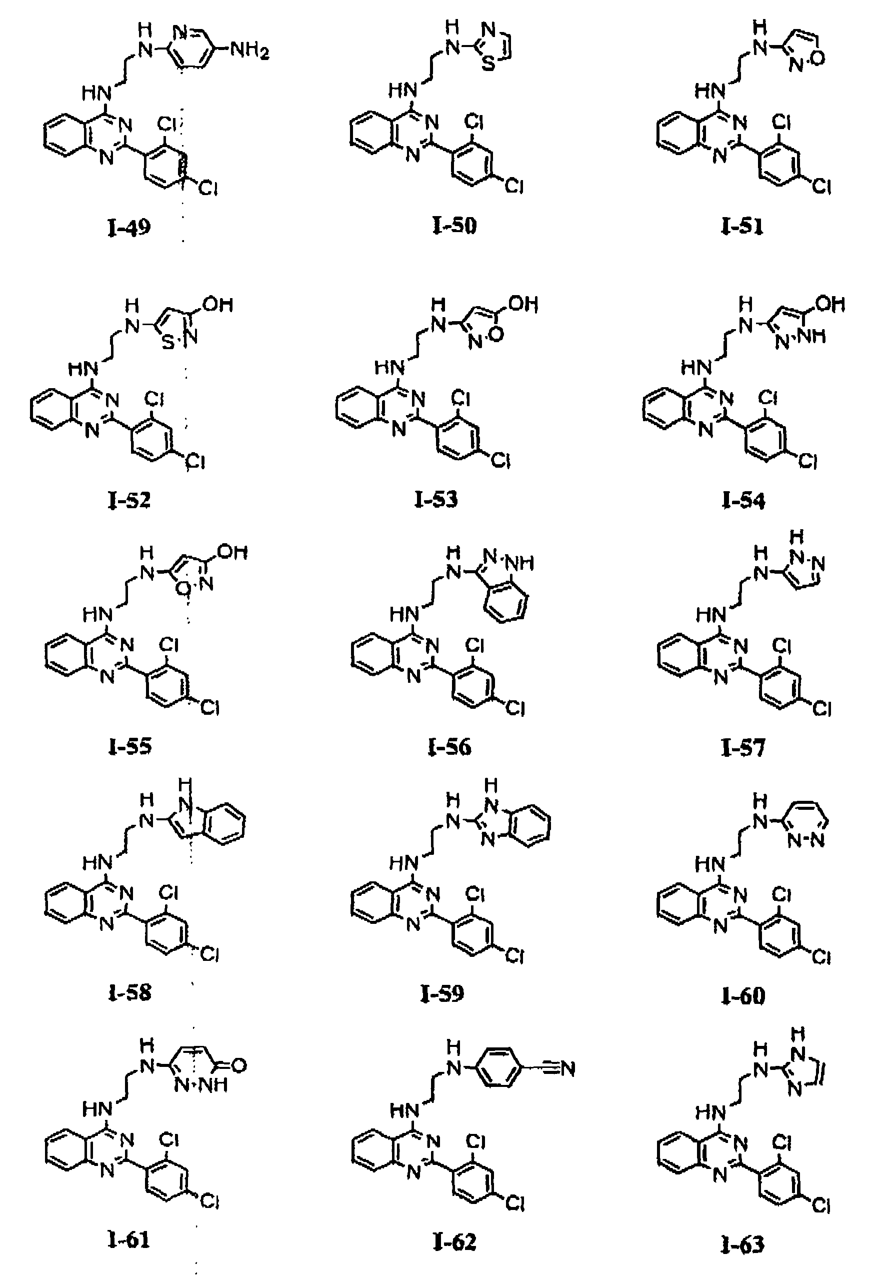

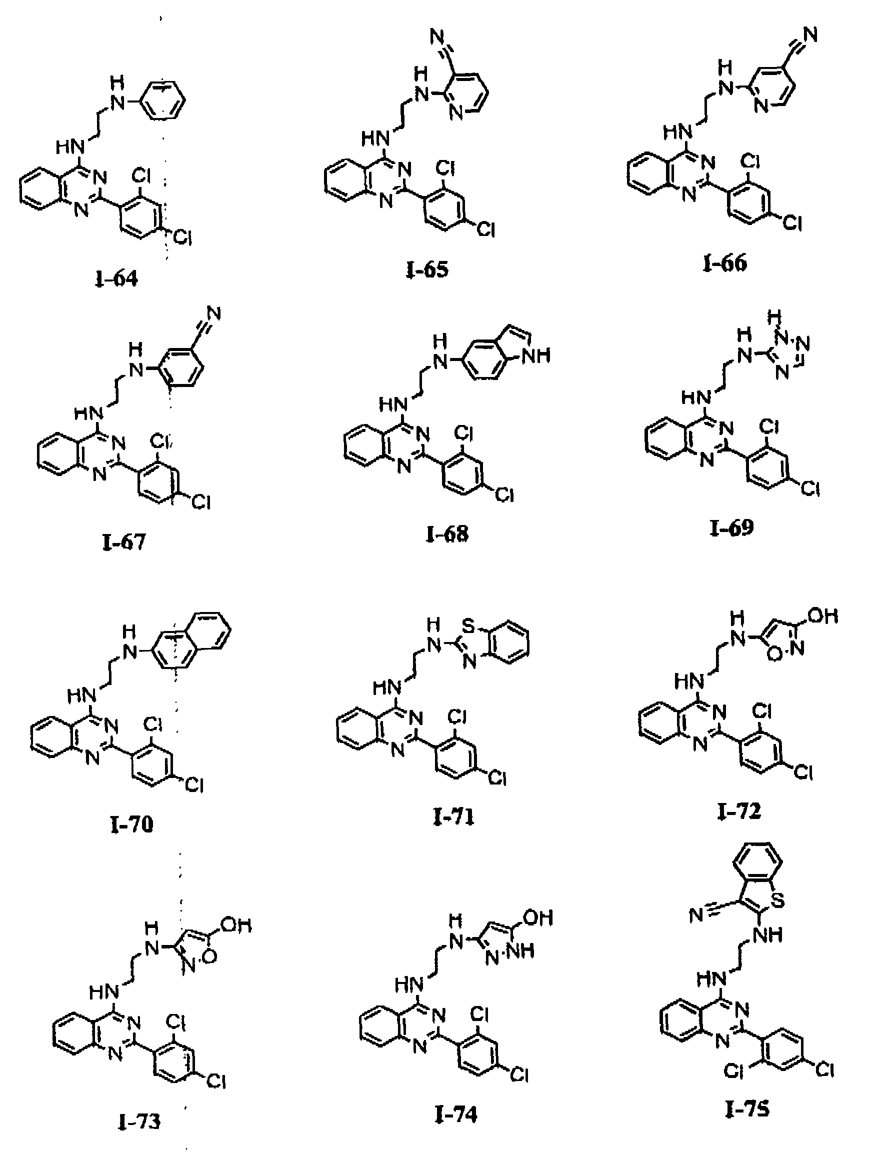

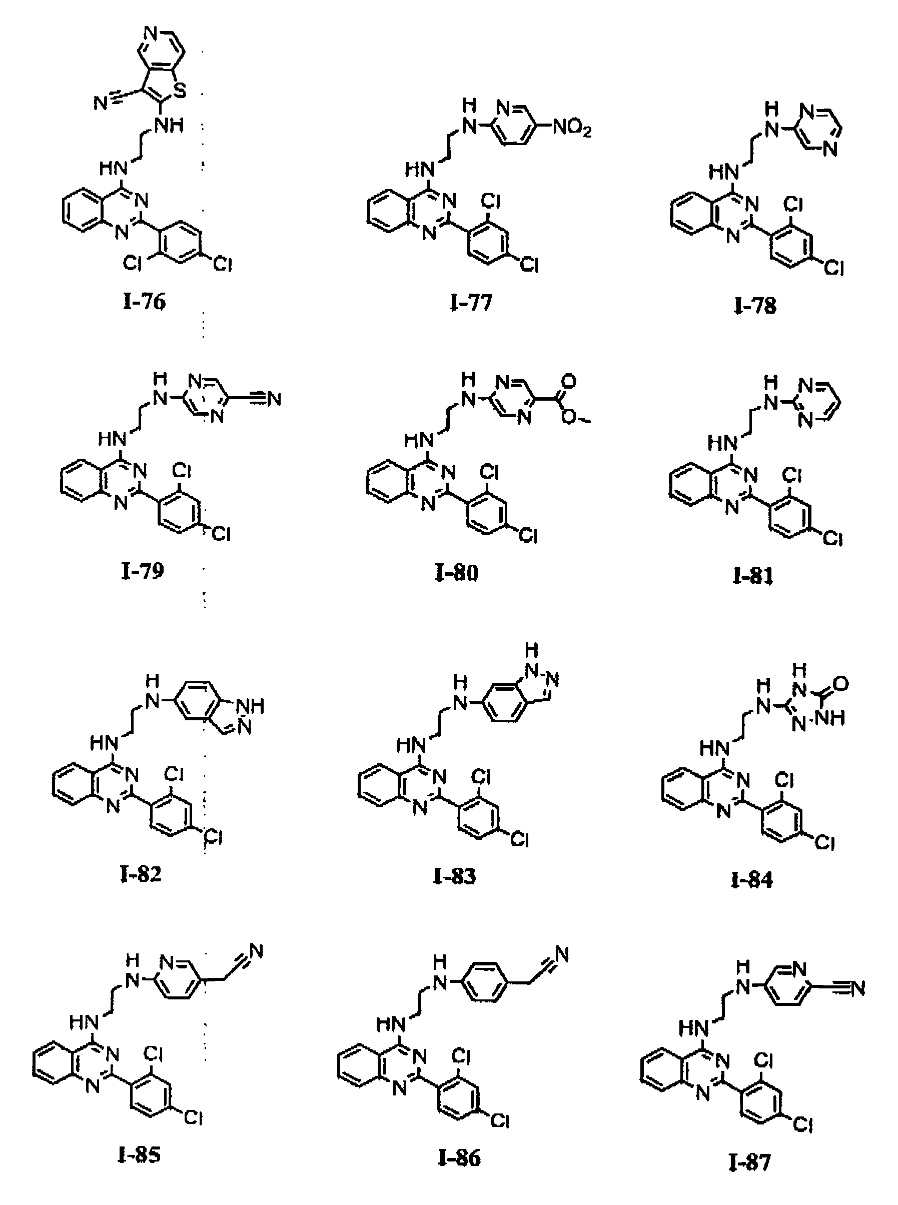

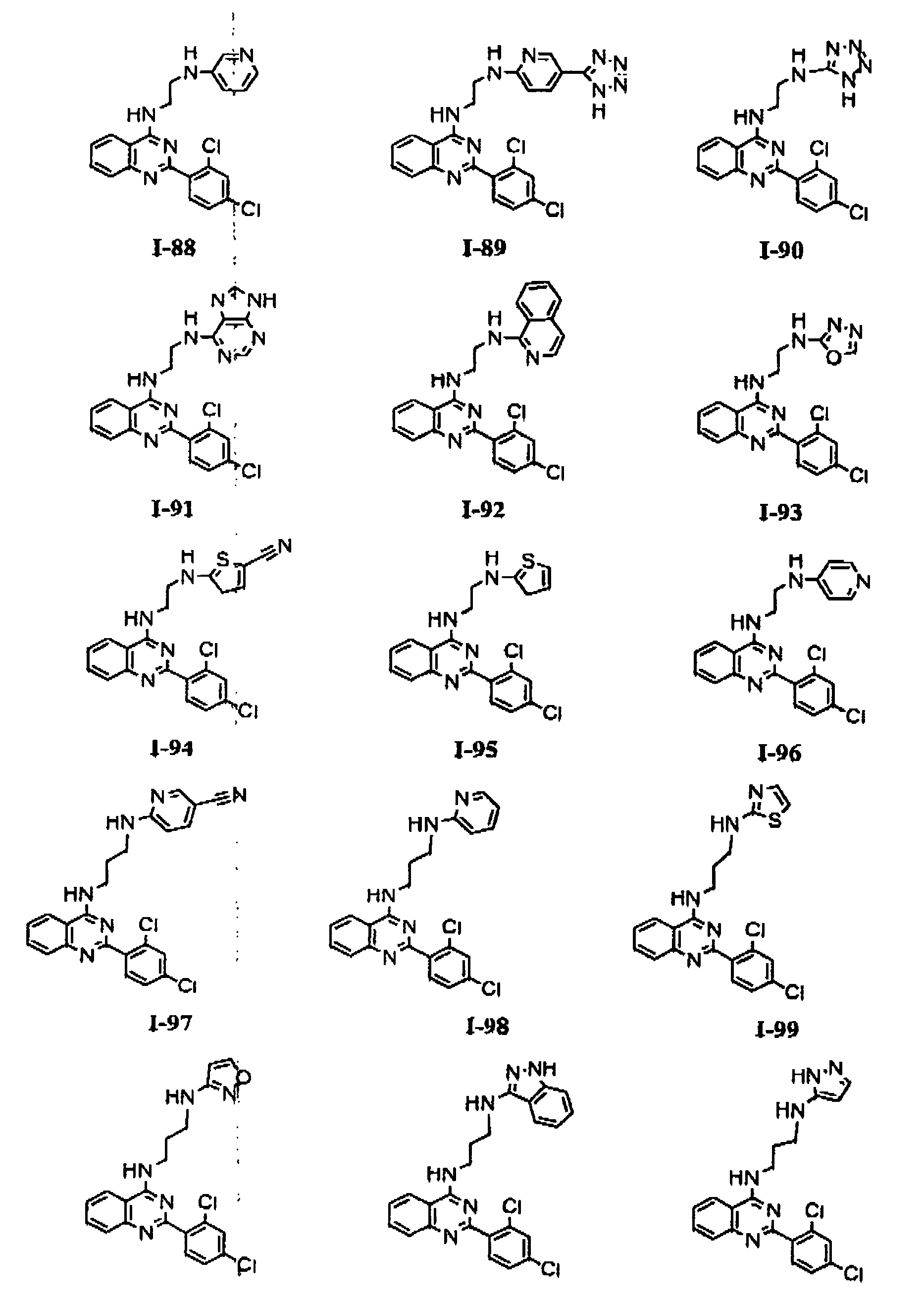

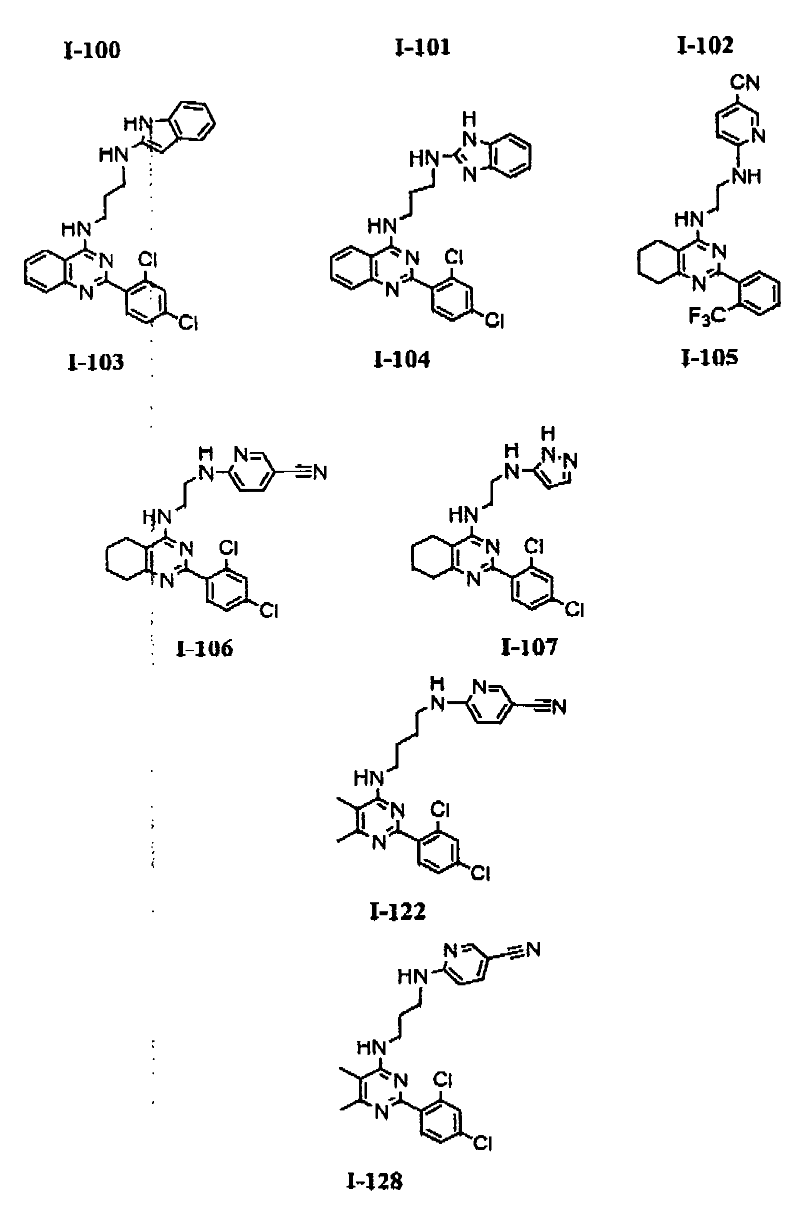

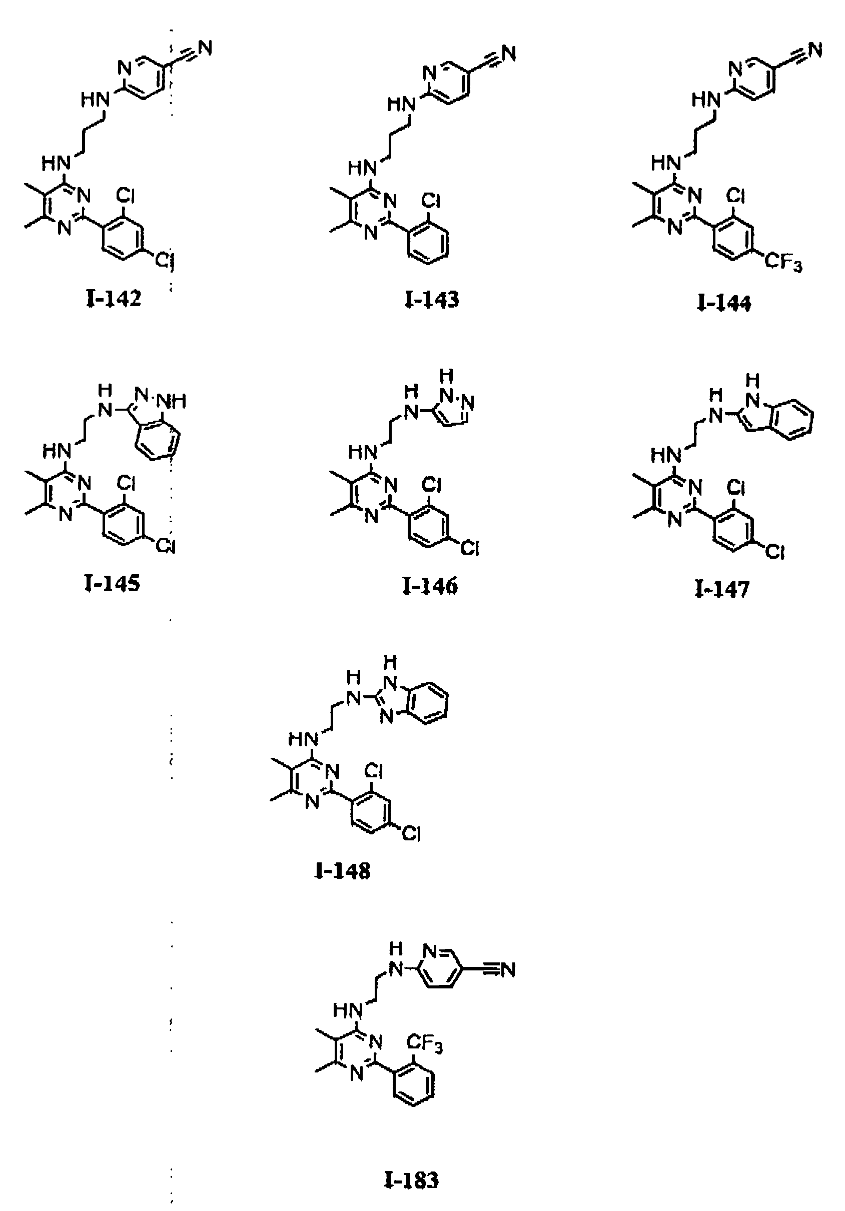

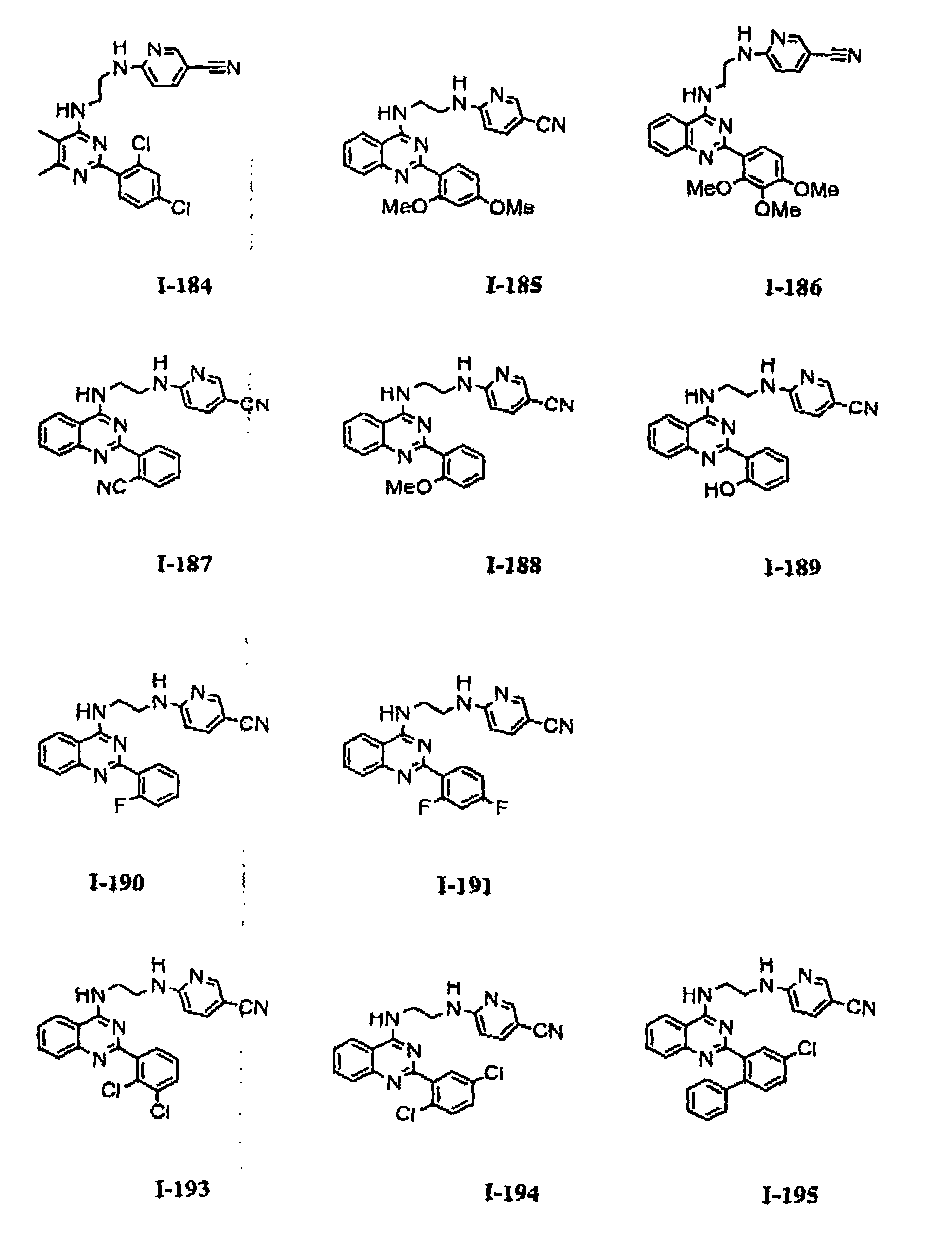

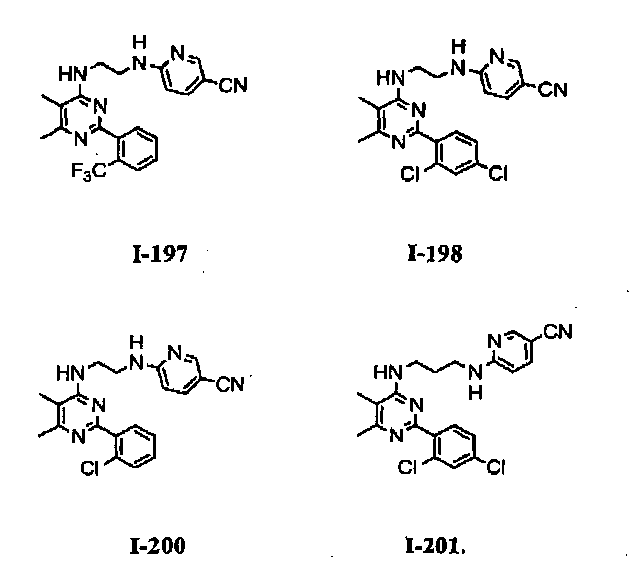

24. A composition comprising a compound of claim 2, and a pharmaceutically acceptable carrier, adjuvant, or vehicle.

25. A composition comprising a therapeutically effective amount of a compound of claim 2, and a pharmaceutically acceptable carrier, adjuvant, or vehicle.

26. The composition of claim 25, wherein the therapeutically effective amount is an amount capable of inhibiting GSK-3 activity.

27. The composition of claim 24, further comprising one of more additional therapeutic agents.

28. A method of inhibiting GSK-3 kinases activity in a biological sample, comprising the step of contacting said biological sample with:
a) a composition according to claim 24; or
b) a compound according to claim 2.

29. The use of: a) a composition according to claim 24; or b) a compound according to claim 2, for the manufacture of a medicament for treating or lessening the severity of an autoimmune disease, an inflammatory disease, a metabolic disorder, a neurological or neurodegenerative disorder, or a cardiovascular disease in a patient.

30. The use of claim 29, wherein the disease is selected from allergy, asthma, diabetes, Alzheimer's disease, Huntington's disease, Parkinson's disease, AIDS-associated dementia, amyotrophic lateral sclerosis (AML, Lou Gehrig's disease), multiple sclerosis (MS), schizophrenia, cardiomyocyte hypertrophy, reperfusion/ischemia, stroke, or baldness.

31. The use of claim 30, wherein the disease is stroke.

32. The use of claim 29, wherein the disease is a neurological or neurodegenerative disorder.

33. The use of claim 29, wherein said medicament is combined with an additional therapeutic agent selected from a treatment for Alzheimer's Disease, a treatment for Parkinson's Disease, an agent for treating Multiple Sclerosis (MS), a treatment for asthma, an anti-inflammatory agent, an immunomodulatory or immunosuppressive agent, a neurotrophic factor, an agent for treating stroke, an agent for treating cardiovascular disease, or an agent for treating diabetes, wherein said additional therapeutic agent is appropriate for the disease being treated.

## Patentansprüche

1. Eine Verbindung der Formel **I:** 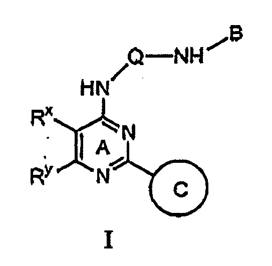 oder ein pharmazeutisch verträgliches Salz davon, wobei
Ring A, zusammengenommen mit R^{x} und R^{y}, ausgewählt ist aus 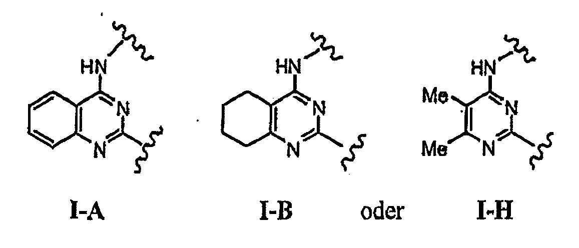 B ein gegebenenfalls substituiertes Pyridyl, Pyrimidinyl, Thiazolyl, Indolyl, Imidazolyl, Oxadiazolyl, Tetrazolyl, Pyrazinyl, Triazolyl, Thienyl, Furanyl, Chinolinyl, Purinyl, Naphthyl, Benzothiazolyl, Benzopyridyl, Benzimidazolyl, Indazolyl, Isothiazolyl, Pyrazolyl, Pyridazinyl, Isoxazolyl, Phenyl, Benzothiophenyl oder Pyridothiophenyl ist;
wobei B gegebenenfalls mit Nitro, Amino, Cyano, Halogen, Thioamido, Amidino, Oxamidino, Alkoxyamidino, Imidino, Guanidino, Sulfonamido, Carboxyl, Formyl, Alkyl, Halogenalkyl, Alkoxy, Halogenalkoxy, Alkoxyalkyl, Alkylaminoalkoxy, Alkylcarbonyl, Aralkylcarbonyl, Heteroaralkylcarbonyl, Alkylthio, Aminoalkyl, Cyanalkyl, Hydroxy, Alkoxycarbonyl, Aryl, Aralkyl, Heteroaryl oder Heteroaralkyl substituiert ist;
Q eine C₁₋₄-Alkylidenkette ist, wobei jede Methyleneinheit von besagtem Q mit R² und R² substituiert ist;
jeder R² und R^{2'} H ist;
Ring C aus einem gegebenenfalls substituierten Phenyl oder Pyridinyl ausgewählt ist, wobei besagter Ring C gegebenenfalls einen oder zwei ortho-Substituenten aufweist, die unabhängig voneinander aus -R¹ ausgewählt sind, oder eine beliebige substituierbare Kohlenstoffposition in Ring C gegebenenfalls unabhängig mit -R⁵ substituiert ist oder zwei benachbarte Substituenten an Ring C gegebenenfalls zusammengenommen mit ihren dazwischenliegenden Atomen einen Naphthyl-, Chinolinyl-, Isochinolinyl- oder Benzodioxolylring bilden, wobei besagter Ring gegebenenfalls mit Halogen, Oxo oder -R⁸ substituiert ist;
R¹ aus -Halogen, -CN, NO₂, T-V-R⁶, Phenyl, einem 5-6-gliedrigen Heteroarylring, einem 5-6-gliedrigen Heterocyclylring oder einer C₁₋₆ aliphatischen Gruppe ausgewählt ist, wobei besagte Phenyl-, Heteroaryl- und Heterocyclylringe jeweils gegebenenfalls mit bis zu drei Gruppen substituiert sind, die unabhängig voneinander aus Halogen, Oxo oder -R⁸ ausgewählt sind, besagte C₁₋₆ aliphatische Gruppe gegebenenfalls mit Halogen, Cyano, Nitro oder Sauerstoff substituiert ist oder R¹ und ein benachbarter Substituent zusammengenommen mit ihren dazwischenliegenden Atomen besagten Ring bilden, der an Ring C fusioniert ist;
R aus einer gegebenenfalls substituierten Gruppe ausgewählt ist, die aus C₁₋₁₀ aliphatischer Gruppe, Aryl, Aralkyl, Heteroaryl oder Heteroaralkyl ausgewählt ist;
T eine Valenzbindung oder eine C₁₋₄-Alkylidenkette ist;
jeder R⁴ unabhängig aus -R⁷, -COR⁷, -CO₂(C₁₋₆ aliphatisch), -CON(R⁷)₂ oder -SO₂R⁷ ausgewählt ist oder zwei R⁴ am selben Stickstoff zusammengenommen einen 5-8-gliedrigen Heterocyclyl- oder Heteroarylring bilden;
jeder R⁵ unabhängig aus -R', Halogen, -OR' -C(=O)R', -CO₂R', COCOR', -NO₂, -CN, -S(O)R', -SO₂R', -SR', -N(R⁴)₂, -CON(R⁴)₂, -SO₂N(R⁴)₂, -OC(=O)R', -N(R⁴)COR', -N(R⁴)CO₂(gegebenenfalls substituierte C₁₋₆ aliphatische Gruppe), -N(R⁴)N(R⁴)₂, -C=NN(R⁴)₂, -C=N-OR', -N(R⁴)CON(R⁴)₂, -N(R⁴)SO₂N(R⁴)₂, -N(R⁴)SO₂R' oder -OC(=O)N(R⁴)₂ ausgewählt ist oder R⁵ und ein benachbarter Substituent zusammengenommen mit ihren dazwischenliegenden Atomen besagten Ring bilden, der an Ring C fusioniert ist;
jeder R' unabhängig aus Wasserstoff oder einer gegebenenfalls substituierten Gruppe ausgewählt ist, die aus einer C₁₋₆ aliphatischen Gruppe, einem C₆₋₁₀-Aryl, einem Heteroarylring mit 5-10 Ringatomen oder einem Heterocyclylring mit 5-10 Ringatomen ausgewählt ist;
V -O-, -S-, -SO-, -SO₂-, -N(R⁶)SO₂-, -SO₂N(R⁶)-, N(R⁶)-, -CO-, -CO₂-, -N(R⁶)CO-, -N(R⁶)C(O)O-, -N(R⁶)CON(R⁶)-, -N(R⁶)SO₂N(R⁶)-, -N(R⁶)N(R⁶)-, -C(O)N(R⁶)-, -OC(O)N(R⁶)-, -C(R⁶)₂O-, -C(R⁶)₂S-, -C(R⁶)₂SO-, -C(R⁶)₂SO₂-, -C(R⁶)₂SO₂N(R⁶)-, -C(R⁶)₂N(R⁶)-, -C(R⁶)₂N(R⁶)C(O)-, -C(R⁶)₂N(R⁶)C(O)O-, -C(R⁶)=NN(R⁶)-, -C(R⁶)=N-O-, -C(R⁶)₂N(R⁶)N(R⁶)-, -C(R⁶)₂N(R⁶)SO₂N(R⁶)- oder -C(R⁶)₂N(R⁶)CON(R⁶)- ist;
jeder R⁶ unabhängig aus Wasserstoff oder einer gegebenenfalls substituierten C₁₋₄ aliphatischen Gruppe ausgewählt ist, oder zwei R⁶-Gruppen am selben Stickstoffatom zusammengenommen mit dem Stickstoffatom einen 5-6-gliedrigen Heterocyclyl- oder Heteroarylring bilden;
jeder R⁷ unabhängig aus Wasserstoff oder einer gegebenenfalls substituierten C₁₋₆ aliphatischen Gruppe ausgewählt ist, oder zwei R⁷ am selben Stickstoffatom zusammengenommen mit dem Stickstoffatom einen 5-8-gliedrigen Heterocyclyl- oder Heteroarylring bilden; und
jeder R⁸ unabhängig aus einer gegebenenfalls substituierten C₁₋₄ aliphatischen Gruppe, -OR⁶-, -SR⁶, -COR⁶, -SO₂R⁶, -N(R⁶)₂, -N(R⁶)N(R⁶)₂, -CN, -NO₂, -CON(R⁶)₂ oder -CO₂R⁶ ausgewählt ist.

2. Die Verbindung nach Anspruch 1, wobei B ein gegebenenfalls substituiertes Pyridyl, Pyrimidinyl, Thiazolyl, Indolyl, Imidazolyl, Oxadiazolyl, Tetrazolyl, Pyrazinyl, Triazolyl, Thienyl, Furanyl, Chinolinyl, Purinyl, Naphthyl, Benzothiazolyl, Benzopyridyl, Benzimidazolyl, Indazolyl, Isothiazolyl, Pyrazolyl, Pyridazinyl, Isoxazolyl, Benzothiophenyl oder Pyridothiophenyl ist und Q -CH₂CH₂-, -CH₂CH₂CH₂-, oder -CH₂CH₂CH₂CH₂- ist.

3. Die Verbindung nach Anspruch 2, wobei B gegebenenfalls substituiertes 2-Pyridyl ist und Verbindungen die allgemeine Formel **II** besitzen: 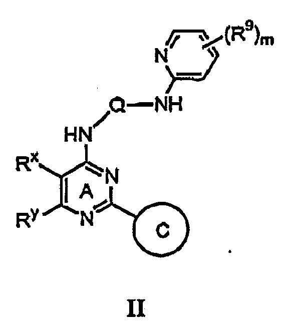 worin m 0 bis 4 ist und jeder vorkommende R⁹ unabhängig aus H, Nitro, Amino, Cyano, Halogen, Thioamido, Amidino, Oxamidino, Alkoxyamidino, Imidino, Guanidino, Sulfonamido, Carboxyl, Formyl, Alkyl, Halogenalkyl, Alkoxy, Halogenalkoxy, Alkoxyalkyl, Alkylaminoalkoxy, Alkylcarbonyl, Aralkylcarbonyl, Heteroaralkylcarbonyl, Alkylthio, Aryl, Aralkyl, Heteroaryl oder Heteroaralkyl ausgewählt ist.

4. Die Verbindung nach Anspruch 3, wobei R⁹-Substituenten aus Wasserstoff, C₁₋₄-Alkyl, Nitro, Amino, Cyano, Cyanmethyl, Trifluormethyl, Hydroxy oder Methoxy ausgewählt sind.

5. Die Verbindung nach Anspruch 2, wobei B Indazolyl, Pyrazolyl oder Thiazolyl ist, das gegebenenfalls mit einem oder mehreren unabhängig voneinander vorkommenden R⁹ substituiert ist, wobei R⁹-Substituenten aus Wasserstoff, C₁₋₄-Alkyl, Nitro, Amino, Cyano, Cyanmethyl, Trifluormethyl, Hydroxy oder Methoxy ausgewählt sind.

6. Die Verbindung nach Anspruch 2, wobei optionale Substituenten an Ring C aus R¹ oder R⁵ ausgewählt sind, wobei jeder vorkommende R¹ unabhängig aus -Halogen, einer gegebenenfalls substituierten C₁₋₆ aliphatischen Gruppe, Phenyl, -COR⁶, -OR⁶, -CN, -SO₂R⁶, -SO₂NH₂, -N(R⁶)₂, -CO₂R⁶, -CONH₂, -NHCOR⁶, -OC(O)NH₂ oder -NHSO₂R⁶ ausgewählt ist; und jeder vorkommende R⁵ aus -Halogen, -CN, -NO₂, -N(R⁴)₂, gegebenenfalls substituierter C₁₋₆ aliphatischer Gruppe, -OR', -C(O)R', -CO₂R', -CONH(R⁴), -N(R⁴)COR', -SO₂N(R⁴)₂ oder -N(R⁴)SO₂R' ausgewählt ist.

7. Die Verbindung nach Anspruch 6, wobei R¹-Gruppen aus -CF₃, -Cl, -F, -CN, -COCH₃, -OCH₃, -OH, -CH₂CH₃, -OCH₂CH₃, -CH₃, -CF₂CH₃, Cyclohexyl, t-Butyl, Isopropyl, Cyclopropyl, -C≡CH, -C≡C-CH₃, -SO₂CH₃, -SO₂NH₂, -N(CH₃)₂, -CO₂CH₃, -CONH₂, -NHCOCH₃, -OC(O)NH₂, -NHSO₂CH₃, oder -OCF₃ ausgewählt sind; und R⁵-Gruppen aus -Cl, -F, -CN, -CF₃, -NH₂, -NH(C₁₋₄ aliphatisch), -N(C₁₋₄ aliphatisch)₂, -O(C₁₋₄ aliphatisch), -C₁₋₄ aliphatisch oder -CO₂(C₁₋₄ aliphatisch) ausgewählt sind.

8. Die Verbindung nach Anspruch 2, die eine der Formeln besitzt: 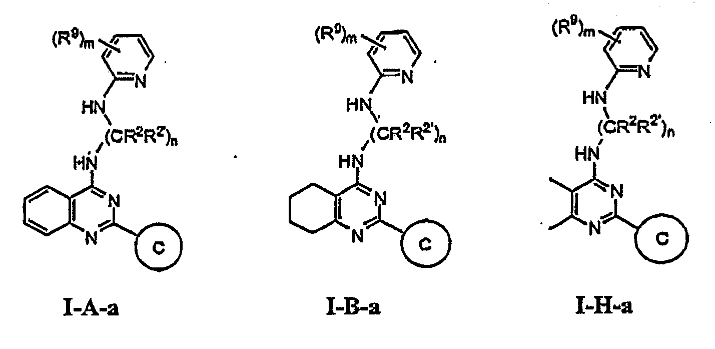 worin m 0 bis 4 ist, und jeder vorkommende R⁹ unabhängig aus H, Nitro, Amino, Cyano, Halogen, Thioamido, Amidino, Oxamidino, Alkoxyamidino, Imidino, Guanidino, Sulfonamido, Carboxyl, Formel, Alkyl, Halogenalkyl, Alkoxy, Halogenalkoxy, Alkoxyalkyl, Alkylaminoalkoxy, Alkylcarbonyl, Aralkylcarbonyl, Heteroaralkylcarbonyl, Alkylthio, Aryl, Aralkyl, Heteroaryl oder Heteroaralkyl ausgewählt ist.

9. Die Verbindung nach Anspruch 8, wobei Ring C ein Phenylring ist und R¹ Halogen, Methyl, Cyano, OMe, OH oder Trifluormethyl ist.

10. Die Verbindung nach Anspruch 2, wobei Ring C ein Phenyl- oder Pyridinylring mit einem oder zwei ortho-Substituenten ist, die unabhängig voneinander aus -R¹ ausgewählt sind, wobei Ring C außerdem mit -R⁵ substituiert ist und wobei, wenn Ring C und zwei benachbarte Substituenten daran ein bicyclisches Ringsystem bilden, das bicyclische Ringsystem aus einem Naphthyl-, Chinolinyl- oder Isochinolinylring ausgewählt ist.

11. Die Verbindung nach Anspruch 2 oder Anspruch 10, wobei R¹ -Halogen, eine gegebenenfalls substituierte C₁₋₆ aliphatische Gruppe, Phenyl, -COR⁶, -OR⁶, -CN, -SO₂R⁶, -SO₂NH₂, -N(R⁶)₂, -CO₂R⁶, -CONH₂, -NHCOR⁶, -OC(O)NH₂ oder -NHSO²R₆ ist.

12. Die Verbindung nach Anspruch 2, wobei Ring C ein Phenyl- oder Pyridinylring mit einem oder zwei ortho-Substituenten ist, die unabhängig voneinander aus -R¹ ausgewählt sind, wobei Ring C außerdem mit -R⁵ substituiert ist und wobei, wenn Ring C und zwei benachbarte Substituenten daran ein bicyclisches Ringsystem bilden, das bicyclische Ringsystem ein Naphthylring ist.

13. Die Verbindung nach Anspruch 2 oder Anspruch 12, wobei R¹ -Halogen, eine C₁₋₆ halogenaliphatische Gruppe, eine C₁₋₆ aliphatische Gruppe, Phenyl, OMe, OH oder -CN ist.

14. Die Verbindung nach einem der Ansprüche 2, 12 oder 13, wobei jeder R⁵ unabhängig aus -Halogen, -CN, -NO₂, -N(R⁴)₂, gegebenenfalls substituierter C₁₋₆ aliphatischer Gruppe, -OR', -C(O)R', CO₂R'; -CONH(R⁴), -N(R⁴)COR', -SO₂N(R⁴)₂ oder -N(R⁴)SO₂R' ausgewählt ist.

15. Die Verbindung nach Anspruch 2, wobei B eine gegebenenfalls substituierte Pyridylgruppe ist, die gegebenenfalls mit 0, 1 oder 2 vorkommenden R⁹ substituiert ist, wobei jeder vorkommende R⁹ unabhängig Wasserstoff, C₁₋₄-Alkyl, Nitro, Amino, Cyano, Cyanmethyl, Trifluormethyl, Hydroxy oder Methoxy ist.

16. Die Verbindung nach Anspruch 15, wobei Ring C ein Phenyl- oder Pyridinylring mit einem oder zwei ortho-Substituenten ist, die unabhängig voneinander aus -R¹ ausgewählt sind, wobei Ring C außerdem mit -R⁵ substituiert ist und wobei, wenn Ring C und zwei benachbarte Substituenten daran ein bicyclisches Ringsystem bilden, das bicyclische Ringsystem ein Naphthylring ist.

17. Die Verbindung nach einem der Ansprüche 2, 15 oder 16, wobei R¹ -Halogen, eine C₁₋₆ halogenaliphatische Gruppe, eine C₁₋₆ aliphatische Gruppe, Phenyl, OMe, OH oder -CN ist.

18. Die Verbindung nach einem der Ansprüche 2, 15, 16 oder 17, wobei jeder R⁵ unabhängig aus -Halogen, -CN, -NO₂, -N(R⁴)₂, gegebenenfalls substituierter C₁₋₆ aliphatischer Gruppe, -OR', -C(O)R', CO₂R'; -CONH(R⁴), -N(R⁴)COR', -SO₂N(R⁴)₂ oder -N(R⁴)SO₂R' ausgewählt ist.

19. Die Verbindung nach Anspruch 2, wobei B eine gegebenenfalls substituierte Pyridylgruppe ist, die gegebenenfalls mit 2 unabhängig voneinander vorkommenden R⁹ substituiert ist, wobei jeder vorkommende R⁹ unabhängig Wasserstoff, C₁₋₄-Alkyl, Nitro, Amino, Cyano, Cyanmethyl, Trifluormethyl, Hydroxy oder Methoxy ist.

20. Die Verbindung nach Anspruch 2 oder Anspruch 19, wobei Ring C ein Phenylring mit einem oder zwei ortho-Substituenten ist, die unabhängig voneinander aus -R¹ ausgewählt sind, wobei Ring C außerdem mit -R⁵ substituiert ist.

21. Die Verbindung nach einem der Ansprüche 2, 19 oder 20, wobei R¹ -Halogen, eine gegebenenfalls mit Halogen substituierte C₁₋₄ aliphatische Gruppe, OMe, OH oder -CN ist.

22. Die Verbindung nach einem der Ansprüche 2, 19, 20 oder 21, wobei jeder R⁵ unabhängig aus -Cl, -F, -CN, -CF₃, -NH₂, -NH(C₁₋₄ aliphatisch), -N(C₁₋₄ aliphatisch)₂, -O(C₁₋₄ aliphatisch), gegebenenfalls substituierter C₁₋₄ aliphatischer Gruppe und -CO₂(C₁₋₄ aliphatisch) ausgewählt ist.

23. Die Verbindung nach Anspruch 1, die aus einer der Verbindungen ausgewählt ist:

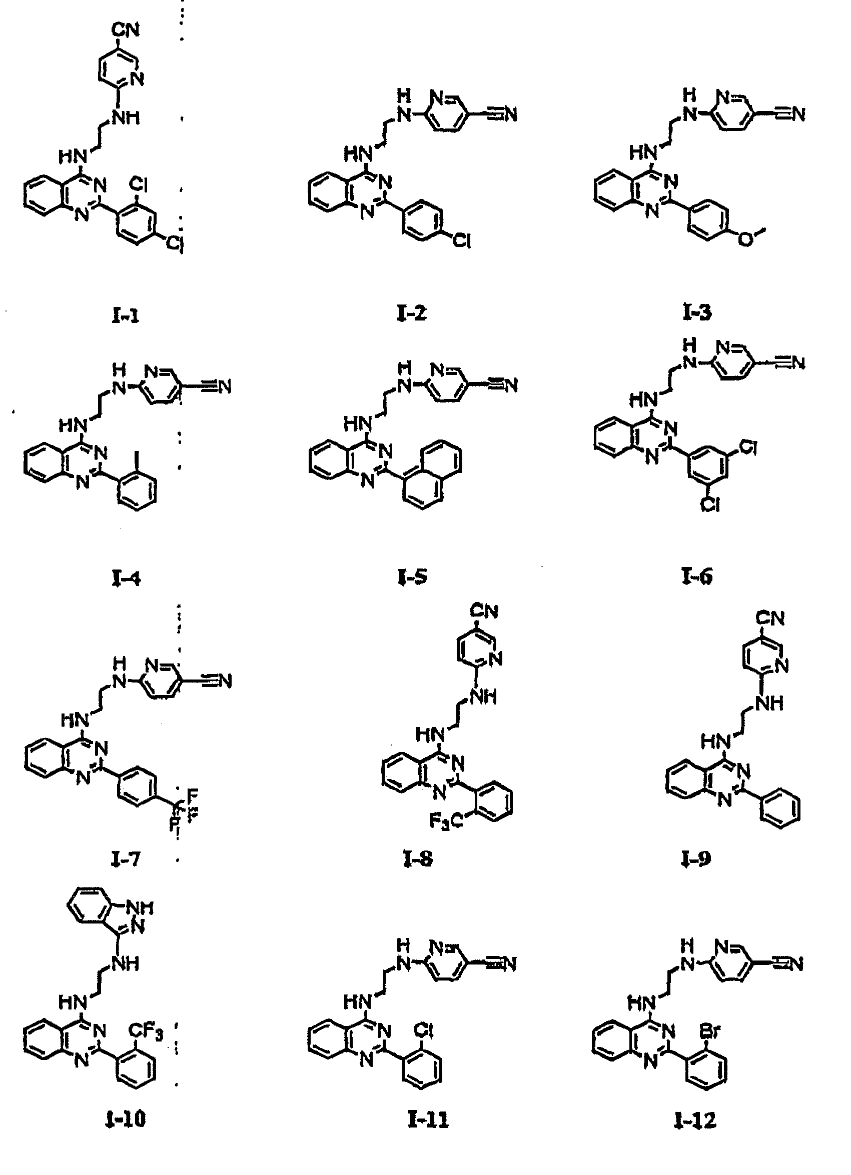

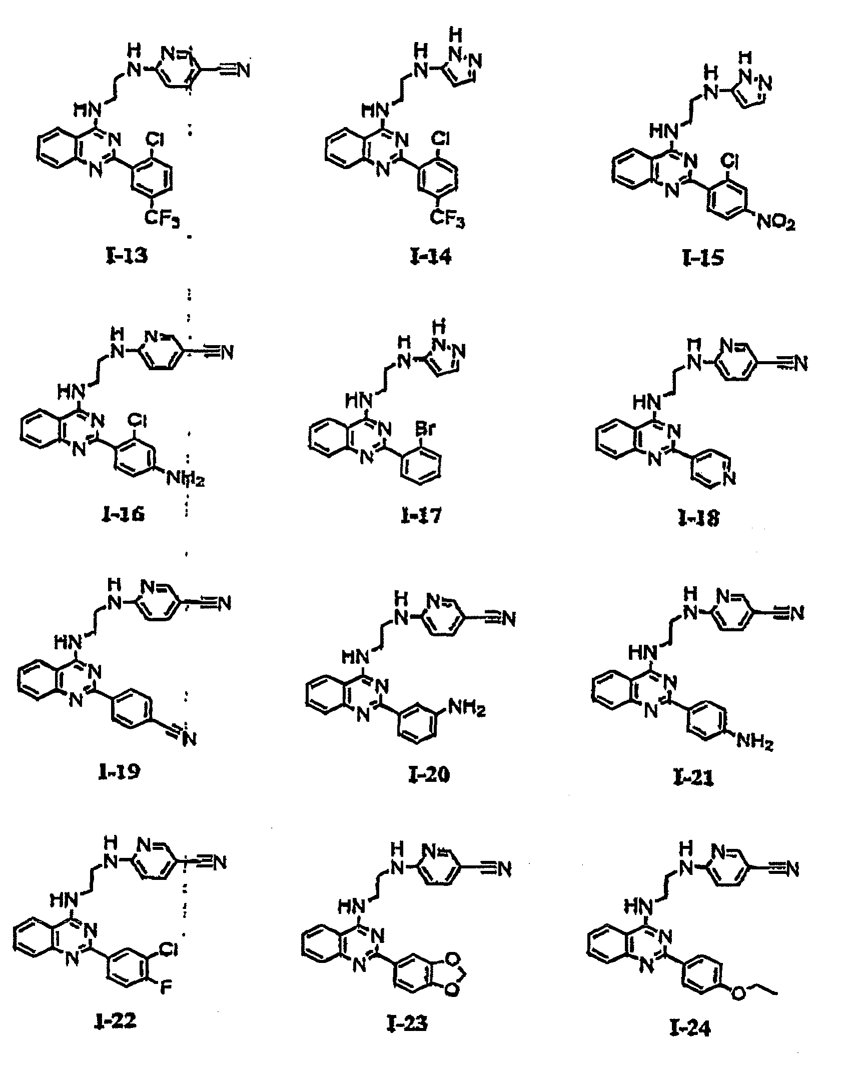

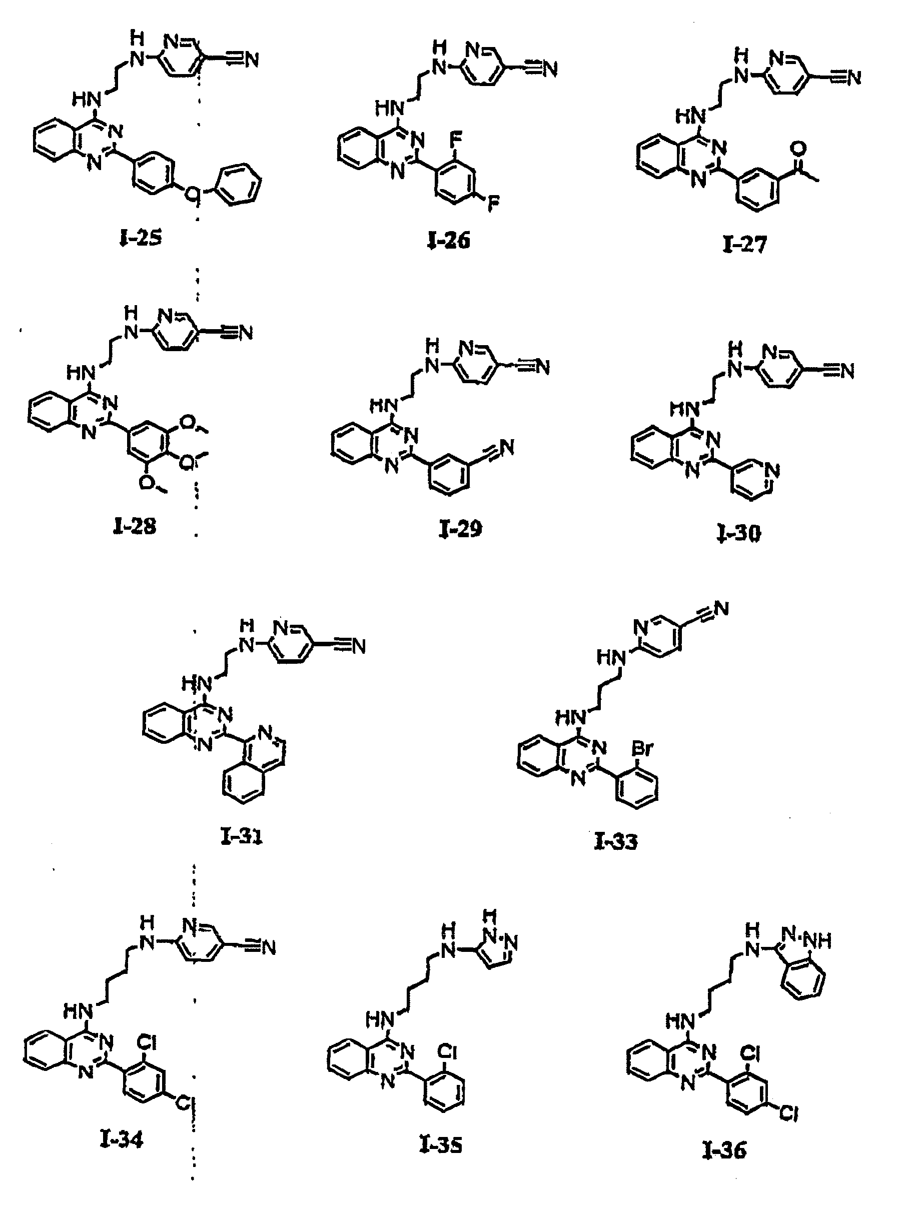

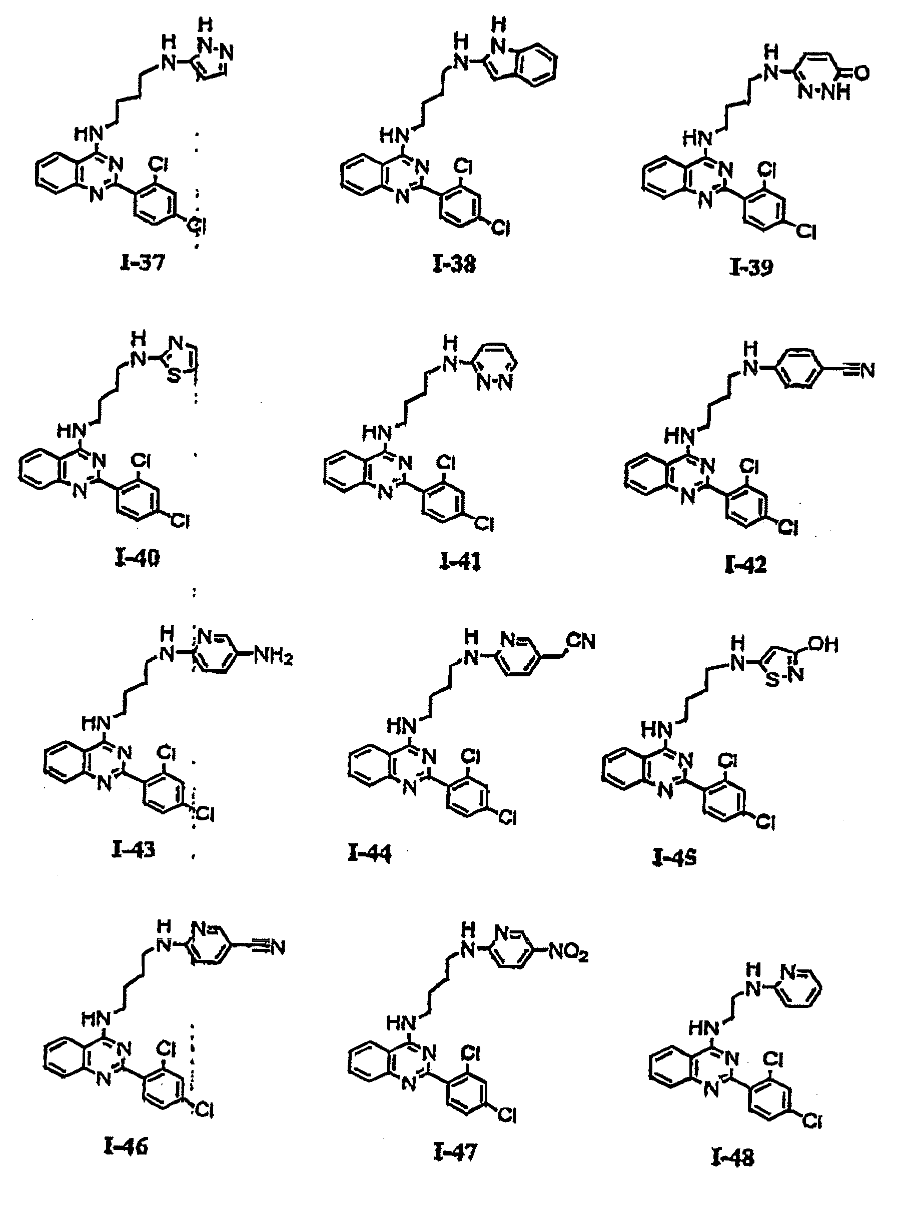

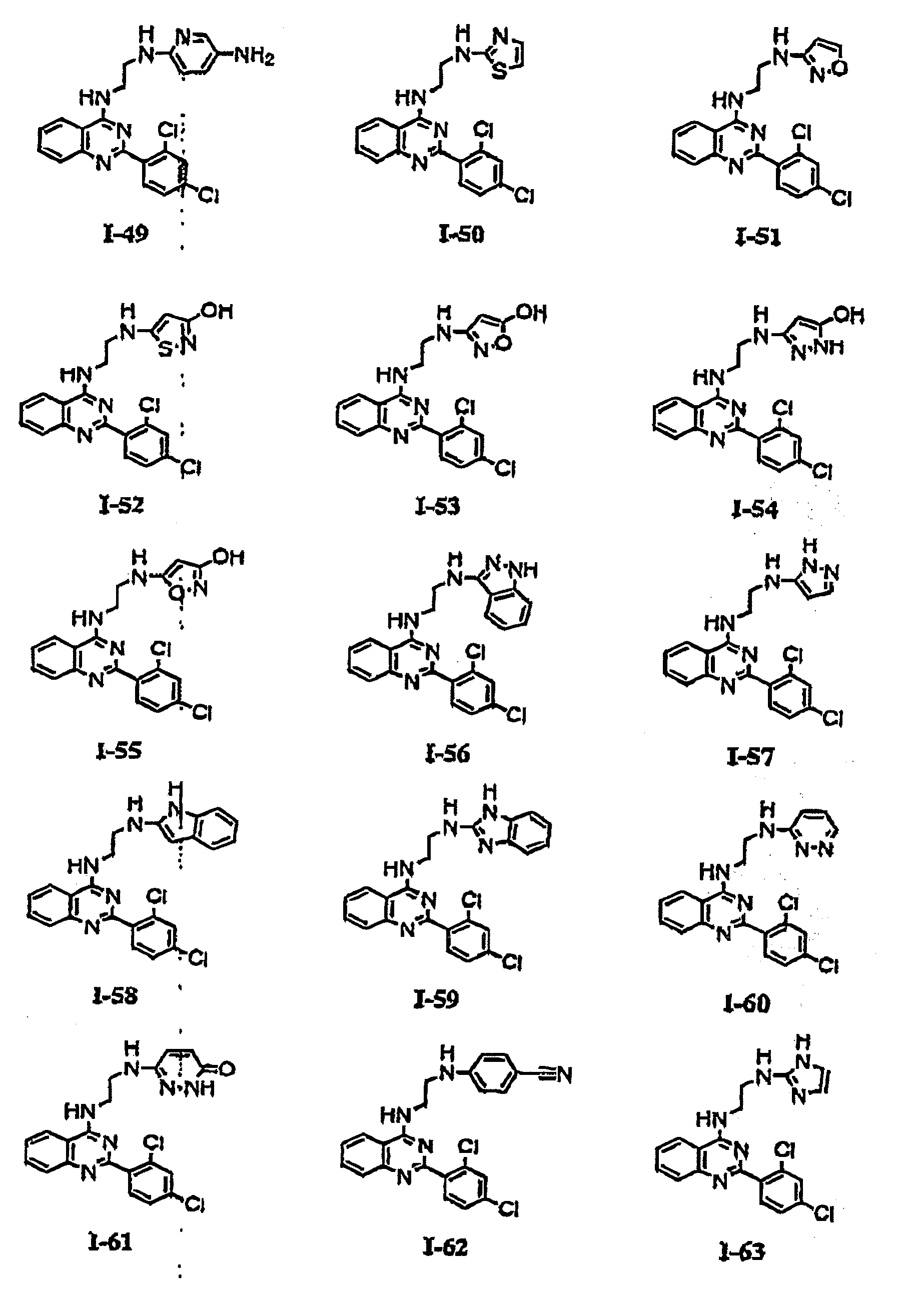

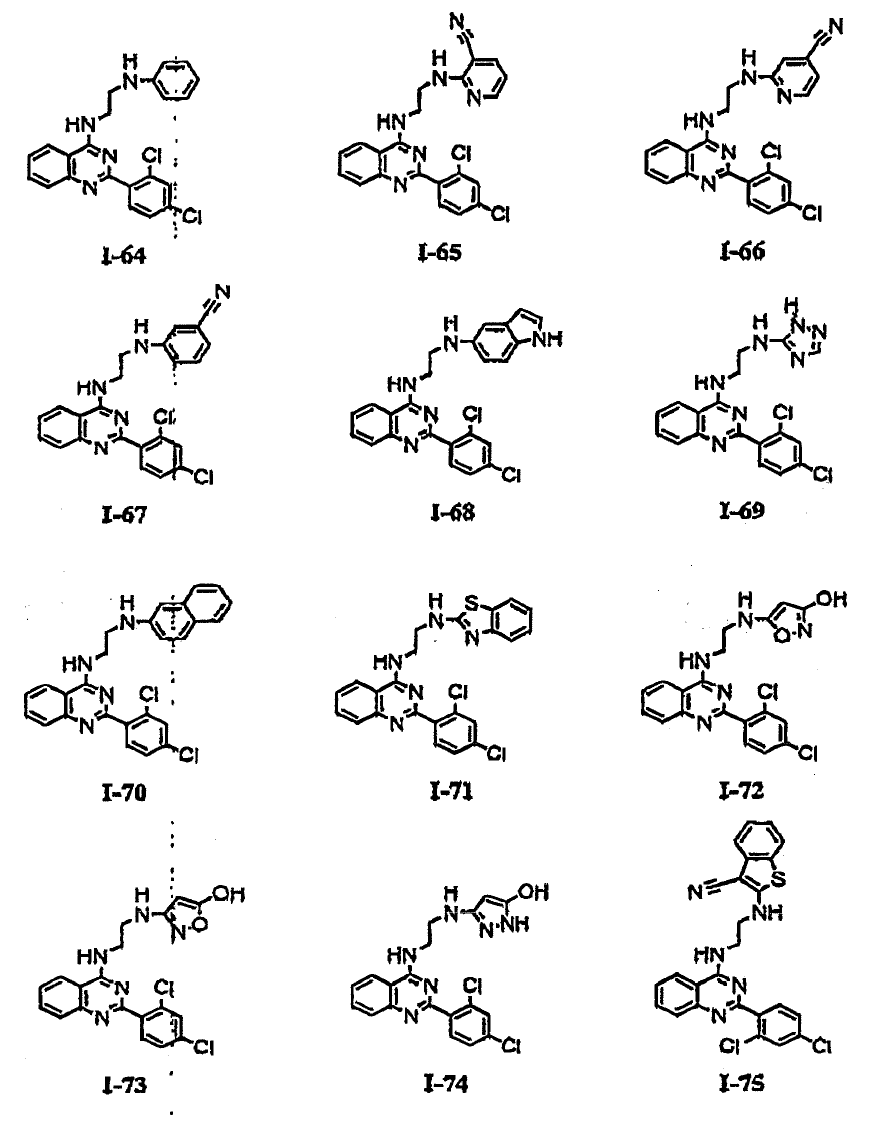

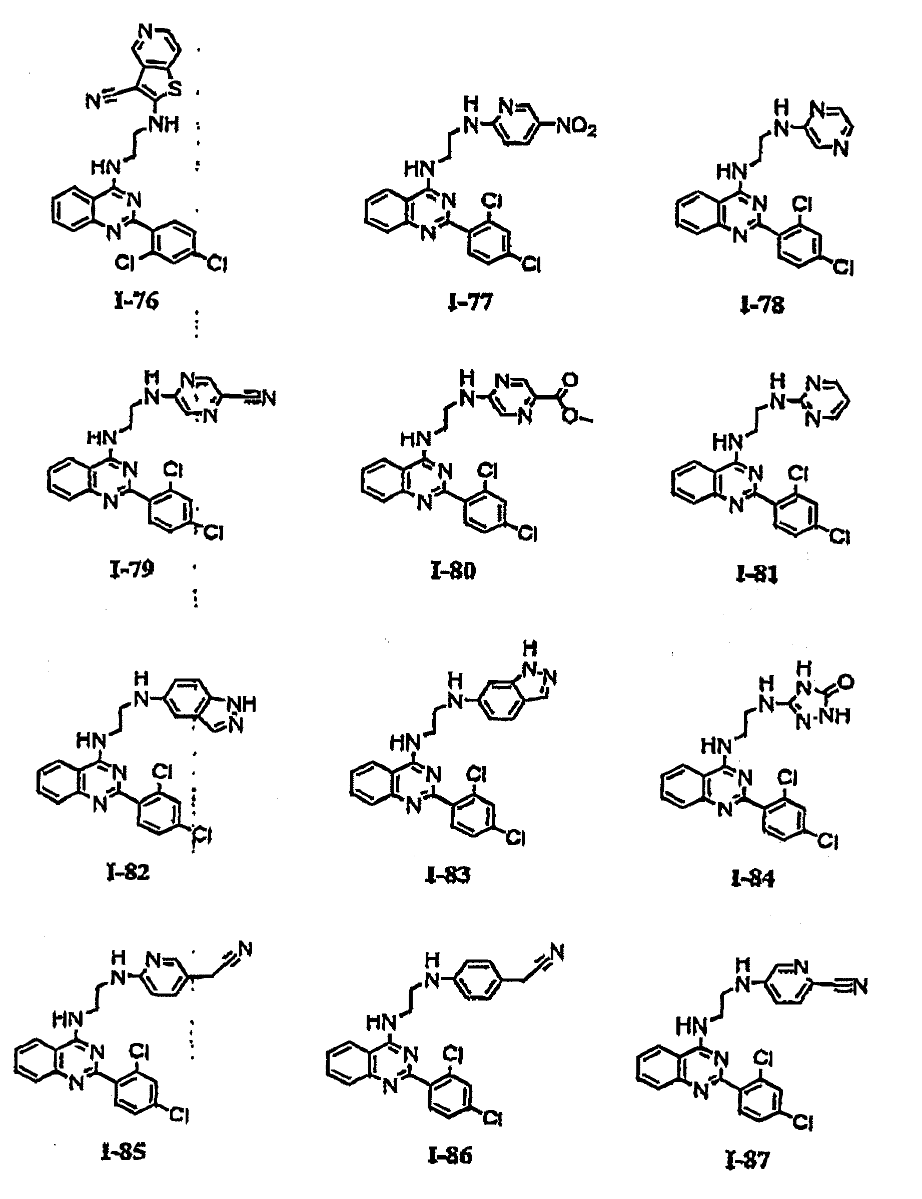

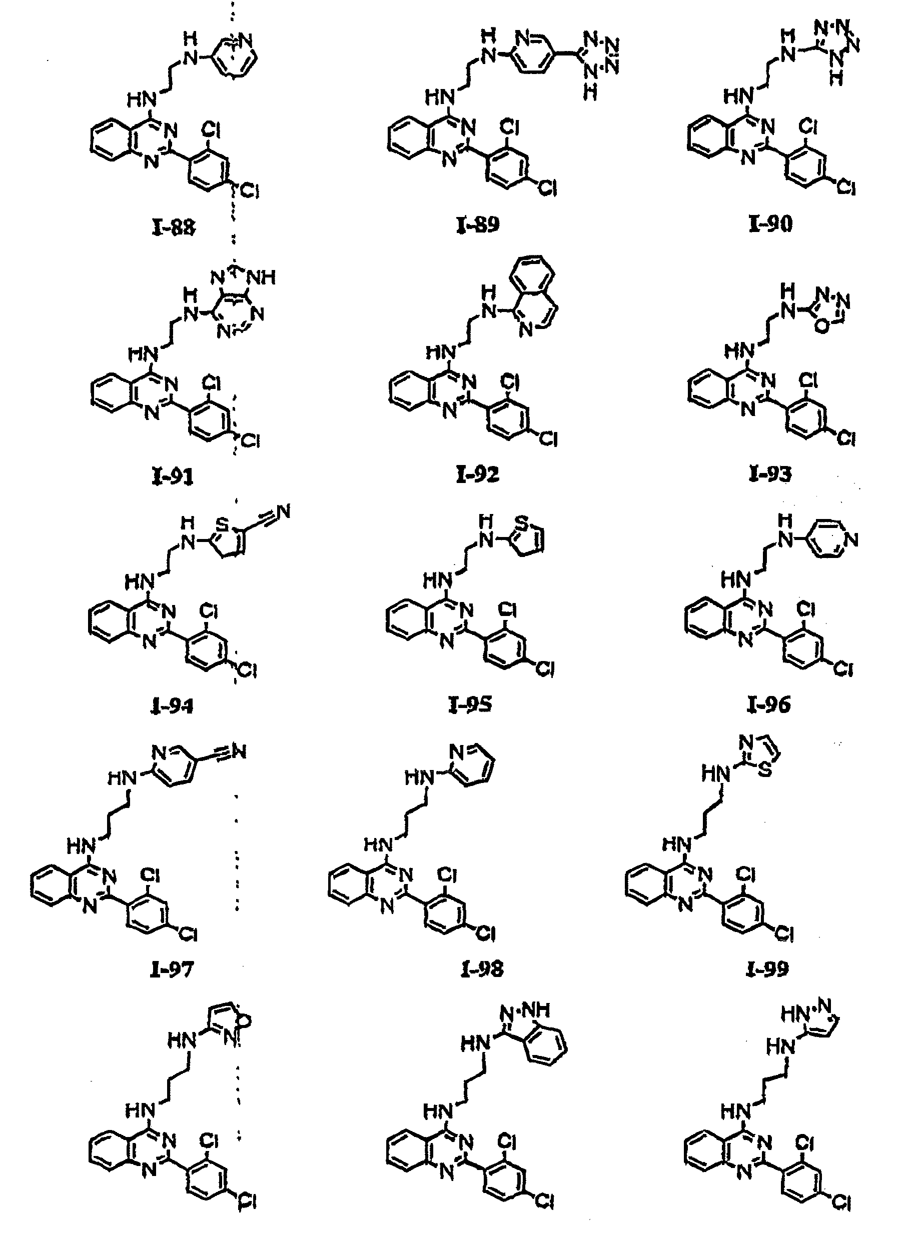

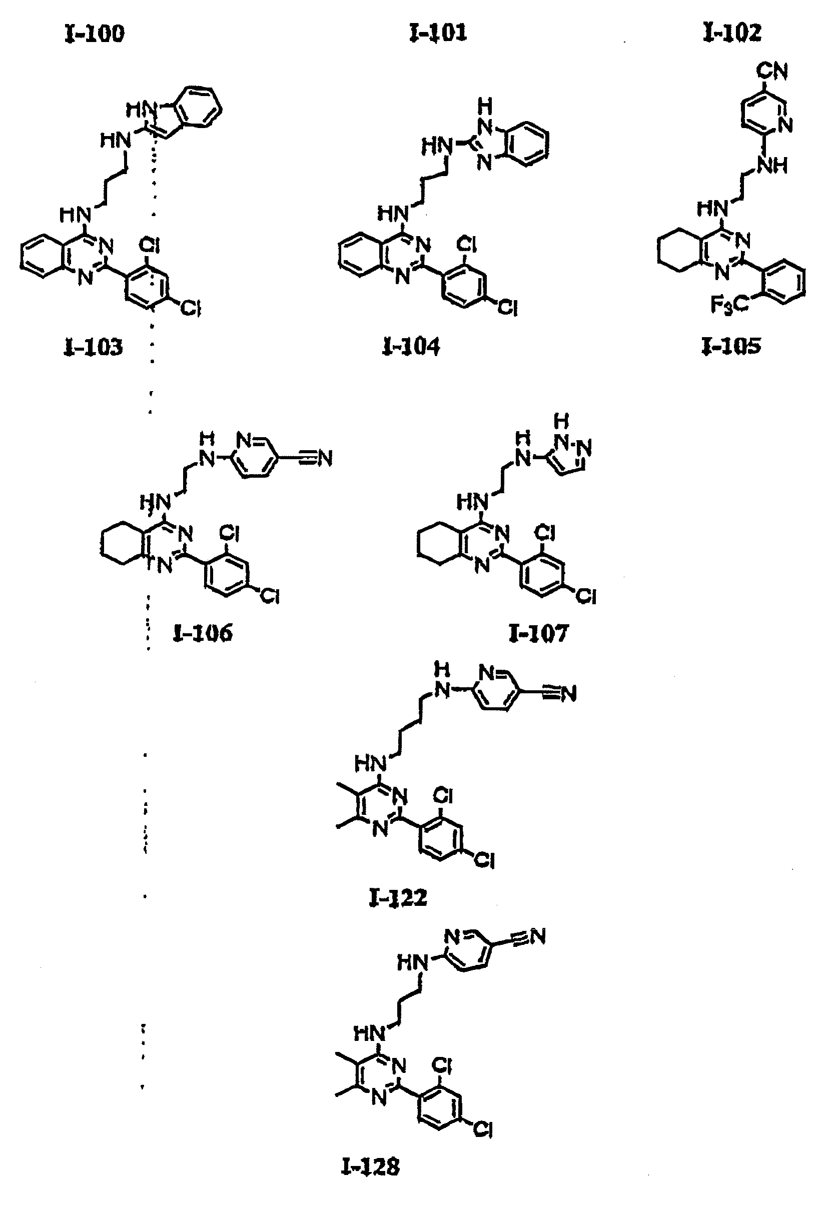

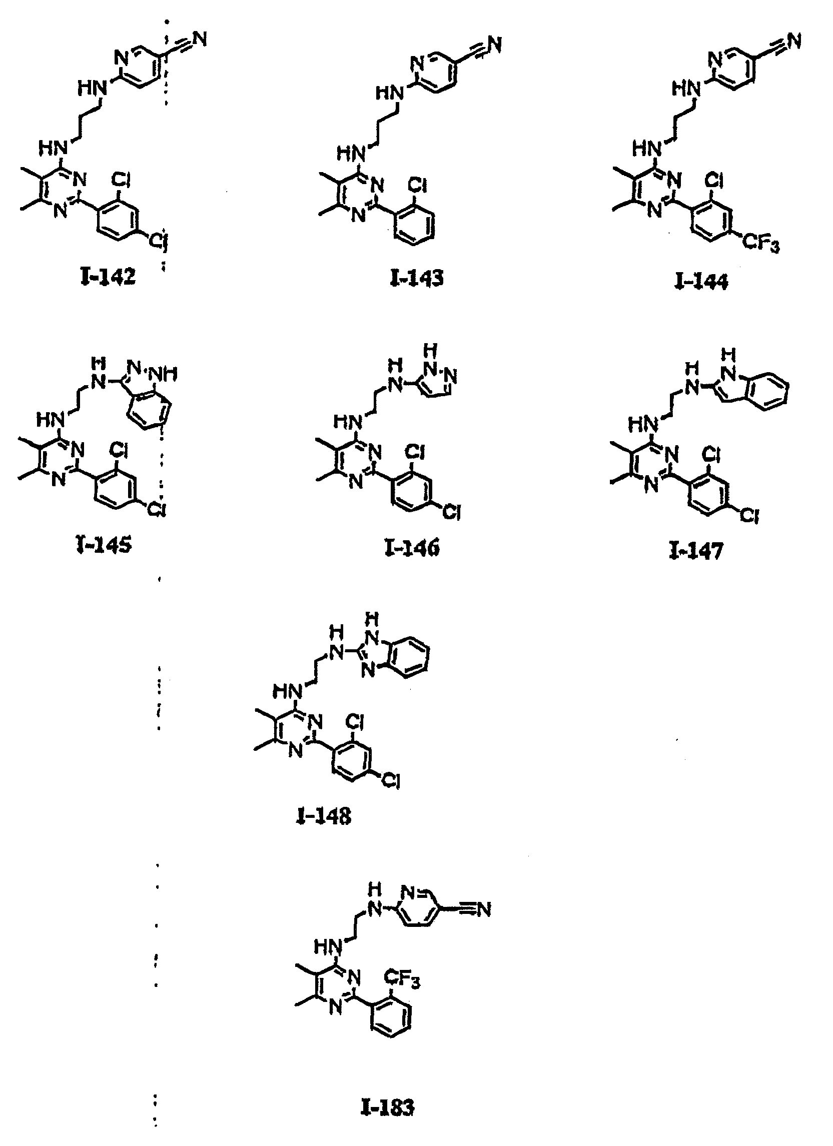

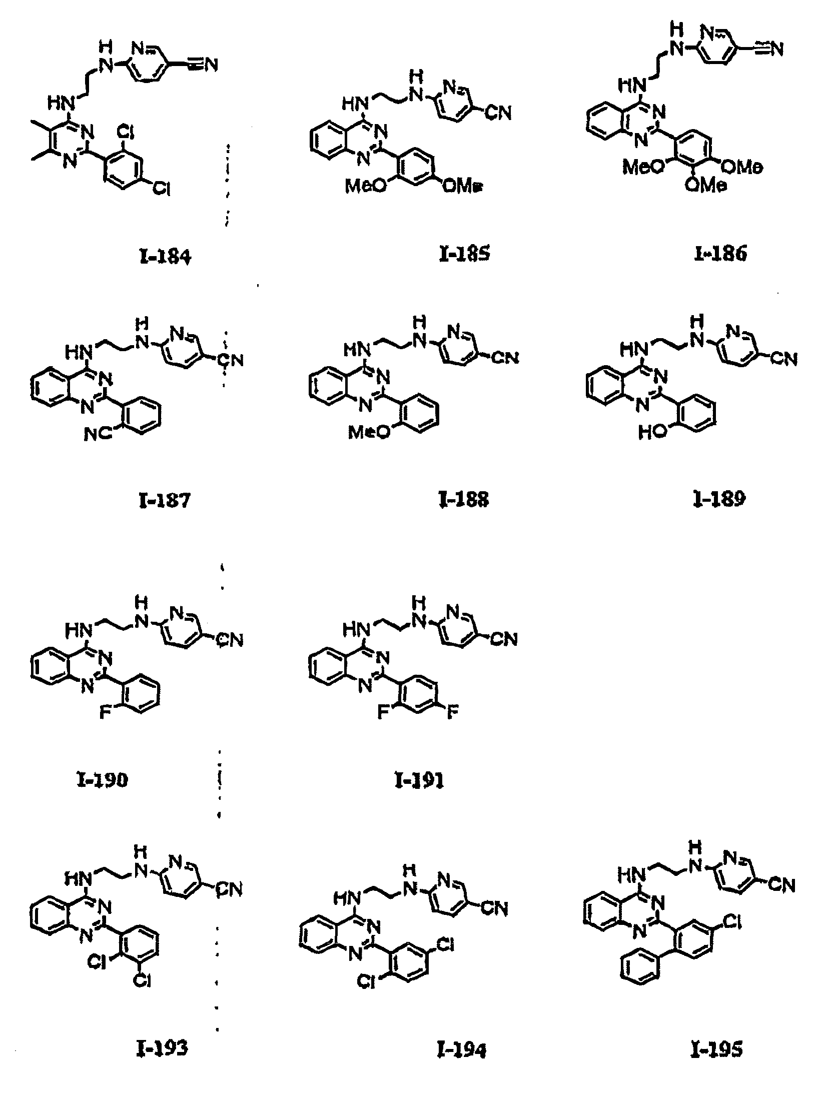

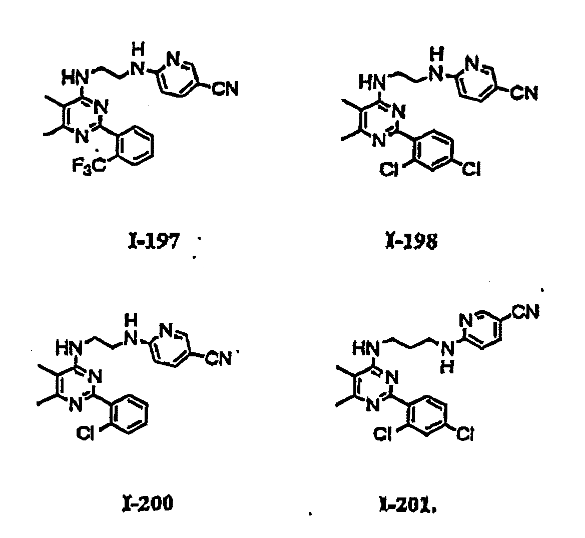

24. Eine Zusammensetzung, umfassend eine Verbindung nach Anspruch 2 und einen pharmazeutisch verträglichen Träger, ein pharmazeutisch verträgliches Adjuvans oder Vehikel.

25. Eine Zusammensetzung, umfassend eine therapeutisch wirksame Menge einer Verbindung nach Anspruch 2 und einen pharmazeutisch verträglichen Träge, ein pharmazeutisch verträgliches Adjuvans oder Vehikel.

26. Die Zusammensetzung nach Anspruch 25, wobei die therapeutisch wirksame Menge eine Menge ist, die in der Lage ist, GSK-3-Aktivität zu hemmen.

27. Die Zusammensetzung nach Anspruch 24, außerdem umfassend ein oder mehrere weitere Therapeutika.

28. Ein Verfahren zur Hemmung von GSK-3-Kinaseaktivität in einer biologischen Probe, umfassend den Schritt des Inkontaktbringens besagter biologischer Probe mit:
a) einer Zusammensetzung gemäß Anspruch 24; oder
b) einer Verbindung gemäß Anspruch 2.

29. Die Verwendung von: a) einer Zusammensetzung gemäß Anspruch 24; oder b) einer Verbindung gemäß Anspruch 2 zur Herstellung eines Medikaments zur Behandlung oder Minderung der Schwere einer Autoimmunkrankheit, einer Entzündungskrankheit, einer Stoffwechselstörung, einer neurologischen oder neurodegenerativen Störung oder einer kardiovaskulären Erkrankung in einem Patienten.

30. Die Verwendung nach Anspruch 29, wobei die Erkrankung aus Allergie, Asthma, Diabetes, Alzheimer Krankheit, Chorea Huntington, Parkinson Krankheit, AIDSassoziierter Demenz, amyotropher Lateralsklerose (AML, Lou-Gehrig-Krankheit), Multipler Sklerose (MS), Schizophrenie, Hypertrophie von Kardiomyozyten, Reperfusion/Ischämie, Schlaganfall oder Haarausfall ausgewählt ist.

31. Die Verwendung nach Anspruch 30, wobei die Erkrankung Schlaganfall ist.

32. Die Verwendung nach Anspruch 29, wobei die Erkrankung eine neurologische oder neurodegenerative Störung ist.

33. Die Verwendung nach Anspruch 29, wobei besagtes Medikament mit einem weiteren Therapeutikum kombiniert ist, das aus einer Behandlung von Alzheimer Krankheit, einer Behandlung von Parkinson Krankheit, einem Agens zur Behandlung von Multipler Sklerose (MS), einer Behandlung von Asthma, einem entzündungshemmenden Agens, einem Immunmodulator oder Immunsuppressivum, einem neurotrophen Faktor, einem Agens zur Behandlung von Schlaganfall, einem Agens zur Behandlung von kardiovaskulärer Erkrankung oder einem Agens zur Behandlung von Diabetes ausgewählt ist, wobei besagtes weiteres Therapeutikum für die zu behandelnde Erkrankung geeignet ist.

## Revendications

1. Composé de formule I : 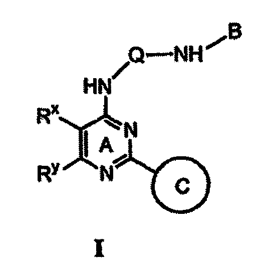 ou sel pharmaceutiquement acceptable de celui-ci, dans lequel :
le cycle A, pris avec R^{x} et R^{y}, est choisi parmi 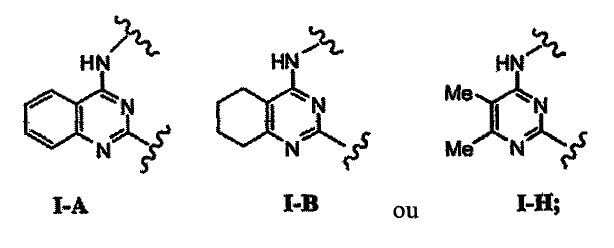
B est un groupe pyridyle; pyrimidinyle, thiazolyle, indolyle, imidazolyle, oxadiazolyle, tétrazolyle, pyrazinyle, triazolyle, thiényle, furanyle, quinoléinyle, purinyle, naphtyle, benzothiazolyle, benzopyridyle, benzimidazolyle, indazolyle, isothiazolyle, pyrazolyle, pyridazinyle, isoxazolyle, phényle, benzothiophényle ou pyridothiophényle facultativement substitué ; où B est facultativement substitué par un groupe nitro, amino, cyano, halogéno, thioamido, amidino, oxamidino, alcoxyamidino, imidino, guanidino, sulfonamido, carboxyle, formyle, alkyle, halogénoalkyle, alcoxy, halogénoalcoxy, alcoxyalkyle, alkylaminoalcoxy, alkylcarbonyle, aralkylcarbonyle, hétéroaralkylcarbonyle, alkylthio, aminoalkyle, cyanoalkyle, hydroxy, alcoxycarbonyle, aryle, aralkyle, hétéroaryle ou hétéroaralkyle ;
Q est une chaîne alkylidène en C_{1 à 4} où chaque motif méthylène dudit Q est substitué par R² et R^{2'} ;
chaque R² et R^{2'} est H ;
le cycle C est choisi parmi un groupe phényle ou pyridinyle facultativement substitué, où ledit cycle C comporte facultativement un ou deux substituants ortho indépendamment choisis parmi -R¹, ou toute position carbone substituable sur le cycle C est facultativement indépendamment substituée par -R⁵, ou deux substituants adjacents sur le cycle C sont facultativement pris avec leurs atomes intervenants pour former un cycle naphtyle, quinoléinyle, isoquinoléinyle ou benzodioxolyle, ledit cycle étant facultativement substitué par un groupe halogéno, oxo ou -R⁸ ;
R¹ est choisi parmi un groupe halogéno, -CN, -NO₂, T-V-R⁶, un groupe phényle, un cycle hétéroaryle à 5 à 6 chaînons, un cycle hétérocyclyle à 5 à 6 chaînons, ou un groupe aliphatique en C_{1 à 6}, lesdits cycles phényle, hétéroaryle, et hétérocyclyle étant chacun facultativement substitués par jusqu'à trois groupes indépendamment choisis parmi halogéno, oxo ou -R⁸, ledit groupe aliphatique en C_{1 à 6} étant facultativement substitué par un groupe halogéno, cyano, nitro ou un atome d'oxygène ou R¹ et un substituant adjacent pris avec leurs atomes intervenants forment ledit cycle condensé avec le cycle C ;
R est un groupe facultativement substitué choisi parmi un groupe aliphatique C_{1 à 10}, aryle, aralkyle, hétéroaryle ou hétéroaralkyle ;
T est une liaison de valence ou une chaîne alkylidène en C_{1 à 4} ;
chaque R⁴ est indépendamment choisi parmi -R⁷, -COR⁷, -CO₂(aliphatique en C_{1 à 6}), -CON(R⁷)₂ ou -SO₂R⁷ ou deux R⁴ sur le même atome d'azote sont pris ensemble pour former un cycle hétérocyclyle ou hétéoaryle à 5 à 8 chaînons ;
chaque R⁵ est indépendamment choisi parmi -R', un groupe halogéno, -OR', -C(=O)R', -CO₂R', -COCOR', -NO₂, -CN, -S(O)R', -SO₂R', -SR', -N(R⁴)₂, -CON(R⁴)₂, -SO₂N(R⁴)₂, -OC(=O)R', -N(R⁴)COR', -N(R⁴)CO₂(aliphatique en C_{1 à 6} facultativement substitué), -N(R⁴)N(R⁴)₂, -C=NN(R⁴)₂, -C=N-OR', -N(R⁴)CON(R⁴)₂, -N(R⁴)SO₂N(R⁴)₂, -N(R⁴)SO₂R' ou -OC(=O)N(R⁴)₂, ou R⁵ et un substituant adjacent pris ensemble avec leurs atomes intervenants forment ledit cycle condensé avec le cycle C ;
chaque R' est indépendamment choisi parmi un atome d'hydrogène ou un groupe facultativement substitué choisi parmi un groupe aliphatique en C_{1 à 6}, un groupe aryle en C_{6 à 10}, un cycle hétéroaryle comportant 5 à 10 atomes de cycle ou un cycle hétérocyclyle comportant 5 à 10 atomes de cycle ;
V est -O-, -S-, -SO-, -SO₂-, -N(R⁶)SO₂-, -SO₂N(R⁶)-, -N(R⁶)-, -CO-, -CO₂-, -N(R⁶)CO-, -N(R⁶)C(O)O-, -N(R⁶)CON(R⁶)-, -N(R⁶)SO₂N(R⁶)-, -N(R⁶)N(R⁶)-, -C(O)N(R⁶)-, -OC(O)N(R⁶)-, -C(R⁶)₂O-, -C(R⁶)₂S-, -C(R⁶)₂SO-, -C(R⁶)₂SO₂-, -C(R⁶)₂SO₂N(R⁶)-, -C(R⁶)₂N(R⁶)-, -C(R⁶)₂N(R⁶)C(O)-, -C(R⁶)₂N(R⁶)C(O)O-, -C(R⁶)=NN(R⁶)-, -C(R⁶)=N-O-, -C(R⁶)₂N(R⁶)N(R⁶)-, -C(R⁶)₂N(R⁶)SO₂N(R⁶)- ou -C(R⁶)₂N(R⁶)CON(R⁶)-;
chaque R⁶ est indépendamment choisi parmi un atome d'hydrogène ou un groupe aliphatique en C_{1 à 4} facultativement substitué, ou deux groupes R⁶ sur le même atome d'azote sont pris ensemble avec l'atome d'azote pour former un cycle hétérocyclyle ou hétéroaryle à 5 à 6 chaînons ;
chaque R⁷ est indépendamment choisi parmi un atome d'hydrogène ou un groupe aliphatique en C_{1 à 6} facultativement substitué, ou deux R⁷ sur le même atome d'azote sont pris ensemble avec l'atome d'azote pour former un cycle hétérocyclyle ou hétéroaryle à 5 à 8 chaînons ; et
chaque R⁸ est indépendamment choisi parmi un groupe aliphatique en C_{1 à 4} facultativement substitué, -OR⁶, -SR⁶, -COR⁶, -SO₂R⁶, -N(R⁶)₂, -N(R⁶)N(R⁶)₂, -CN, -NO₂, -CON(R⁶)₂ ou -CO₂R⁶.

2. Composé selon la revendication 1, dans lequel B est un groupe pyridyle, pyrimidinyle, thiazolyle, indolyle, imidazolyle, oxadiazolyle, tétrazolyle, pyrazinyle, triazolyle, thiényle, furanyle, quinoléinyle, purinyle, naphtyle, benzothiazolyle, benzopyridyle, benzimidazolyle, indazolyle, isothiazolyle, pyrazolyle, pyridazinyle, isoxazolyle, benzothiophényle ou pyridothiophényle facultativement substitué et Q est -CH₂CH₂-, -CH₂CH₂CH₂- ou -CH₂CH₂CH₂CH₂-.

3. Composé selon la revendication 2, dans lequel B est un groupe 2-pyridyle facultativement substitué et les composés ont la formule générale II : 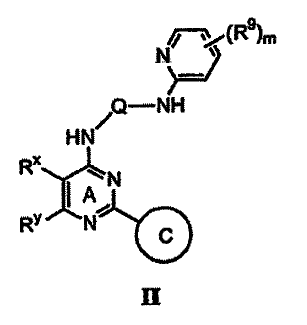 dans laquelle m vaut 0 à 4, et chaque occurrence de R⁹ est indépendamment choisie parmi H, un groupe nitro, amino, cyano, halogéno, thioamido, amidino, oxamidino, alcoxyamidino, imidino, guanidino, sulfonamido, carboxyle, formyle, alkyle, halogénoalkyle, alcoxy, halogénoalcoxy, alcoxyalkyle, alkylaminoalcoxy, alkylcarbonyle, aralkylcarbonyle, hétéroaralkylcarbonyle, alkylthio, aryle, aralkyle, hétéroaryle ou hétéroaralkyle.

4. Composé selon la revendication 3, dans lequel les substituants R⁹ sont choisis parmi un atome d'hydrogène, un groupe alkyle en C_{1 à 4}, nitro, amino, cyano, cyanométhyle, trifluorométhyle, hydroxy ou méthoxy.

5. Composé selon la revendication 2, dans lequel B est un groupe indazolyle, pyrazolyle ou thiazolyle facultativement substitué par une ou plusieurs occurrences indépendantes de R⁹, où les substituants R⁹ sont choisis parmi un atome d'hydrogène, un groupe alkyle en C_{1 à 4}, nitro, amino, cyano, cyanométhyle, trifluorométhyle, hydroxy ou méthoxy.

6. Composé selon la revendication 2, dans lequel les substituants facultatifs sur le cycle C sont choisis parmi R¹ ou R⁵, où chaque occurrence de R¹ est indépendamment choisie parmi un groupe halogéno, un groupe aliphatique en C_{1 à 6} facultativement substitué, phényle, -COR⁶, -OR⁶, -CN, -SO₂R⁶, -SO₂NH₂, -N(R⁶)₂, -CO₂R⁶, -CONH₂, -NHCOR⁶, -OC(O)NH₂ ou -NHSO₂R⁶ ; et chaque occurrence de R⁵ est choisie parmi un groupe halogéno, -CN, -NO₂, -N(R⁴)₂, un groupe aliphatique en C_{1 à 6} facultativement substitué, -OR', -C(O)R', -CO₂R', -CONH(R⁴), -N(R⁴)COR', -SO₂N(R⁴)₂ ou -N(R⁴)SO₂R'.

7. Composé selon la revendication 6, dans lequel les groupes R¹ sont choisis parmi -CF₃, -Cl, -F, -CN, -COCH₃, -OCH₃, -OH, -CH₂CH₃, -OCH₂CH₃, -CH₃, -CF₂CH₃, cyclohexyle, t-butyle, isopropyle, cyclopropyle, -C≡CH, -C≡C-CH₃, -SO₂CH₃, -SO₂NH₂, -N(CH₃)₂, -CO₂CH₃, -CONH₂, -NHCOCH₃, -OC(O)NH₂, -NHSO₂CH₃ ou -OCF₃ ; et les groupes R⁵ sont choisis parmi -Cl, -F, -CN, -CF₃, -NH₂, -NH(aliphatique en C_{1 à 4}), -N(aliphatique en C_{1 à 4})₂, -O(aliphatique en C_{1 à 4}), aliphatique en C_{1 à 4} ou -CO₂(aliphatique en C_{1 à 4}).

8. Composé selon la revendication 2, ayant l'une des formules : 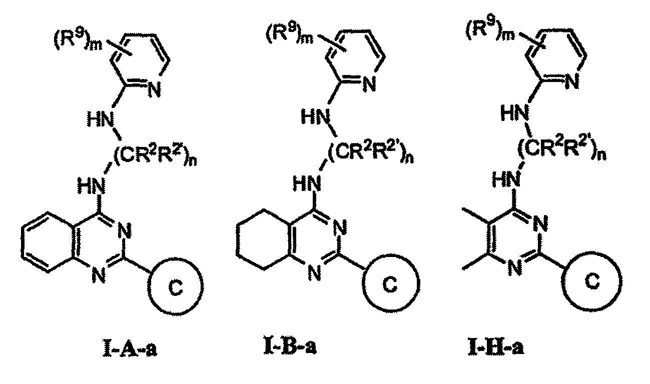 dans lesquelles m vaut 0 à 4, et chaque occurrence de R⁹ est indépendamment choisie parmi H, un groupe nitro, amino, cyano, halogéno, thioamido, amidino, oxamidino, alcoxyamidino, imidino, guanidino, sulfonamido, carboxyle, formyle, alkyle, halogénoalkyle, alcoxy, halogénoalcoxy, alcoxyalkyle, alkylaminoalcoxy, alkylcarbonyle, aralkylcarbonyle, hétéroaralkylcarbonyle, alkylthio, aryle, aralkyle, hétéroaryle ou hétéroaralkyle.

9. Composé selon la revendication 8, dans lequel le cycle C est un cycle phényle et R¹ est un groupe halogéno, méthyle, cyano, OMe, OH ou trifluorométhyle.

10. Composé selon la revendication 2, dans lequel le cycle C est un cycle phényle ou pyridinyle comportant un ou deux substituants ortho indépendamment choisis parmi -R¹, où le cycle C est en outre substitué par -R⁵ et où, lorsque le cycle C et deux substituants adjacents dessus forment un système de cycle bicyclique, le système de cycle bicyclique est choisi parmi un cycle naphtyle, quinoléinyle ou isoquinoléinyle.

11. Composé selon la revendication 2 ou la revendication 10, dans lequel R¹ est un groupe halogéno, un groupe aliphatique en C_{1 à 6} facultativement substitué, phényle, -COR⁶, -OR⁶, -CN, -SO₂R⁶, -SO₂NH₂, -N(R⁶)₂, -CO₂R⁶, -CONH₂, -NHCOR⁶, -OC(O)NH₂ ou -NHSO₂R⁶.

12. Composé selon la revendication 2, dans lequel le cycle C est un cycle phényle ou pyridinyle comportant un ou deux substituants ortho indépendamment choisis parmi -R¹, où le cycle C est en outre substitué par -R⁵ et où, lorsque le cycle C et deux substituants adjacents dessus forment un système de cycle bicyclique, le système de cycle bicyclique est un cycle naphtyle.

13. Composé selon la revendication 2 ou la revendication 12, dans lequel R¹ est un groupe halogéno, un groupe halogénoaliphatique en C_{1 à 6}, un groupe aliphatique en C_{1 à 6}, phényle, OMe, OH ou -CN.

14. Composé selon l'une quelconque des revendications 2, 12 ou 13, dans lequel chaque R⁵ est indépendamment choisi parmi un groupe halogéno, -CN, -NO₂, -N(R⁴)₂, un groupe aliphatique en C_{1 à 6} facultativement substitué, -OR', -C(O)R', -CO₂R', -CONH(R⁴), -N(R⁴)COR', -SO₂N(R⁴)₂ ou -N(R⁴)SO₂R'.

15. Composé selon la revendication 2, dans lequel B est un groupe pyridyle facultativement substitué par 0, 1 ou 2 occurrences de R⁹, où chaque occurrence de R⁹ est indépendamment un atome d'hydrogène, un groupe alkyle en C_{1 à 4}, nitro, amino, cyano, cyanométhyle, trifluorométhyle, hydroxy ou méthoxy.

16. Composé selon la revendication 15, dans lequel le cycle C est un cycle phényle ou pyridinyle comportant un ou deux substituants ortho indépendamment choisis parmi -R¹, où le cycle C est en outre substitué par -R⁵ et où, lorsque le cycle C et deux substituants adjacents dessus forment un système de cycle bicyclique, le système de cycle bicyclique est un cycle naphtyle.

17. Composé selon l'une quelconque des revendications 2, 15 ou 16, dans lequel R¹ est un groupe halogéno, un groupe halogénoaliphatique en C_{1 à 6}, un groupe aliphatique en C_{1 à 6}, phényle, OMe, OH ou -CN.

18. Composé selon l'une quelconque des revendications 2, 15, 16 ou 17, dans lequel chaque R⁵ est indépendamment choisi parmi un groupe halogéno, -CN, -NO₂, -N(R⁴)₂, un groupe aliphatique en C_{1 à 6} facultativement substitué, -OR', -C(O)R', -CO₂R', -CONH(R⁴), -N(R⁴)COR', -SO₂N(R⁴)₂ ou -N(R⁴)SO₂R'.

19. Composé selon la revendication 2, dans lequel B est un groupe pyridyle facultativement substitué par 2 occurrences indépendantes de R⁹, où chaque occurrence de R⁹ est indépendamment un atome d'hydrogène, un groupe alkyle en C_{1 à 4}, nitro, amino, cyano, cyanométhyle, trifluorométhyle, hydroxy or méthoxy.

20. Composé selon la revendication 2 ou la revendication 19, dans lequel le cycle C est un cycle phényle comportant un ou deux substituants ortho indépendamment choisis parmi -R¹, où le cycle C est en outre substitué par -R⁵.

21. Composé selon l'une quelconque des revendications 2, 19 ou 20, dans lequel R¹ est un groupe halogéno, un groupe aliphatique en C_{1 à 4} facultativement substitué par un atome d'halogène, OMe, OH, ou -CN.

22. Composé selon l'une quelconque des revendications 2, 19, 20 ou 21, dans lequel chaque R⁵ est indépendamment choisi parmi -Cl, -F, -CN, -CF₃, -NH₂, -NH(aliphatique en C_{1 à 4}), -N(aliphatique en C_{1 à 4})₂, -O(aliphatique en C_{1 à 4}), un groupe aliphatique en C_{1 à 4} facultativement substitué et -CO₂(aliphatique en C_{1 à 4}).

23. Composé selon la revendication 1, choisi parmi l'un des composés :

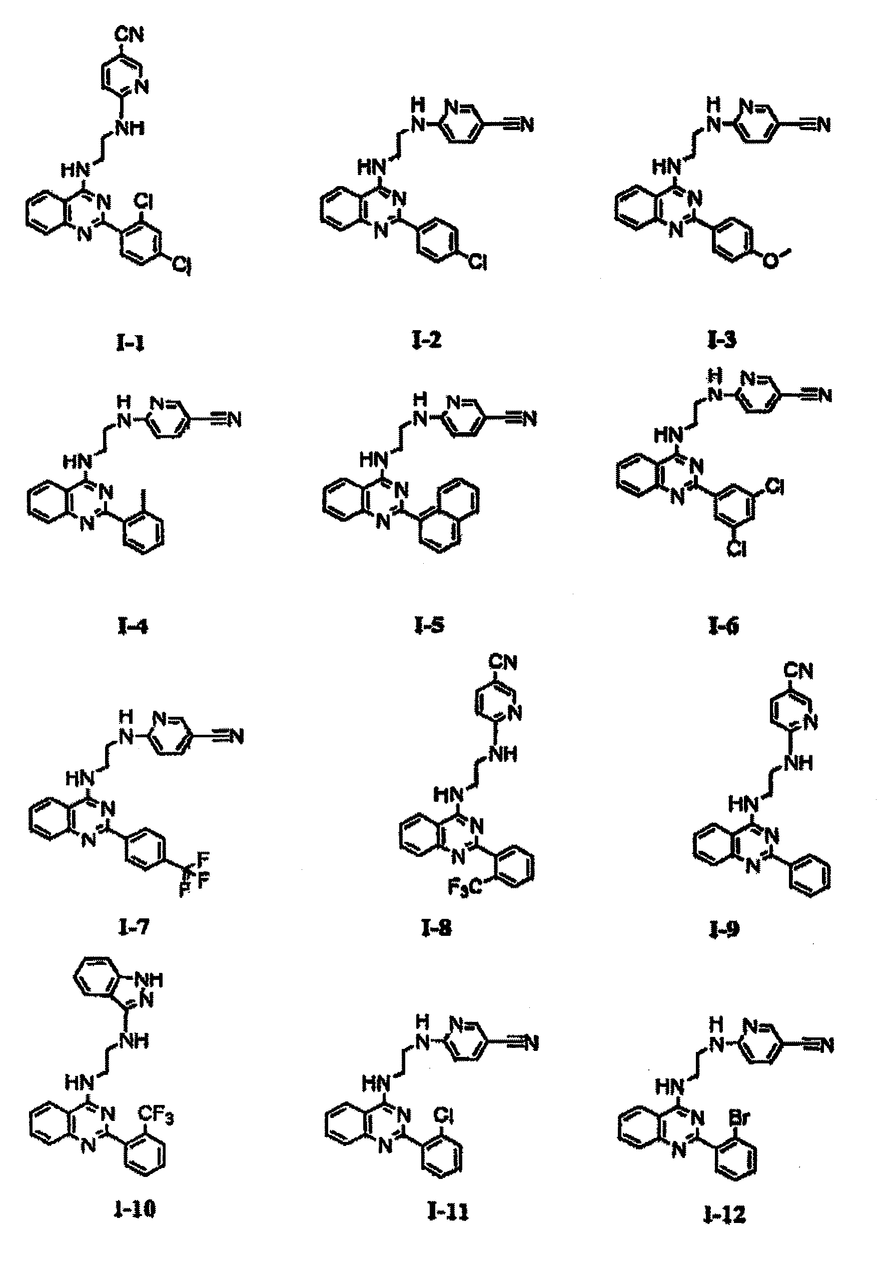

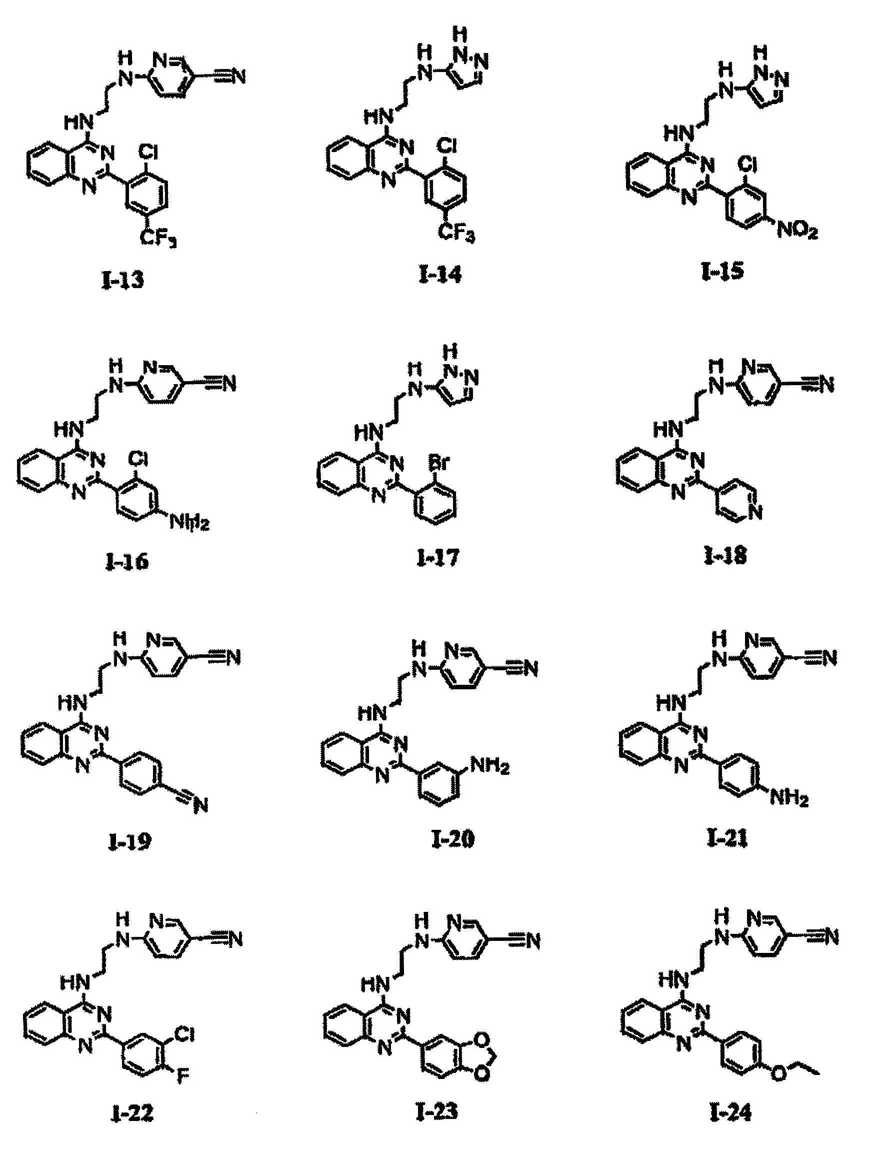

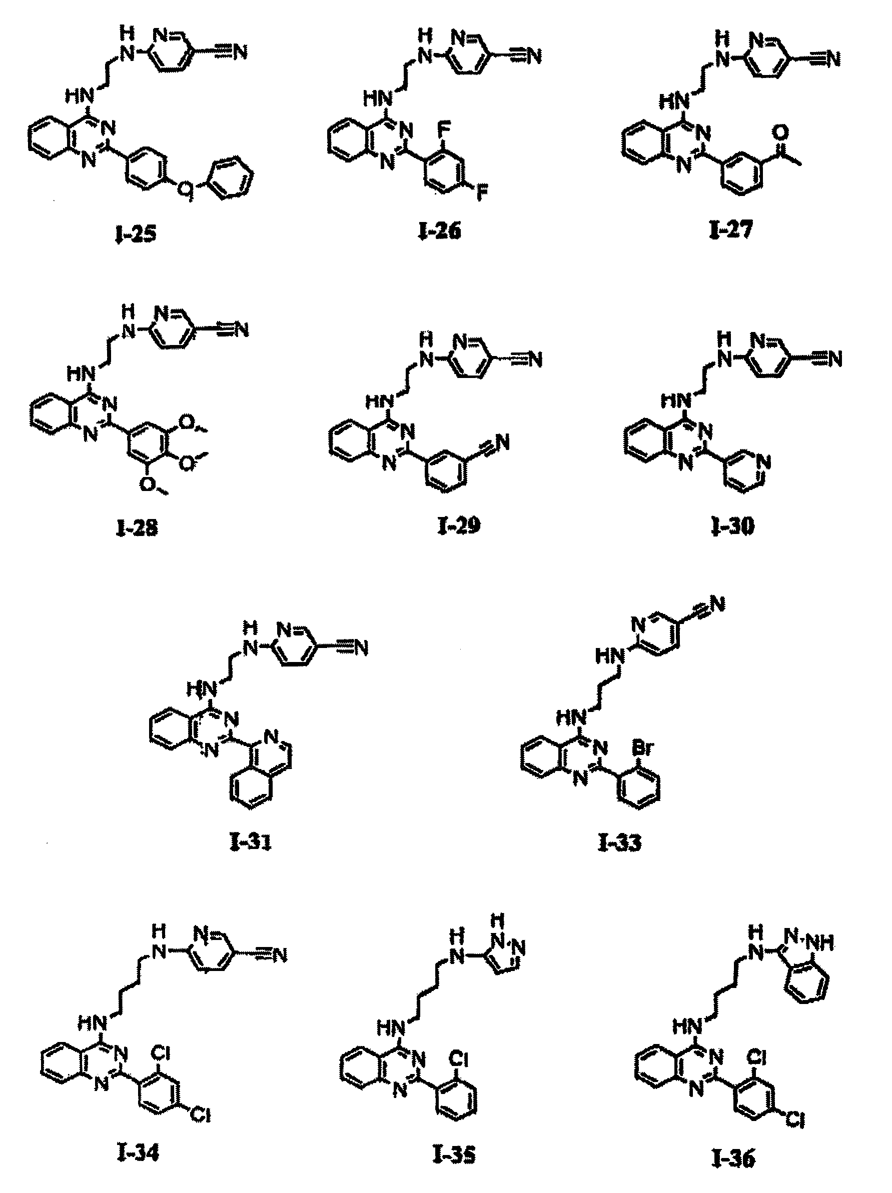

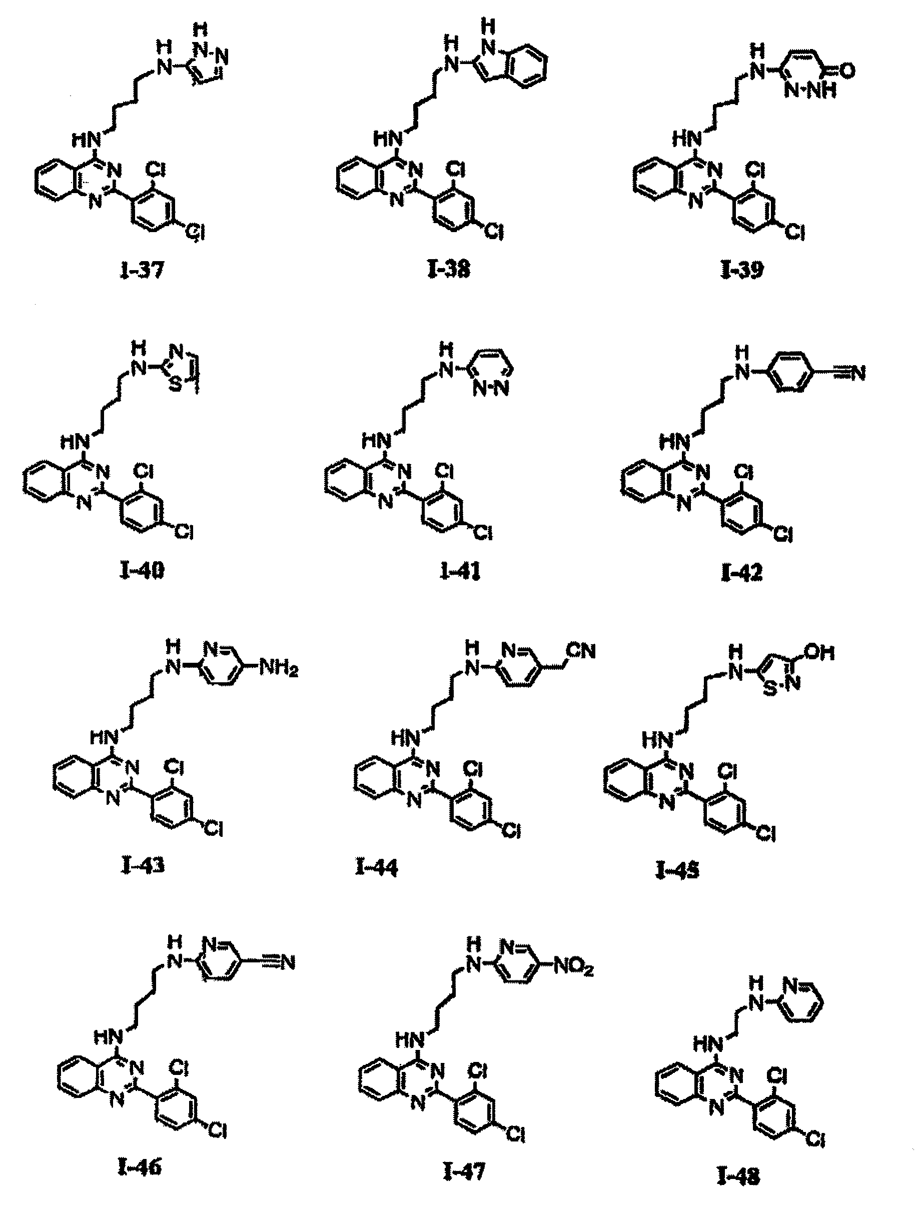

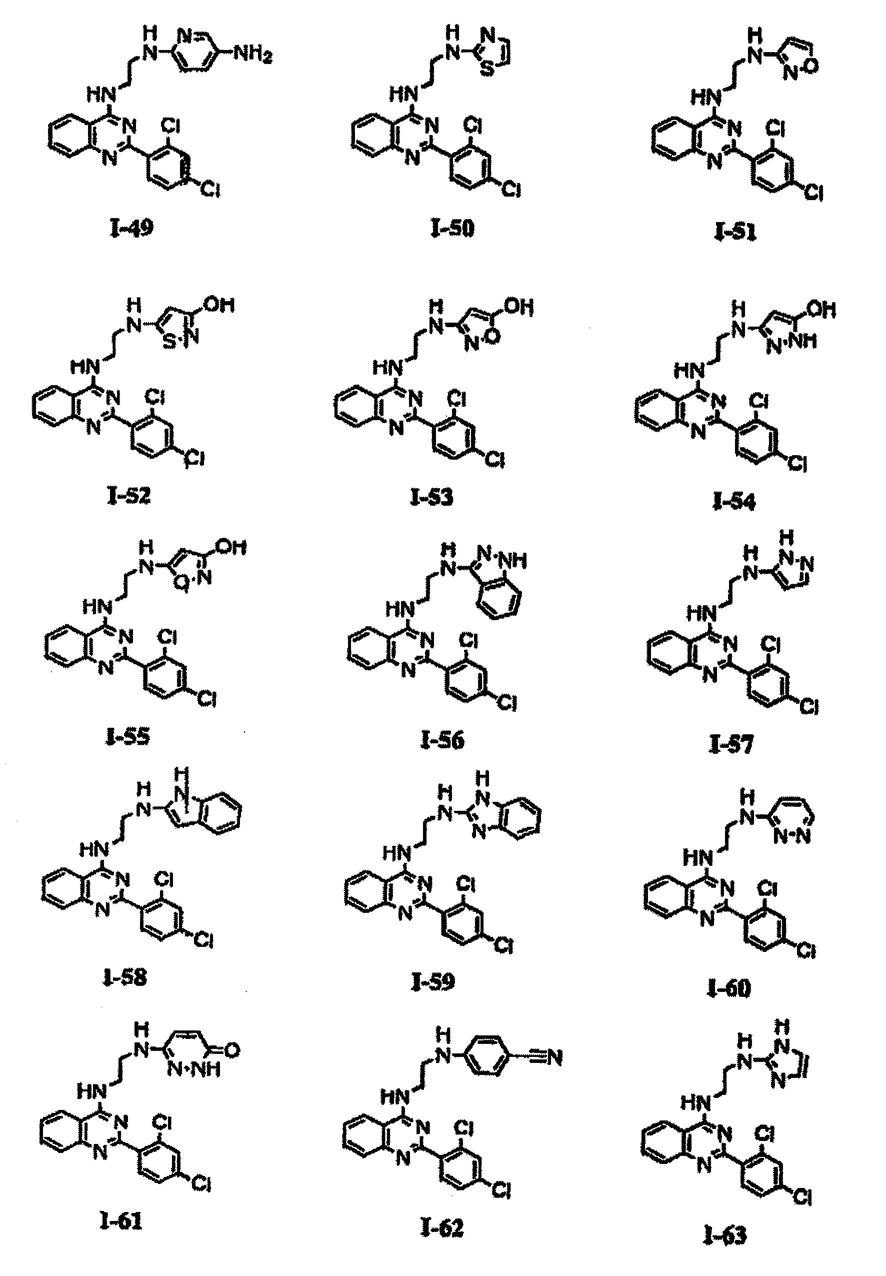

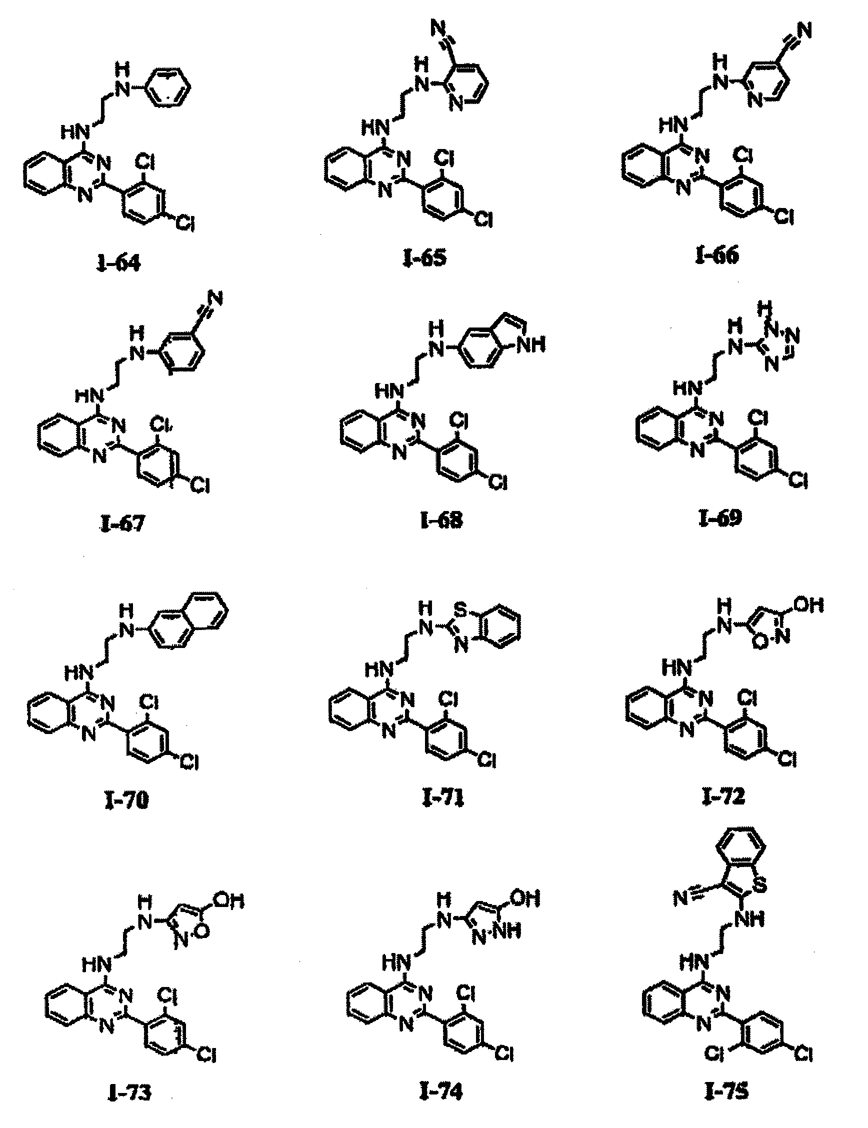

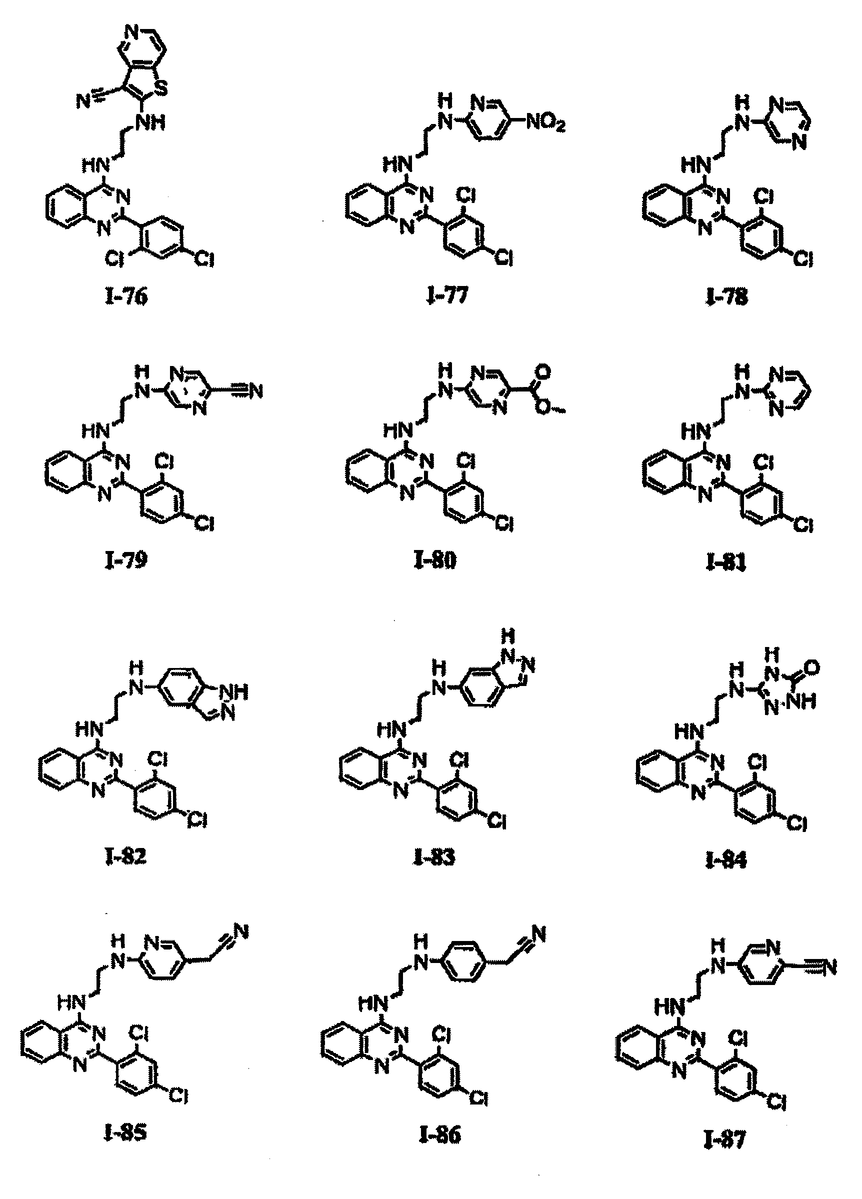

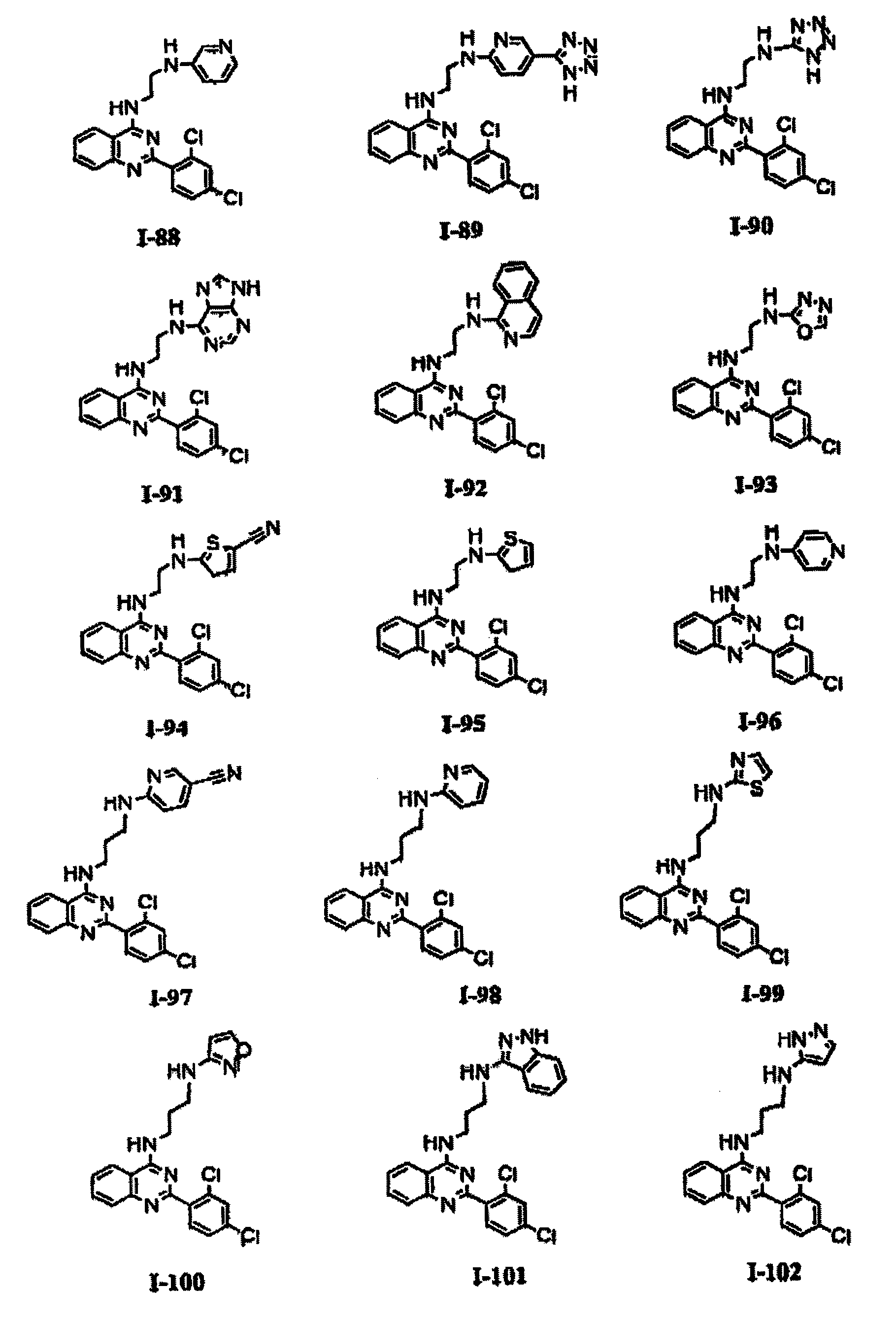

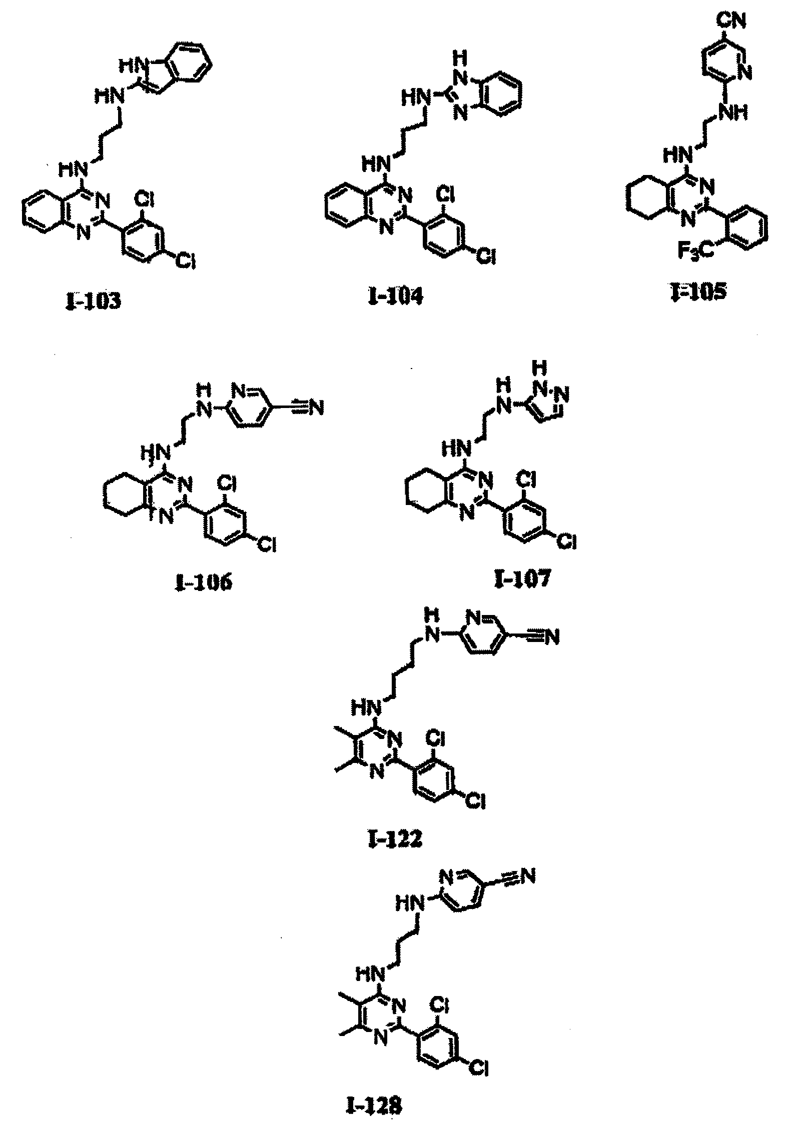

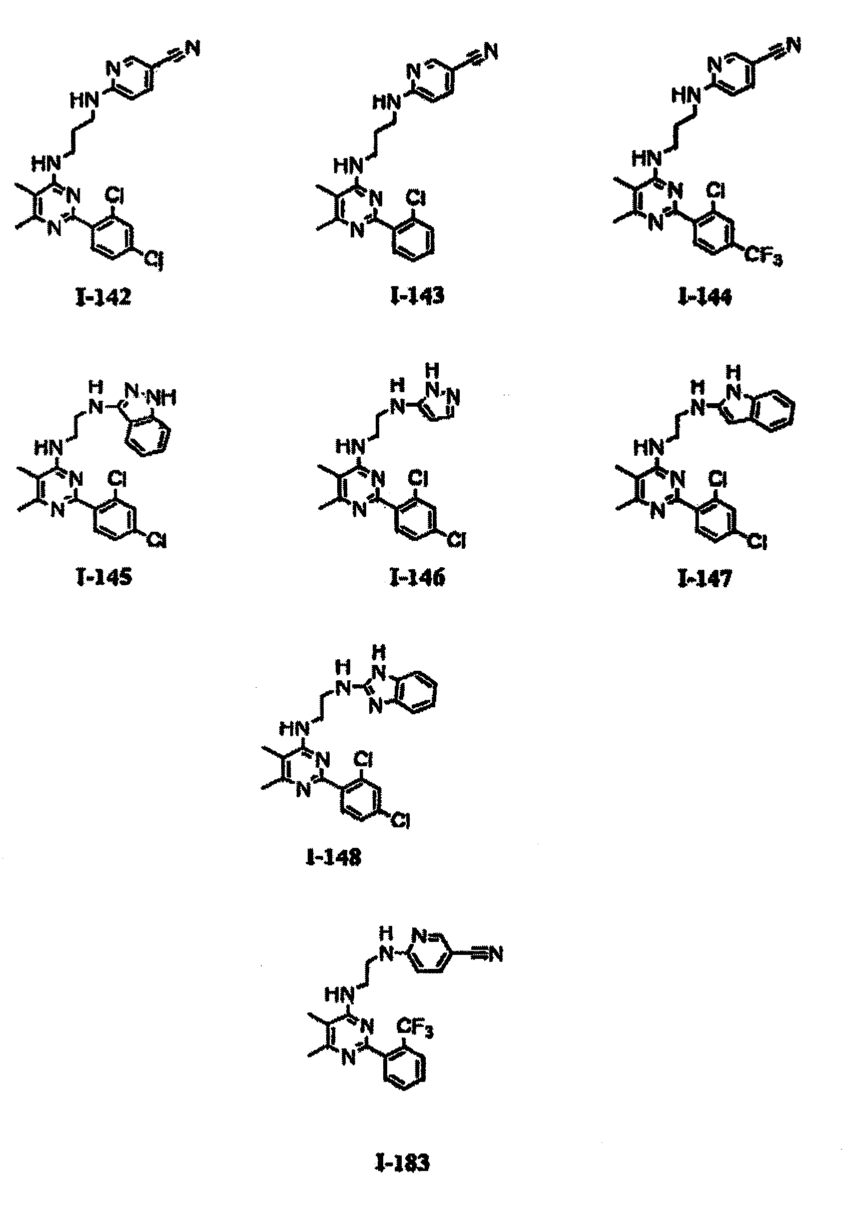

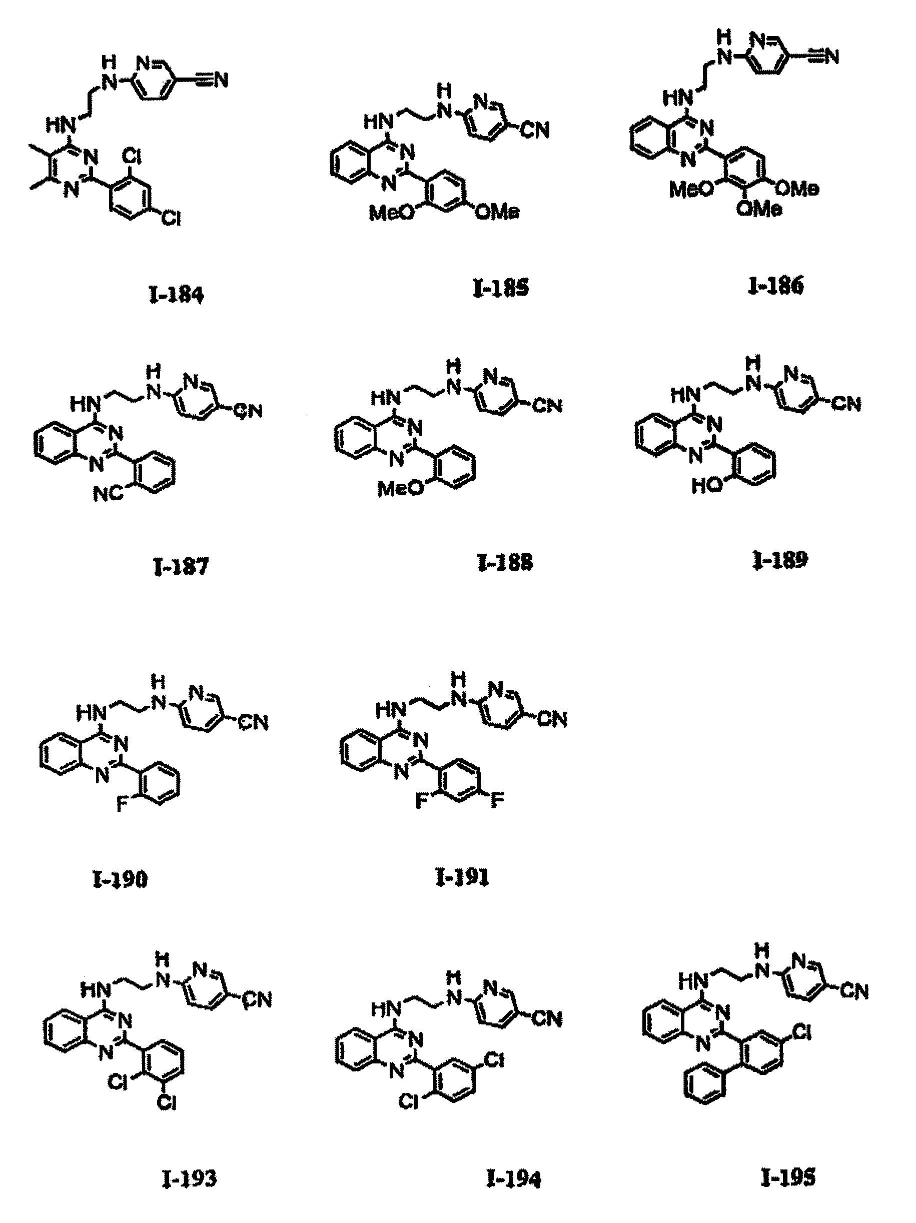

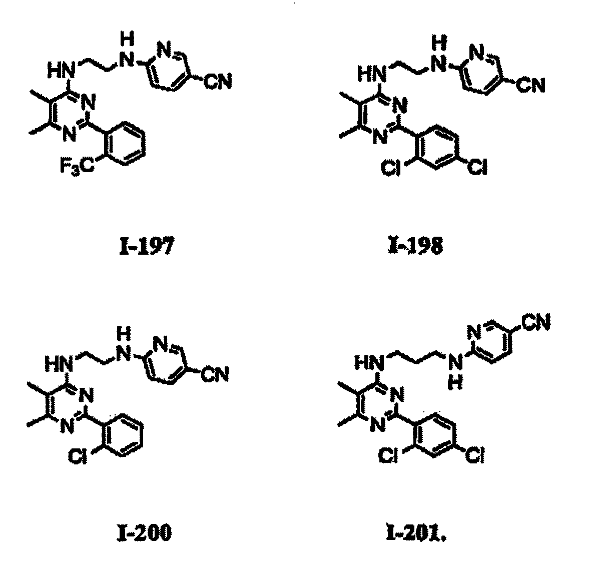

24. Composition comprenant un composé selon la revendication 2, et un support, adjuvant ou véhicule pharmaceutiquement acceptable.

25. Composition comprenant une quantité thérapeutiquement efficace d'un composé selon la revendication 2, et un support, adjuvant ou véhicule pharmaceutiquement acceptable.

26. Composition selon la revendication 25, dans laquelle la quantité thérapeutiquement efficace est une quantité capable d'inhiber l'activité de GSK-3.

27. Composition selon la revendication 24, comprenant en outre un ou plusieurs agents thérapeutiques additionnels.

28. Procédé d'inhibition de l'activité de la kinase GSK-3 dans un échantillon biologique, comprenant l'étape consistant à mettre en contact ledit échantillon biologique avec :
a) une composition selon la revendication 24 ; ou
b) un composé selon la revendication 2.

29. Utilisation : a) d'une composition selon la revendication 24 ; ou b) d'un composé selon la revendication 2, pour la fabrication d'un médicament destiné à traiter ou à atténuer la sévérité d'une maladie auto-immune, d'une maladie inflammatoire, d'un trouble métabolique, d'un trouble nerveux ou neurodégénératif ou d'une maladie cardiovasculaire chez un patient.

30. Utilisation selon la revendication 29, dans laquelle la maladie est choisie parmi une allergie, l'asthme, le diabète, la maladie d'Alzheimer, la maladie d'Huntington, la maladie de Parkinson, la démence associée au sida, la sclérose latérale amyotrophique (AML, maladie de Lou Gehrig), la sclérose en plaques (MS), la schizophrénie, l'hypertrophie du cardiomyocyte, la reperfusion/l'ischémie, l'accident cérébro-vasculaire ou la calvitie.

31. Utilisation selon la revendication 30, dans laquelle la maladie est un accident cérébro-vasculaire.

32. Utilisation selon la revendication 29, dans laquelle la maladie est un trouble nerveux ou neurodégénératif.

33. Utilisation selon la revendication 29, dans laquelle ledit médicament est combiné avec un agent thérapeutique additionnel choisi parmi un traitement contre la maladie d'Alzheimer, un traitement contre la maladie de Parkinson, un agent pour traiter la sclérose en plaques (MS), un traitement contre l'asthme, un agent anti-inflammatoire, un agent immunomodulateur ou immunosuppresseur, un facteur neurotrophique, un agent pour traiter l'accident cérébro-vasculaire, un agent pour traiter la maladie cardiovasculaire ou un agent pour traiter le diabète, où ledit agent thérapeutique additionnel est approprié pour la maladie traitée.
